# EUROPÄISCHE PATENTSCHRIFT

(11) **EP 1 658 266 B1**
(45) Veröffentlichungstag und Bekanntmachung des Hinweises auf die Patenterteilung: **11.08.2010**
(21) Anmeldenummer: 04764024.8
(22) Anmeldetag: 12.08.2004
(51) Int. Cl.: C07C 381/00, A61K 31/155, A61P 9/10, A01N 1/02

(54) **PENTAFLUOROSULFANYLPHENYL-SUBSTITUIERTE BENZOYLGUANIDINE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
PENTAFLUOROSULFANYL PHENYL-SUBSTITUTED BENZOYLGUANIDINES, METHOD FOR THEIR PRODUCTION, THEIR USE AS MEDICAMENTS OR DIAGNOSTIC AGENTS AND MEDICAMENT CONTAINING SAID COMPOUNDS
BENZOYLGUANIDINES A SUBSTITUTION PENTAFLUOROSULFANYLPHENYLE, LEURS PROCEDES DE PRODUCTION, LEUR UTILISATION EN TANT QUE MEDICAMENTS OU AGENTS DE DIAGNOSTIC, AINSI QUE MEDICAMENT LES CONTENANT

(30) Priorität: 22.08.2003 DE 10338554
(43) Veröffentlichungstag der Anmeldung: 24.05.2006
(73) Patentinhaber: Sanofi-Aventis Deutschland GmbH, 65929 Frankfurt am Main (DE)
(72) Erfinder: KLEEMANN, Heinz-Werner, 65474 Bischofsheim (DE)
(86) Internationale Anmeldenummer: PCT/EP2004/009020
(87) Internationale Veröffentlichungsnummer: WO 2005/021492

(56) Entgegenhaltungen:
- EP-A- 0 723 956
- WO-A-03/097591
- WO-A-03/106410

## Beschreibung

Die Erfindung betrifft Pentafluorosulfanylphenyl-substituierte Benzoylguanidine der Formeln I und II 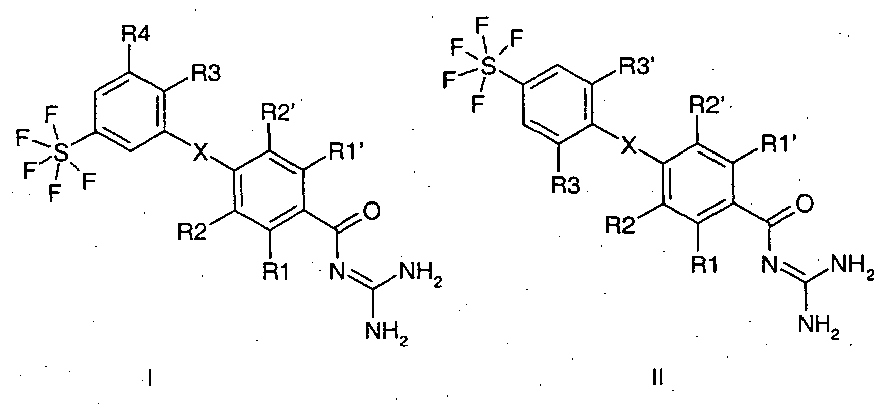 worin bedeuten
- R1 und R1': unabhängig voneinander Wasserstoff, Alkyl mit 1, 2, 3 oder 4 C-Atomen, Alkoxy mit 1, 2, 3 oder 4 C-Atomen, F, Cl, Br, I, -CN, NR5R6, -Oₐ-(CH₂)_{b}-(CF₂)_{c}-CF₃ oder -(SO_{d})ₑ-(CH₂)_{f}-(CF₂)_{g}-CF₃;
R5 und R6 unabhängig voneinander Wasserstoff, Alkyl mit 1, 2, 3 oder 4 C- Atomen oder -CH₂-CF₃;
d Null, 1 oder 2;
a, b, c, e, f und g unabhängig voneinander Null oder 1;
- R2 und R2': unabhängig voneinander Wasserstoff, F, Cl, Br, I, -CN, -SO₂CH₃, Alkoxy mit 1, 2, 3 oder 4 C-Atomen, NR5R6, -Oₐ-(CH₂)_{b}-(CF₂)_{c}-CF₃, -(SOₕ)ₖ-(CH₂)ₗ-(CF₂)ₘ-
CF₃, Alkyl mit 1, 2, 3, 4, 5 oder 6 C-Atomen, Cycloalkyl mit 3, 4, 5, 6, 7 oder 8 C- Atomen, in dem 1, 2, 3 oder 4 Wasserstoff-Atome durch Fluoratome ersetzt sein können, -(CH₂)ₙ -Phenyl, das unsubstituiert ist oder substituiert ist mit 1, 2 oder 3 Resten ausgewählt aus der Gruppe bestehend aus F, Cl, Br, I, -Oₒ-(CH₂)ₚ-CF₃, Alkoxy mit 1, 2, 3 oder 4 C-Atomen, Alkyl mit 1, 2, 3 oder 4 C-Atomen und -SO₂CH₃, oder -(CH₂)_{q} -Heteroaryl, das unsubstituiert ist oder substituiert ist mit 1, 2 oder 3 Resten ausgewählt aus der Gruppe bestehend aus F, Cl, Br, I, -Oᵣ(CH₂)ₛ-CF₃, Alkoxy mit 1, 2, 3 oder 4 C-Atomen, Alkyl mit 1, 2, 3 oder 4 C- Atomen und -SO₂CH₃;
R5 und R6 unabhängig voneinander Wasserstoff, Alkyl mit 1, 2, 3 oder 4 C- Atomen oder -CH2-CF₃;
a, b und c unabhängig voneinander Null oder 1;
h Null, 1 oder 2;
k Null oder 1;
l Null, 1, 2, 3 oder 4;
m und o unabhängig voneinander Null oder 1;
p Null, 1, 2 oder 3;
n Null, 1, 2, 3 oder 4;
r Null oder 1;
s Null, 1, 2 oder 3;
q Null, 1, 2, 3 oder 4;
- R3 und R3': unabhängig voneinander Wasserstoff, F, Cl, Br, I, -CN, -SO₂CH₃, Alkyl mit 1, 2, 3, 4, 5 oder 6 C-Atomen, Alkoxy mit 1, 2, 3 oder 4 C-Atomen oder -Oₜ-(CH₂)ᵤ-CF₃;
t Null oder 1;
u Null, 1, 2 oder 3;
- R4: Wasserstoff, F, Cl, Br, I, -CN, -SO₂CH₃, NR5R6, -(SOᵥ)_{w}-(CH₂)ₓ-(CF₂)_{y}-CF₃, -O_{z}-(CH₂)ₐₐ -(CF₂)_{bb}-CF₃, Alkyl mit 1, 2, 3, 4, 5 oder 6 C-Atomen, Alkoxy mit 1, 2, 3 oder 4 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R5 und R6 unabhängig voneinander Wasserstoff, Alkyl mit 1, 2, 3 oder 4 C- Atomen oder -CH₂-CF₃;
v Null, 1 oder 2;
x Null, 1, 2, 3 oder 4;
w, y, z, aa und bb unabhängig voneinander Null oder 1;
oder
- R4: -(CH₂)_{cc} -Phenyl, das unsubstituiert ist oder substituiert ist mit 1, 2 oder 3 Resten ausgewählt aus der Gruppe bestehend aus F, Cl, Br, I, -O_{dd}-(CH₂)ₑₑ-CF₃, Alkoxy mit 1, 2, 3 oder 4 C-Atomen, Alkyl mit 1, 2, 3 oder 4 C-Atomen und -SO₂CH₃;
dd Null oder 1;
ee Null, 1, 2 oder 3;
cc Null, 1, 2, 3 oder 4;
oder
- R4: -(CH₂)_{ff}-Heteroaryl, das unsubstituiert ist oder substituiert ist mit 1, 2 oder 3 Resten ausgewählt aus der Gruppe bestehend aus F, Cl, Br, I, -Ogg-(CH₂)ₕₕ- CF₃, Alkoxy mit 1, 2, 3 oder 4 C-Atomen, Alkyl mit 1, 2, 3 oder 4 C-Atomen und -SO₂CH₃;
gg Null oder 1;
hh Null, 1, 2 oder 3;
ff Null, 1, 2, 3 oder 4;
- X: eine direkte Bindung, O, NR7, S(O)ₖₖ;
R7 Wasserstoff, Alkyl mit 1, 2, 3 oder 4 C-Atomen, -(CH₂)ₘₘ-CF₃ oder -SO₂CH₃
kk Null, 1 oder 2;
mm Null, 1, 2 oder 3;
wobei -Oₐ-(CH₂)_{b}-(CF₂)_{c}-CF₃ in den Definitionen von R1 und R1' und R2 und R2' unabhängig voneinander gewählt werden kann,
wobei NR5R6 in den Definitionen von R1 und R1', R2 und R2' und R4 unabhängig voneinander gewählt werden kann,
sowie deren pharmazeutisch verträgliche Salze.

Bevorzugt sind Verbindungen der Formeln I und II, in denen bedeuten:
- R1 und R1': unabhängig voneinander Wasserstoff, Alkyl mit 1, 2, 3 oder 4 C-Atomen, Methoxy, Ethoxy, F, Cl, NR5R6, -O-CH₂-CF₃ oder -(SO_{d})ₑ-(CH₂)_{f}-CF₃;
R5 und R6 unabhängig voneinander Wasserstoff, Alkyl mit 1, 2, 3 oder 4 C-Atomen oder -CH₂-CF₃;
d Null, 1 oder 2;
e und f unabhängig voneinander Null oder 1;
- R2 und R2': unabhängig voneinander Wasserstoff, F, Cl, -SO₂CH₃, -(SOₕ)ₖ-(CH₂)ₗ-CF₃, Alkyl mit 1, 2, 3 oder 4 C-Atomen, Cycloalkyl mit 3, 4, 5, 6 oder 7 C-Atomen, in dem 1, 2, 3 oder 4 Wasserstoff-Atome durch Fluoratome ersetzt sein können, Phenyl, das unsubstituiert ist oder substituiert ist mit 1 - 2 Resten ausgewählt aus der Gruppe bestehend aus F, Cl, -Oₒ-(CH₂)ₚ-CF₃, Methoxy, Ethoxy, Alkyl mit 1, 2, 3 oder 4 C-Atomen und -SO₂CH₃, oder Heteroaryl, das unsubstituiert ist oder substituiert ist mit 1 - 2 Resten ausgewählt aus der Gruppe bestehend aus F, Cl, -Oᵣ-(CH₂)ₛ- CF₃, Methoxy, Ethoxy, Alkyl mit 1, 2, 3 oder 4 C-Atomen und -SO₂CH₃;
h Null, 1 oder 2;
k Null oder 1;
l Null, 1, 2, 3 oder 4;
o Null oder 1;
p Null, 1, 2 oder 3;
r Null oder 1;
s Null, 1, 2 oder 3;
- R3 und R3': unabhängig voneinander Wasserstoff, F, Cl, -SO₂CH₃, Alkyl mit 1, 2, 3 oder 4 C- Atomen, Methoxy, Ethoxy oder -Oₜ-(CH₂)ᵤ-CF₃,
t Null oder 1;
u Null, 1, 2 oder 3;
- R4: Wasserstoff, F, Cl, -SO₂CH₃, -(SOᵥ)_{w}-(CH₂)ₓ-CF₃, -O_{z}-(CH₂)ₐₐ-CF₃, Alkyl mit 1, 2, 3 oder 4 C-Atomen, Methoxy, Ethoxy oder Cycloalkyl mit 3, 4, 5, 6 oder 7 C- Atomen, in dem 1, 2, 3 oder 4 Wasserstoff-Atome durch Fluoratome ersetzt sein können;
v Null, 1 oder 2;
w, x, z und aa unabhängig voneinander Null oder 1;
oder
- R4: Phenyl, das unsubstituiert ist oder substituiert ist mit 1 - 2 Resten ausgewählt aus der Gruppe bestehend aus F, Cl, -O_{dd}-(CH₂)ₑₑ-CF₃, Methoxy, Ethoxy, Alkyl mit 1, 2, 3 oder 4 C-Atomen und -SO₂CH₃;
dd und ee unabhängig voneinander Null oder 1;
oder
- R4: Heteroaryl, das unsubstituiert ist oder substituiert ist mit 1 - 2 Resten ausgewählt aus der Gruppe bestehend aus F, Cl, -O_{gg}-(CH₂)ₕₕ-CF₃, Methoxy, Ethoxy, Alkyl mit 1, 2, 3 oder 4 C-Atomen und -SO₂CH₃;
gg und hh unabhängig voneinander Null oder 1;
- X: eine direkte Bindung, O, NR7, S(O)ₖₖ
R7 Wasserstoff, Alkyl mit 1, 2, 3 oder 4 C-Atomen, -CH₂-CF₃ oder -SO₂CH₃;
kk Null, 1 oder 2;
sowie deren pharmazeutisch verträgliche Salze.

Besonders bevorzugt sind Verbindungen der Formeln I und II, in denen bedeuten:
- R1 und R1': unabhängig voneinander Wasserstoff, Alkyl mit 1, 2, 3 oder 4 C-Atomen, Methoxy, Ethoxy, F, Cl, NR5R6, -O-CH₂-CF₃ oder -(SO_{d})ₑ -(CH₂)_{f} -CF₃;
R5 und R6 unabhängig voneinander Wasserstoff, Alkyl mit 1, 2, 3 oder 4 C- Atomen oder -CH₂-CF₃;
d Null, 1 oder 2;
e und f unabhängig voneinander Null oder 1;
- R2 und R2': unabhängig voneinander Wasserstoff, F, Cl, -SO₂CH₃, -(SOₕ)ₖ -(CH₂)ₗ -CF₃, Methyl, Cycloalkyl mit 3, 4, 5, 6 oder 7 C-Atomen, in dem 1, 2, 3 oder 4 Wasserstoff-Atome durch Fluoratome ersetzt sein können, Phenyl, das unsubstituiert ist oder substituiert ist mit einem Rest ausgewählt aus der Gruppe bestehend aus F, Cl, -Oₒ-(CH₂)ₚ-CF₃, Methoxy, Ethoxy, Alkyl mit 1, 2, 3 oder 4 C-Atomen und -SO₂CH₃, oder Heteroaryl, das unsubstituiert ist oder substituiert ist mit einem Rest ausgewählt aus der Gruppe bestehend aus F, Cl, -Oᵣ(CH₂)ₛ CF₃, Methoxy, Ethoxy, Alkyl mit 1, 2, 3 oder 4 C-Atomen und -SO₂CH₃;
h Null, 1 oder 2;
k, l, o, p, r und s unabhängig voneinander Null oder 1;
- R3 und R3': unabhängig voneinander Wasserstoff, F, Cl, -SO₂CH₃, Methyl, Methoxy, Ethoxy oder -Oₜ-(CH₂)ᵤ-CF₃;
t und u unabhängig voneinander Null oder 1;
- R4: Wasserstoff, F, Cl, -SO₂CH₃, -(SOᵥ)_{w}-(CH₂)ₓ-CF₃, -O_{z}-(CH₂)ₐₐ-CF₃, Methyl, Methoxy, Ethoxy oder Cycloalkyl mit 3, 4, 5, 6 oder 7 C-Atomen, in dem 1, 2, 3 oder 4 Wasserstoff-Atome durch Fluoratome ersetzt sein können;
v Null, 1 oder 2;
w, x, z und aa unabhängig voneinander Null oder 1; X
- X: eine direkte Bindung, O, NR7 oder S(O)ₖₖ;
R7 Wasserstoff, Methyl, Ethyl, -CH₂-CF₃ oder -SO₂CH₃;
kk Null, 1 oder 2
sowie deren pharmazeutisch verträgliche Salze.

Ganz besonders bevorzugt sind Verbindungen der Formeln I und II, in denen bedeuten:
- R1 und R1': unabhängig voneinander Wasserstoff, Methyl, F, Cl, -CF₃ oder -O-CH₂-CF₃;
- R2 und R2': unabhängig voneinander Wasserstoff, F, Cl, -SO₂CH₃, -SO₂-CF₃, -CF₃ oder Methyl;
- R3 und R3': unabhängig voneinander Wasserstoff, F, Cl, -SO₂CH₃, Methyl, -CF₃ oder -O-CH₂-CF₃;
- R4: Wasserstoff, F, Cl, -SO₂CH₃, -O-CH₂-CF₃ oder Methyl;
- X: eine direkte Bindung, O, NR7 oder S(O)_{kk;}
R7 Wasserstoff, Methyl, Ethyl, -CH₂-CF₃ oder -SO₂CH₃;
kk Null, 1 oder 2;
sowie deren pharmazeutisch verträgliche Salze.

In einer Ausführungsform sind dabei Verbindungen der Formeln I und II bevorzugt, in denen R1 und R1' unabhängig voneinander durch Wasserstoff, Methyl, F, Cl, -CF₃ oder -O-CH₂-CF₃ beschrieben werden, besonders bevorzugt sind Verbindungen, in denen R1 und R1' unabhängig voneinander durch Wasserstoff oder Methyl beschrieben werden.

In einer weiteren Ausführungsform sind Verbindungen der Formeln I und II bevorzugt, in denen R2 und R2' unabhängig voneinander durch Wasserstoff, F, Cl, -SO₂CH₃, - SO₂CF₃, -CF₃ oder Methyl beschrieben werden, besonders bevorzugt sind Verbindungen, in denen R2 und R2' unabhängig voneinander durch Wasserstoff oder - SO₂CH₃ beschrieben werden.

In einer weiteren Ausführungsform sind Verbindungen der Formeln I und II bevorzugt, in denen R3 und R3' unabhängig voneinander durch Wasserstoff, F, Cl, -SO₂CH₃, Methyl, -CF₃ oder -O-CH₂-CF₃ beschrieben werden, besonders bevorzugt sind Verbindungen, in denen R3 und R3' unabhängig voneinander durch Wasserstoff oder Methyl beschrieben werden.

In einer weiteren Ausführungsform sind Verbindungen der Formeln I und II bevorzugt, in denen R4 durch Wasserstoff, F, Cl, -SO₂CH₃, -O-CH₂-CF₃ oder Methyl beschrieben wird, besonders bevorzugt sind Verbindungen, in denen R4 durch Wasserstoff beschrieben wird.

In einer weiteren Ausführungsform sind Verbindungen der Formeln I und II bevorzugt, in denen X eine direkte Bindung ist oder durch O, NR7 oder S(O)ₖₖ beschrieben wird, wobei R7 Wasserstoff, Methyl, Ethyl, -CH₂-CF₃ oder -SO₂CH₃ ist, bevorzugt Wasserstoff oder Methyl, und wobei kk Null, 1 oder 2 ist, bevorzugt Null oder 2.

Speziell bevorzugt sind Verbindungen ausgewählt aus der Gruppe:
N-[5-Methansulfonyl-2-methyl-4-(3-pentafluorosulfanyl-phenoxy)-benzoyl]-guanidin,
N-[5-Methansulfonyl-2-methyl-4-(4-pentafluorosulfanyl-phenoxy)-benzoyl]-guanidin,
N-[5-Methansulfonyl-2-methyl-4-(4-penfafluorosulfanyl-phenylsulfanyl)-benzoyl]-guanidin,
N-[5-Methansulfonyl-2-methyl-4-(4-pentaflluorosulfanyl-phenylsulfonyl)-benzoyl]-guanidin,
N-[5-Methansulfonyl-2-methyl-4-(3-pentafluorosulfanyl-phenylamino)-benzoyl]-guanidin,
N-[5-Methansulfonyl-2-methyl-4-(4-pentafluorosulfanyl-phenylamino)-benzoyl]-guanidin,
N-{5-Methansulfonyl-2-methyl-4-[methyl-(3-pentafluorosulfanyl-phenyl)-amino]-benzoyl}-guanidin,
N-{5-Methansulfonyl-2-methyl-4-[methyl-(4-pentafluorosulfanyl-phenyl)-amino]-benzoyl}-guanidin,
N-(2-Methansulfonyl-5-methyl-4'-pentafluorosulfanyl-biphenyl-4-carbonyl)-guanidin,
N-(2-Methansulfonyl-5-methyl-3'-pentafluorosulfanyl-biphenyl-4-carbonyl)-guanidin und
N-(2-Methansulfonyl-5,2'-dimethyl-4'-pentafluorosulfanyl-biphenyl-4-carbonyl)-guanidin.

Enthalten die Substituenten R1, R1', R2, R2', R3, R3' oder R4 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben in allen Verhältnissen vorliegen.

Die vorliegende Erfindung umfasst alle tautomeren Formen der Verbindungen der Formeln I und II.

Alkylreste können geradkettig oder verzweigt sein. Dies gilt auch, wenn sie Substituenten tragen oder als Substituenten anderer Reste auftreten, beispielsweise in Fluoralkylresten, Alkoxyresten, Fluoralkoxyresten, Alkylaminoresten, Dialkylaminoresten und Alkylsulfonylresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), Pentyl und Hexyl. Bevorzugte Alkylreste sind Methyl, Ethyl, n-Propyl und Isopropyl, besonders bevorzugt Methyl und Ethyl. Substituierte Alkylreste können in beliebigen Positionen substituiert sein.

Beispiele für Cycloalkylreste sind Cyclopropyl, Cyclobutyl, Cyclopentyl, Cyclohexyl Cycloheptyl oder Cyclooctyl. Substituierte Cycloalkylreste können in beliebigen Positionen substituiert sein.

Phenylreste können unsubstituiert sein oder einfach oder mehrfach, zum Beispiel einfach, zweifach oder dreifach, durch gleiche oder verschiedene Reste substituiert sein. Dies gilt ebenso für Phenylreste in Gruppen wie zum Beispiel Phenylalkyl oder Phenyloxy. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Heteroarylreste sind aromatische Ringverbindungen, in denen ein oder mehrere Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombinationen aus verschiedenen Heteroatomen. Die Heteroarylreste können über alle Positionen gebunden sein, zum Beispiel über die 1-Position, 2-postition, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein. Dies gilt ebenso für die Heteroarylreste wie zum Beispiel im Rest Heteroarylalkyl. Heteroaryl bedeutet zum Beispiel Furyl, Thienyl, Pyrrolyl, Imidazolyl, Pyrazolyl, Triazolyl, Tetrazolyl, Oxazolyl, Isoxazolyl, Oxadiazolyl, Thiadiazolyl, Thiazolyl, Isothiazolyl, Pyridyl, Pyrazinyl, Pyrimidinyl, Pyridazinyl, Indolyl, Benzimidazolyl, Indazolyl, Chinolyl, Isochinolyl, Phthalazinyl, Chinoxalinyl, Chinazolinyl und Cinnolinyl.

Als Heteroarylreste gelten insbesondere 2- oder 3-Thienyl, 2- oder 3-Furyl, 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3-oder 5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, 2- oder 3-Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also z.B. 1-Oxy-2-, 3- oder 4-pyridyl. Besonders bevorzugt sind die Heteroarylreste 2- oder 3-Thienyl, 2- oder 3-Furyl, 1-, 2- oder 3- Pyrrolyl, 1-, 2-, 4- oder 5-Imidazolyl, 2-, 3-, 4-, 5-, 6-, 7- oder 8-Chinolyl, 1-, 3-, 4- oder 5-Pyrazolyl, 2-, 3- oder 4-Pyridyl, 2- oder 3-Pyrazinyl, 2-, 4-, 5- oder 6-Pyrimidinyl und 3- oder 4-Pyridazinyl.

Gegenstand der Erfindung sind auch die im folgenden beschriebenen Verfahren zur Herstellung der Verbindungen der Formeln I und/oder II.

Die vorliegende Erfindung betrifft ein Verfahren zur Herstellung einer Verbindung der Formeln I oder II und/oder deren pharmazeutisch verträgliche Salze, worin X Sauerstoff ist (Schema 1), **dadurch gekennzeichnet, dass** man
a) ein Phenol der Formeln III oder IV mit einem Aromaten der Formel V zu einer Verbindung der Formeln VIa oder VIIa umsetzt
   und
b) eine Verbindung der Formeln Via oder VIIa mit Guanidin zum Acylguanidin der Formeln la oder IIa umsetzt, 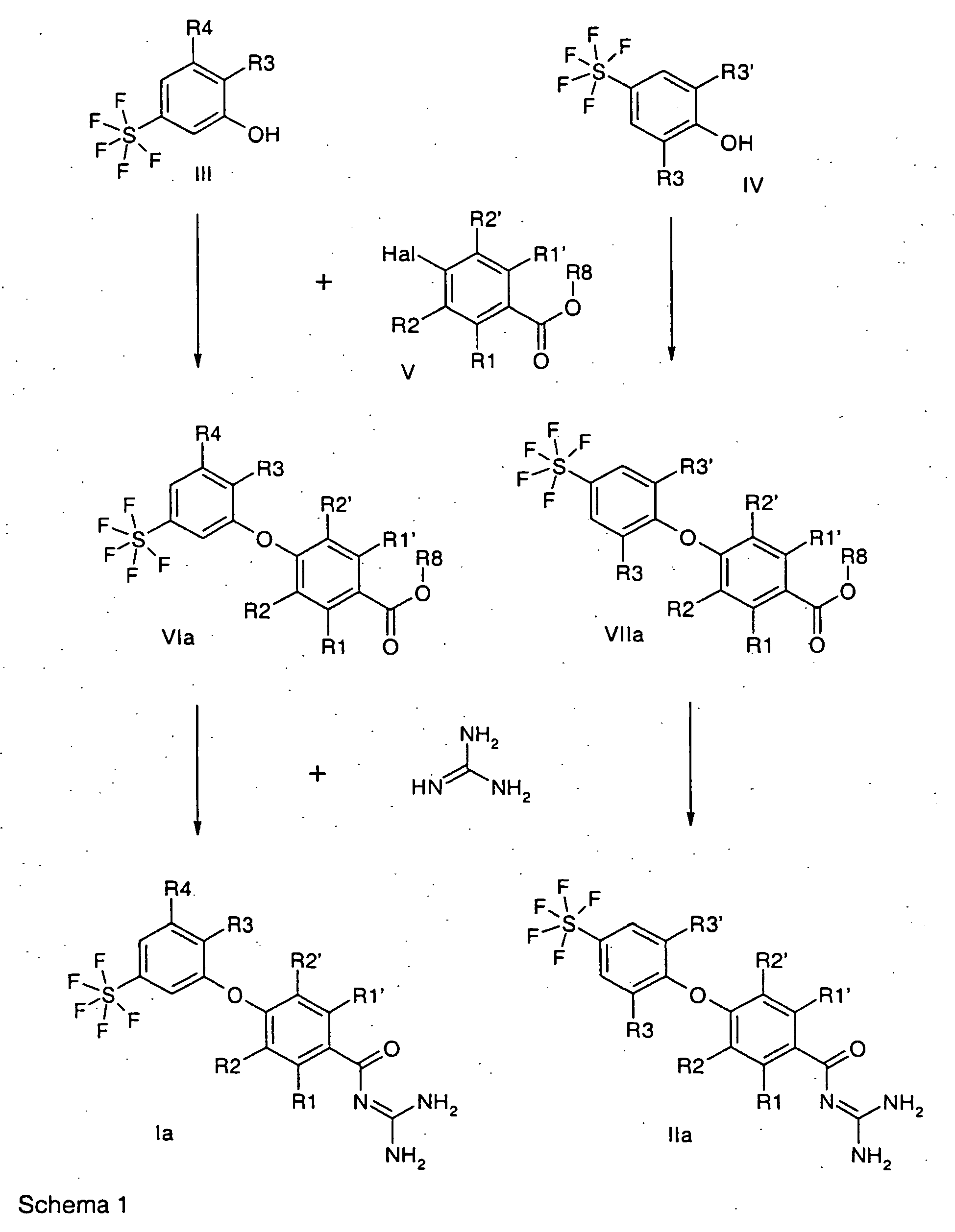
worin R1, R1', R2, R2', R3, R3' und R4 die oben angegebene Bedeutung besitzen und worin bedeuten
- Hal: F, Cl, Br oder I,
- R8: Wasserstoff oder Alkyl mit 1, 2, 3 oder 4 C-Atomen.

Die Phenole der Formeln III oder IV werden in einem geeigneten Lösungsmittel, bevorzugt in einem dipolar aprotischen Lösungsmittel wie zum Beispiel Acetonitril, DMF, NMP oder DMSO, mit Hilfe einer anorganischen Base, wie zum Beispiel K₂CO₃ oder Cs₂CO₃, oder mit Hilfe einer organischen Base, wie zum Beispiel Triethylamin oder TBTMG, bei einer Temperatur zwischen 0°C und dem Siedepunkt des verwendeten Lösungsmittels deprotoniert und dann mit dem elektrophilen Aromaten der Formel V in einer nucleophilen aromatischen Substitution bei einer Temperatur zwischen 0°C und dem Siedepunkt des verwendeten Lösungsmittels, bevorzugt zwischen RT und 150°C, zu Verbindungen der Formeln VIa oder VIIa umgesetzt.

Die Umsetzung der Verbindungen der Formeln VIa oder VIIa zu den Acylguanidinen der Formeln Ia oder IIa erfolgt entweder mit freier Guanidin-Base oder bevorzugt mit Guanidinium-Chlorid, das zunächst zusammen mit KOtBu in einem inerten Lösungsmittel, bevorzugt DMF oder NMP gerührt wird und dann zusammen mit dem Ester bei einer Temperatur zwischen 0°C und dem Siedepunkt des Lösungsmittels, bevorzugt zwischen RT und 100°C, gerührt wird.

Die Ester der Formel VIa oder VIIa, in denen R8 Alkyl ist, können auch zunächst zu den Carbonsäuren verseift und anschließend, vorzugsweise in Gegenwart eines Aktivierungsmittels, mit Guanidin zu den Acylguanidinen der Formeln la oder IIa umgesetzt werden.

Die Ausgangsverbindungen der Formeln III, IV und V sind käuflich erhältlich oder können nach oder analog zu in der Literatur beschriebenen, dem Fachmann bekannten Verfahren hergestellt werden.

Die vorliegende Erfindung betrifft weiterhin ein Verfahren zur Herstellung einer Verbindung der Formel I oder II und/oder deren pharmazeutisch verträglichen Salzen, worin X NR7 ist (Schema 2), **dadurch gekennzeichnet, dass** man
a) ein Anilin der Formeln VIII oder IX mit einem Sulfonsäurechlorid, zum Beispiel Methansulfonsäurechlorid, sulfoniert zu einer Verbindung der Formeln X oder XI,
b) eine Verbindung der Formeln X oder XI mit einem Aromaten der Formel V zu einer Verbindung der Formeln Vlb oder Vllb umsetzt,
c) zur Herstellung einer Verbindung der Formeln Ib oder IIb, in der R7 verschieden von Wasserstoff ist, eine Verbindung der Formeln Vlb oder VIIb zu einer Verbindung der Formeln Vlc oder VIIc derivatisiert,
   und
d) eine Verbindung der Formeln Vlb/c oder VIIb/c mit Guanidin zu einem Acylguanidin der Formeln Ib oder IIb umsetzt, 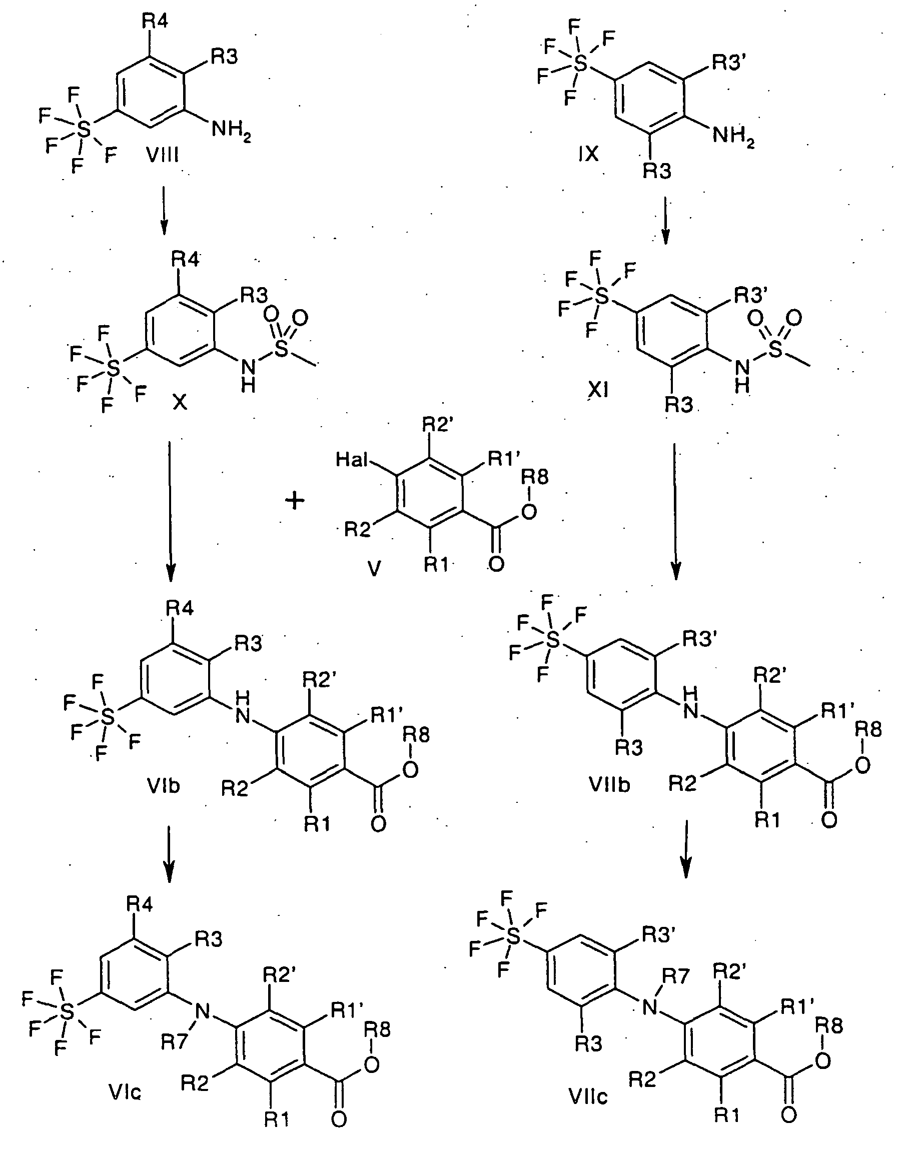 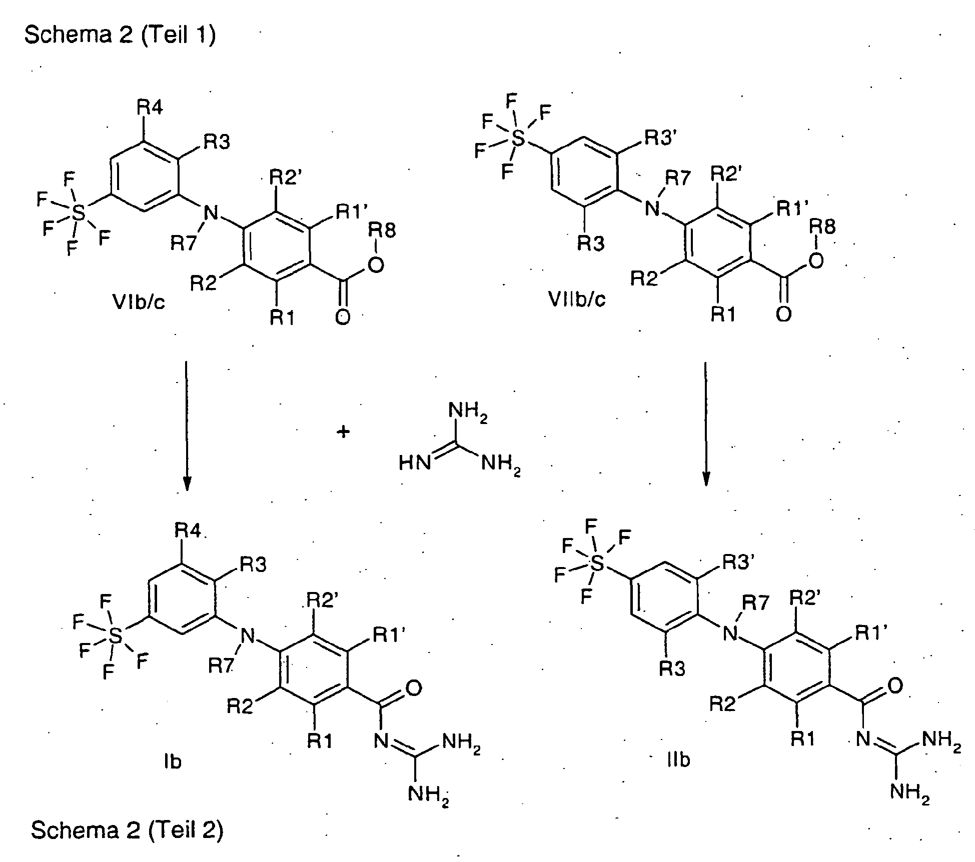
worin R1, R1', R2, R2', R3, R3', R4 und R7 die oben angegebene Bedeutung besitzen und worin bedeuten
- Hal: F, Cl, Br oder I
- R8: Wasserstoff oder Alkyl mit 1, 2, 3 oder 4 C-Atomen.

Die Aniline der Formeln VIII oder IX werden in einem geeigneten Lösungsmittel, bevorzugt einem inerten Lösungsmittel, wie zum Beispiel CH₂Cl₂, DMF oder NMP, mit einem Sulfonsäurechlorid, zum Beispiel Methansulfonsäurechlorid, in Gegenwart einer Base, wie zum Beispiel Triethylamin, bei einer Temperatur zwischen -30°C und 100°C, bevorzugt bei einer Temperatur zwischen 0°C und 60°C sulfoniert. Dabei entstehen entweder die mono-sulfonierten Aniline der Formeln X oder XI oder die entsprechenden bis-sulfonierten Aniline oder ein Gemisch der beiden.

Im Fall der bis-sulfonierten Aniline oder des Gemisches der beiden wird die Abspaltung einer Sulfonyl-Gruppe durch Hydrolyse mit einem geeigneten Nucleophil, bevorzugt einer wäßrigen Alkalilösung, wie zum Beispiel wäßrige NaOH-Lösung, bei einer Temperatur zwischen RT und 120°C durchgeführt.

Die Verbindungen der Formeln X und XI werden in einem geeigneten Lösungsmittel, bevorzugt in einem dipolar aprotischen Lösungsmittel, wie zum Beispiel Acetonitril, DMF, NMP oder DMSO, mit Hilfe einer anorganischen Base, wie zum Beispiel K₂CO₃ oder Cs₂CO₃, oder mit Hilfe einer organischen Base, wie zum Beispiel Triethylamin oder TBTMG, bei einer Temperatur zwischen 0°C und dem Siedepunkt des verwendeten Lösungsmittels deprotoniert und dann mit einem elektrophilen Aromaten der Formel V in einer nucleophilen aromatischen Substitution bei einer Temperatur zwischen 0°C und dem Siedepunkt des verwendeten Lösungsmittels, bevorzugt zwischen RT und 160°C, zu den Verbindungen der Formeln Vlb oder Vllb umgesetzt. Zur optionalen Derivatisierung werden die Verbindungen der allgemeinen Formeln VIb oder Vllb zunächst mit einer anorganischen Base, wie zum Beispiel NaH, oder einer organischen Base, wie zum Beispiel TBTMG, bei einer Temperatur zwischen -30°C und 80°C, bevorzugt bei RT deprotoniert und dann mit einem geeigneten Elektrophil, wie zum Beispiel Methyliodid oder Methansulfonylchlorid, bei einer Temperatur zwischen -30°C und 80°C, bevorzugt bei RT, zu Derivaten der Formeln VIc oder Vllc umgesetzt.

Die Ester der Formeln Vlb/c oder VIIb/c können, wie zum Beispiel für R7 ist Wasserstoff, anschließend zu den Carbonsäuren verseift werden, indem sie in einem geeigneten Lösungsmittel, wie zum Beispiel Methanol oder Ethanol, gelöst werden und eine wäßrige Alkalilösung, zum Beispiel eine wäßrige NaOH-Lösung, zugegeben wird. Das Reaktionsgemisch wird bei -30°C bis zum Siedepunkt des der Lösungsmittels, bevorzugt bei RT, umgesetzt.

Die Carbonsäuren werden zunächst mit einer dem Fachmann bekannten Aktivierungsmethoden, bevorzugt mit CDI, in einem inerten Lösungsmittel, bevorzugt DMF oder NMP, bei einer Temperatur zwischen -30°C und 100°C, bevorzugt bei RT, aktiviert und anschließend mit Guanidin, das bevorzugt aus Guanidiniumchlorid und KOtBu in DMF oder NMP erzeugt wurde, bei einer Temperatur zwischen 0°C und 100°C, bevorzugt zwischen RT und 80°C, zu den Acylguanidinen der Formeln Ib oder IIb umgesetzt.

Weiterhin können die Ester der Formeln VIb/c oder VIIb/c auch direkt wie bei Schema 1 beschrieben mit entweder freier Guanidin-Base oder bevorzugt mit Guanidin-Chlorid in Gegenwart einer Base zu den Acylguanidinen der Formel Ib oder IIb umgesetzt werden.

Die Ausgangsverbindungen der Formeln VIII, IX und V sind käuflich erhältlich oder können nach oder analog zu in der Literatur beschriebenen, dem Fachmann bekannten Verfahren hergestellt werden.

Die vorliegende Erfindung betrifft weiterhin ein Verfahren zur Herstellung einer Verbindung der Formel I oder II und/oder deren pharmazeutisch verträgliche Salze, worin X S(O)ₖₖ ist (Schema 3), **dadurch gekennzeichnet, dass** man
a) ein Thiophenol der Formeln XII oder XIII mit einem Aromaten der Formel V zu einer Verbindung der Formeln VIe oder VIIe umsetzt,
b) zur Herstellung einer Verbindung der Formeln Ic oder IIc, in der kk verschieden von Null ist, eine Verbindung der Formeln VIe oder VIIe zu einer Verbindung der Formeln Vlf oder Vllf oxidiert
   und
c) eine Verbindung der Formeln VIe/f oder VII/ef mit Guanidin zu einem Acylguanidin der Formeln Ic oder IIc umsetzt, 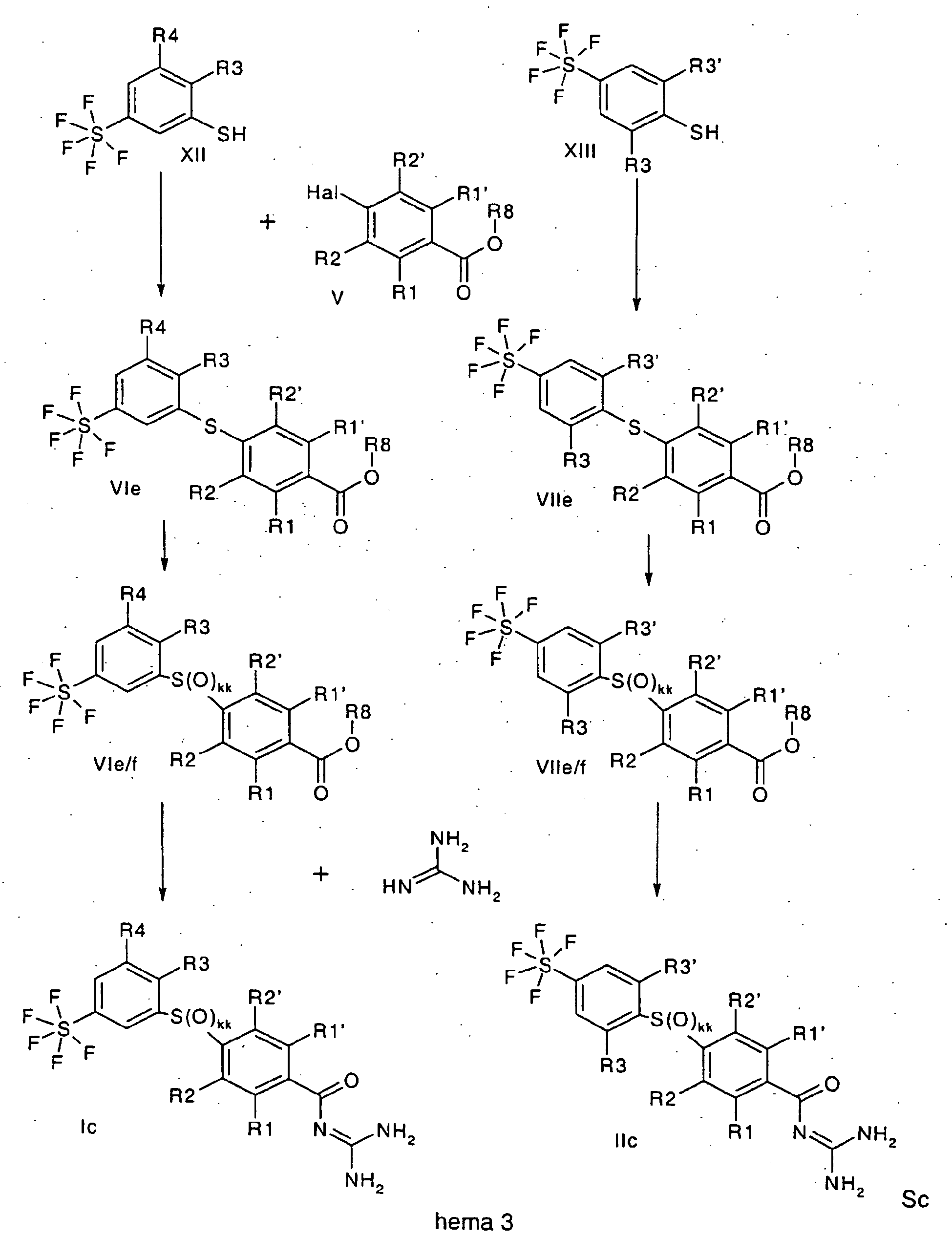 worin R1, R1', R2, R2', R3, R3',R4 und kk die oben angegebene Bedeutung besitzen und worin bedeuten

- Hal: F, Cl, Br oder I
- R8: Wasserstoff oder Alkyl mit 1, 2, 3 oder 4 C-Atomen

Die Thiophenole der Formeln XII oder XIII werden in einem geeigneten Lösungsmittel, bevorzugt in einem dipolar aprotischen Lösungsmittel wie zum Beispiel Acetonitril, DMF, NMP oder DMSO, mit Hilfe einer anorganischen Base, wie zum Beispiel K₂CO₃ oder Cs₂CO₃, oder mit Hilfe einer organischen Base, wie zum Beispiel Triethylamin oder TBTMG, bei einer Temperatur zwischen 0°C und dem Siedepunkt des verwendeten Lösungsmittels deprotoniert und dann mit dem elektrophilen Aromaten der Formel V in einer nucleophilen aromatischen Substitution bei einer Temperatur zwischen 0°C und dem Siedepunkt des verwendeten Lösungsmittels, bevorzugt zwischen RT und 150°C, zu Verbindungen der Formeln Vle oder VIIe umgesetzt.

Im Fall, dass kk Null ist, kann anschließend die Umsetzung der Ester der Formeln VIe oder VIIe mit freier Guanidin-Base oder Guanidinium-Chlorid in Gegenwart einer Base zu den Acylguanidinen der Formeln Ic oder IIc, wie oben in Schema 1 beschrieben, erfolgen.

Zur optionalen Oxidation zu Verbindungen der Formel VIf oder Vllf, in denen kk 1 oder 2 ist, werden die Thioether der Formeln VIe oder VIIe in einem inerten Lösungsmittel wie zum Beispiel CH₂Cl₂ gelöst und mit einem Peroxid-Derivat, bevorzugt mit mCPBA, je nach Stöchiometrie zu den Sulfoxiden oder Sulfonen der Formeln VIf oder VIIf bei einer Temperatur zwischen -30°C und dem Siedepunkt des Lösungsmittels, bevorzugt bei RT, umgesetzt. Die so erhaltenen Ester der Formeln VIf oder VIIf werden wie unter Schema 1 beschrieben mit freier Guanidin-Base oder Guanidinium-Chlorid in Gegenwart einer Base zu den Acylguanidinen der Formeln Ic und IIc umgesetzt.

Die Ester der Formel VIe/f oder VIIe/f, in denen R8 Alkyl ist, können auch zunächst zu den Carbonsäuren verseift und anschließend, vorzugsweise in Gegenwart eines Aktivierungsmittels, mit Guanidin zu den Acylguanidinen der Formeln Ic oder IIc umgesetzt werden.

Die Ausgangsverbindungen der Formeln XII, XIII und V sind käuflich erhältlich oder können nach oder analog zu in der Literatur beschriebenen, dem Fachmann bekannten Verfahren hergestellt werden, zum Beispiel kann die Herstellung der Verbindungen der Formlen XII und XIII analog zu Rundel, Wolfgang; Chem. Ber. (1968), 101(8), 2956) erfolgen.

Die Erfindung betrifft weiterhin ein Verfahren zur Herstellung einer Verbindung der Formel I oder II und/oder deren pharmazeutisch verträgliche Salze, worin X eine direkte Bindung ist (Schema 4), **dadurch gekennzeichnet, dass** man
a) ein Halogenid der Formel XIV oder XV in einer Suzuki-Kopplung mit einem Benzoesäureester der Formel Va zu einem Biphenyl-Derivat der Formel VIg oder VIIg koppelt
   und
b) eine Verbindung der Formel VIg und Vllg mit Guanidin zu einem Acylguanidin der Formel Id oder IId umsetzt, 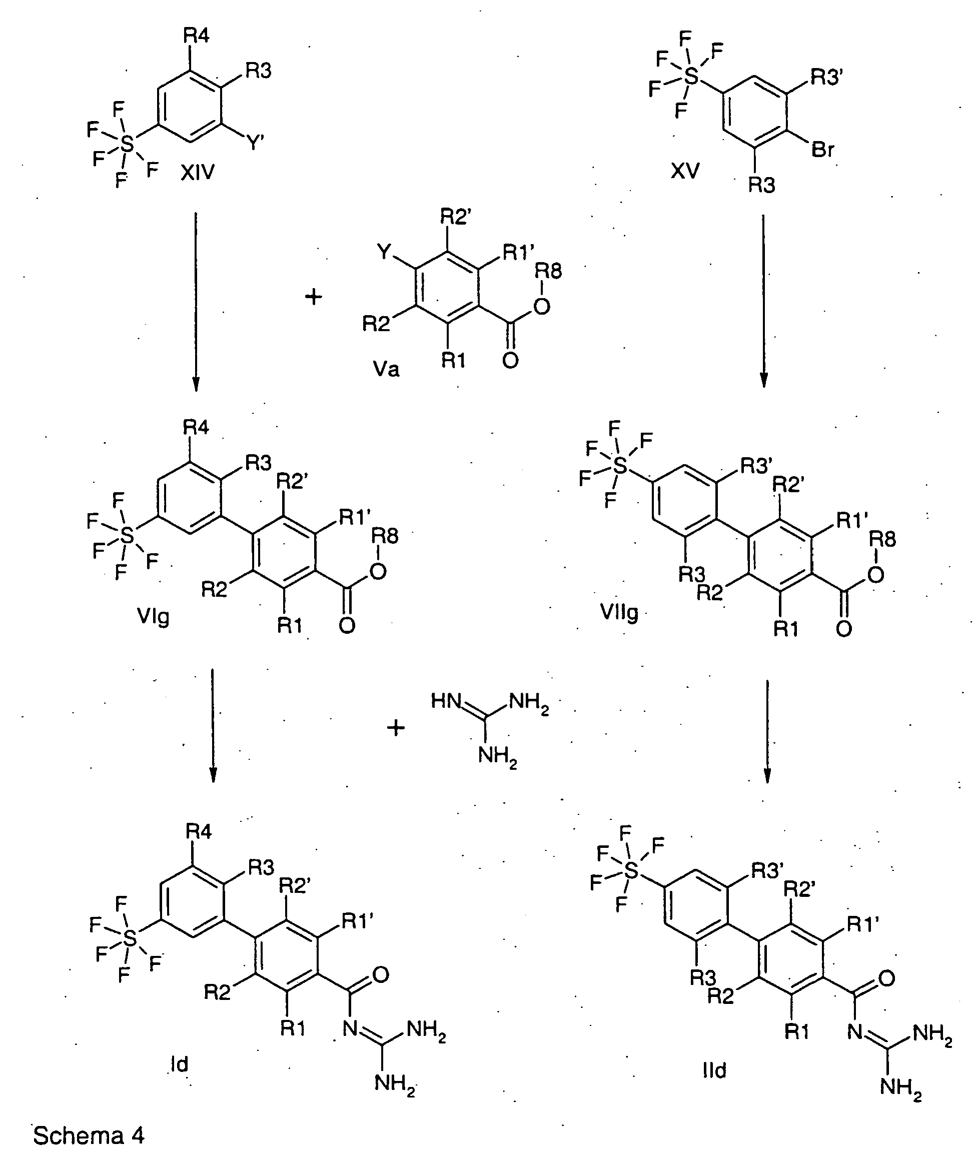
worin R1, R1', R2, R2', R3, R3' und R4 die oben angegebene Bedeutung besitzen und worin bedeuten
- Y und Y': unabhängig voneinander Cl, Br oder I
- R8: Wasserstoff oder Alkyl mit 1, 2, 3 oder 4 C-Atomen.

Die Pentafluorosulfanyl-Arylhalogenide der Formeln XIV oder XV werden in einer Suzuki-Kopplung mit dem Benzoesäureester der Formel Va gekoppelt. Hierzu wird entweder aus den Halogeniden der Formeln XIV oder XV zunächst nach dem Fachmann bekannten Verfahren die entsprechende Arylboronsäure hergestellt. Oder die Arylboronsäure wird zum Beispiel mit Hilfe der Verbindung der Formel XVI 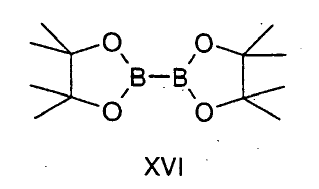 in der Reaktion intermediär hergestellt. Hierzu wird das Pentafluorosulfanyl-Arylhalogenid zusammen mit Bis-(pinacolato)-dibor der Formel XVI und einer Base, wie zum Beispiel K₂CO₃, und einem Katalysator, wie zum Beispiel Pd(dppf)₂, in einem geeigneten Lösungsmittel, bevorzugt DMF oder NMP, bei einer Temperatur zwischen RT und 120°C, bevorzugt bei 60°C-100°C, gerührt. Dies Reaktionsgemisch wird anschließend mit dem Ester der Formel Va bei einer Temperatur zwischen RT und 120°C, bevorzugt zwischen 60°Cund 100°C zu den Biphenyl-Derivaten der Formeln VIg oder Vllg umgesetzt. Die so erhaltenen Ester der allgemeinen Formeln Vlg und VIIg werden, wie unter Schema 1 beschrieben, zu den Acylguanidinen der Formeln Id oder IId umgesetzt.

Die Ausgangsverbindungen der Formeln XIV, XV, XVI und Va sind käuflich erhältlich oder können nach oder analog zu in der Literatur beschriebenen, dem Fachmann bekannten Verfahren hergestellt werden.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Zur Erfindung gehören auch Vorprodukte der Formeln VI und VII und deren Salze, 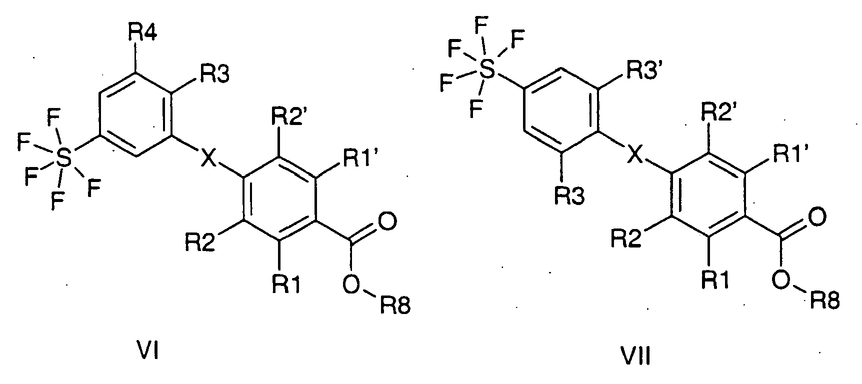 wobei R1, R1', R2, R2', R3, R3', R4 und X die oben genannten Bedeutungen haben und R8 Wasserstoff oder Alkyl mit 1, 2, 3 oder 4 C-Atomen ist, und ihre Verwendung als Syntheseintermediate, zum Beispiel zur Herstellung von Arzneimittelwirkstoffen wie zum Beispiel Verbindungen der Formel I oder II und/oder deren pharmazeutisch verträgliche Salze.

Pentafluorosulfanylphenyl-substituierte Benzoylguanidine der Formeln I und II sind im allgemeinen schwache Basen und können Säure unter Bildung von Salzen binden. Als Säureadditionssalze kommen insbesondere Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen der Formeln I und II sind substituierte Acylguanidine und inhibieren den zellulären Natrium-Protonen-Antiporter (Na⁺/H⁺-Exchanger, NHE), insbesondere den Subtyp NHE1.

Gegenüber bekannten NHE-Inhibitoren zeichnen sich die erfindungsgemäßen Verbindungen durch eine außerordentlich hohe Wirksamkeit in der Inhibition des Na⁺/H⁺-Austauschs aus, sowie durch verbesserte ADMET-Eigenschaften aus, zum Beispiel durch längere S9-Stabilitäten (Leberstabilitäten, Stabilität gegenüber enzymatischem Angriff) und längere Halbwertszeiten in vivo. Dabei weisen sie ein gutes Resorptionsverhalten und eine hohe Bioverfügbarkeit auf.

Aufgrund der NHE-inhibitorischen Eigenschaften eignen sich die Verbindungen der Formel I und II und deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHEbedingten Schädigungen verursacht werden.

Da NHE Inhibitoren in überwiegender Weise über ihre Beeinflussung der zellulären pH-Regulation wirken, können diese generell in günstiger Weise mit anderen, den intrazellulären pH-Wert regulierenden Verbindungen kombiniert werden, wobei Inhibitoren der Enzymgruppe der Carboanhydratasen, Inhibitoren der Bicarbonatiorien transportierenden Systeme wie des Natrium-Bicarbonat-Cotransporters (NBC) oder des Natrium abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

So eignen sich die erfindungsgemäßen Inhibitoren des NHE zur Behandlung von Krankheiten, die durch Ischämie und durch Reperfusion hervorgerufen werden.

Die hier beschriebenen Verbindungen sind infolge ihrer pharmakologischen Eigenschaften als antiarrhythmische Arzneimittel geeignet.

Durch ihre cardioprotektive Komponente eignen sich die NHE-Inhibitoren der Formel I und II und deren pharmazeutisch verträgliche Salze hervorragend zur Infarktprophylaxe und der Infarktbehandlung sowie zur Behandlung der Angina Pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I und II und deren pharmazeutisch verträgliche Salze infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die erfindungsgemäßen Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Es zeigte sich, dass die erfindungsgemäßen Verbindungen außerordentlich wirksame Arzneimittel sind gegen lebensbedrohliche Arrhythmien. Kammerflimmern wird beendet und der physiologische Sinus-Rhythmus des Herzens wiederhergestellt.

Da NHE1-Inhibitoren menschliches Gewebe und Organe, insbesondere das Herz, nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist die kombinierte Verabreichung mit Verbindungen der Formel I und II und deren pharmazeutisch verträgliche Salze geeignet, die cytotoxischen, insbesondere cardiotoxischen Nebenwirkungen der genannten Verbindungen zu inhibieren. Durch die Verminderung der cytotoxischen Effekte, insbesondere der Cardiotoxizität, infolge Ko-Medikation mit NHE1-Inhibitoren kann außerdem die Dosis der cytotoxischen Therapeutika erhöht und/oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Außerdem können die erfindungsgemäßen NHE1-Inhibitoren der Formel I und II und deren pharmazeutisch verträgliche Salze bei einer herzschädigenden Überproduktion von Schilddrüsenhormonen, der Thyreotoxikose, oder bei der externen Zufuhr von Schilddrüsenhormonen Verwendung finden. Die Verbindungen der Formel I und II und deren pharmazeutisch verträgliche Salze eignen sich somit zur Verbesserung der Therapie mit cardiotoxischen Arzneimitteln.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie zum Beispiel zur Behandlung des Schlaganfalls oder des Hirnödems geeignet sind.

Die Verbindungen der Formel I und II und deren pharmazeutisch verträgliche Salze eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychischer Depressionszustände, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäß verwendeten Verbindungen der Formel I und II und deren pharmazeutisch verträgliche Salze ebenfalls zur Behandlung von Formen des Schocks, wie beispielweise des allergischen, cardiogenen, hypovolämischen und des bakteriellen Schocks.

Die Verbindungen der Formel I und II und deren pharmazeutisch verträgliche Salze können ebenfalls zur Prävention und zur Behandlung thrombotischer Erkrankungen verwendet werden, da sie als NHE-Inhibitoren die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand-Faktors und thrombogener Selectin-Proteine, hemmen bzw. verhindern. Damit kann die pathogene Wirkung bedeutender thrombogener Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichem tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-Rezeptor-Antagonisten, Phosphodiesterase-Inhibitoren, Factor-VIIa-Antagonisten, Clopidogrel, Ticolopidin usw. Eine kombinierte Anwendung der vorliegenden NHE-Inhibitoren mit NCBE-Inhibitoren und/oder mit Inhibitoren der Carboanhydratase, wie beispielsweise mit Acetazolamid, ist besonders günstig.

Darüber hinaus zeichnen sich die erfindungsgemäß verwendeten Verbindungen der Formel I und II und deren pharmazeutisch verträgliche Salze durch starke inhibierende Wirkung auf die Proliferation von Zellen, beispielsweise der Fibroblasten-Zellproliferation und der Proliferation der glatten Gefäßmuskelzellen, aus. Deshalb kommen die Verbindungen der Formel I und II und deren pharmazeutisch verträgliche Salze als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden.

Es konnte gezeigt werden, dass durch NHE-Inhibitoren die Zellmigration inhibiert wird. Daher kommen die Verbindungen der Formel I und II und deren pharmazeutisch verträgliche Salze als wertvolle Therapeutika für Krankheiten in Frage, bei denen die Zellmigration eine primäre oder sekundäre Ursache darstellt, wie beispielsweise Krebserkrankungen mit ausgeprägter Neigung zur Metastasierung.

Die Verbindungen der Formel I und II und deren pharmazeutisch verträgliche Salze zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen. Sie können somit zur Behandlung von Organhypertrophien und -hyperplasien, beispielsweise des Herzens und der Prostata verwendet werden. Sie sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Da der NHE bei essentiellen Hypertonikern signifikant erhöht ist, eignen sich die Verbindungen der Formel I und II und deren pharmazeutisch verträgliche Salze zur Prävention und zur Behandlung des Bluthochdrucks und zur Behandlung von Herz-Kreislauferkrankungen. Dabei können sie allein oder mit einem geeigneten Kombinations- und Formulierungspartner zur Bluthochdruckbehandlung und zur Behandlung von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin- Rezeptoragonisten, Inhibitoren und Aktivatoren von Kaliumkanälen wie Glibenclamid, Glimepirid, Diazoxid, Cromokalim, Minoxidil und deren Derivate, Aktivatoren des mitochondrialen ATP-sensitiven Kaliumkanals (mitoK(ATP) Kanal), Inhibitoren des Kv1.5 usw.

Es zeigte sich, dass NHE1-Inhibitoren eine signifikante antiphlogistische Wirkung haben und somit als Antiinftammatorika verwendet werden können. Dabei fällt die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet.

Weiterhin können NHE1 Inhibitoren zur Behandlung von Erkrankungen, die durch Protozoen verursacht werden, von Malaria oder der Hühnercoccidose, verwendet werden.

Es wurde außerdem gefunden, dass NHE1 Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL- und LDL-Serumcholesterinfraktionen zu senken. Es wurde nun gefunden, dass NHE1-Inhibitoren bezüglich der Beeinflussung der Serumlipidspiegel wertvolle therapeutisch verwertbare Eigenschaften zeigen. So erniedrigen sie signifikant die erhöhte Serum Konzentration von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien, zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie zum Beispiel beim Diabetes vorkommen. Darüber hinaus führen die Verbindungen der Formel I und II und deren pharmazeutisch verträgliche Salze zu einer deutlichen Reduktion der durch Stoffwechselanomalien induzierten Infarkte und insbesondere zu einer signifikanten Verminderung der induzierten Infarktgröße und dessen Schweregrades.

Die genannten Verbindungen finden deshalb vorteilhaft Verwendung zur Herstellung eines Medikaments zur Behandlung von Hypercholesterinämie, zur Herstellung eines Medikaments zur Prävention der Atherogenese, zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion-induzierter ischämischer Schäden und postischämischer Reperfusionsschäden, zur Herstellung eines Medikaments zur Prävention und Behandlung von Hypercholesterinämie- und endothelialer Dysfunktion-induzierter kardialer Hypertrophien und Cardiomyopathien und der Herzinsuffizienz (CHF), zur Herstellung eines Medikaments zur Prävention und Behandlung von Hypercholesterinämie- und endothelialer Dysfunktion-induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzym (ACE)-Hemmern und Angiotensin-Rezeptorantagonisten. Eine Kombination eines NHE-Inhibitors der Formel I und II und deren pharmazeutisch verträgliche Salze mit einem blutfettspiegelsenkenden Wirkstoff, bevorzugt mit einem HMG-CoA-Reduktaseinhibitor (zum Beispiel Lovastatin oder Pravastatin), wobei letzterer eine hypolipidämische Wirkung herbeiführt und dadurch die hypolipidämischen Eigenschaften des NHE-Inhibitors der Formel I und II und deren pharmazeutisch verträgliche Salze steigert, erweist sich als günstige Kombination mit verstärkter Wirkung und vermindertem Wirkstoffeinsatz.

So führen Verbindungen der Formel I und II und deren pharmazeutisch verträgliche Salze zu einen wirksamen Schutz gegen Endothelschädigungen unterschiedlicher Genese. Mit diesem Schutz der Gefäße gegen das Syndrom der endothelialen Dysfunktion sind Verbindungen der Formel I und II und deren pharmazeutisch verträgliche Salze wertvolle Arzneimittel zur Prävention und zur Behandlung koronarer Gefäßspasmen, peripherer Gefäßkrankheiten; insbesondere von claudicatio intermittens, der Atherogenese und der Atherosklerose, der linksventrikulären Hypertrophie und der dilatierten Kardiomyopathie, und thrombotischer Erkrankungen.

Außerdem wurde gefunden, dass Benzoylguanidine der Formel I und II und deren pharmazeutisch verträgliche Salze geeignet in der Behandlung des nichtinsulinabhängigen Diabetes (NIDDM) sind, wobei die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR-Agonisten, wie Rosiglitazon, Pioglitazon etc., mit einem Insulinpräparat unterschiedlicher Verabreichungsform, mit einem DB4-Inhibitor, mit einem Insulinsensitizer oder mit Meglitinid zu kombinieren.

Neben den akuten antidiabetischen Effekten wirken die Verbindungen der Formel I und II und deren pharmazeutisch verträgliche Salze der Entstehung diabetischer Spätkomplikationen entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den soeben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

Die erfindungsgemäßen NHE-Inhibitoren der Formel I und II und deren pharmazeutisch verträgliche Salze zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden. Alterungsprozesses zusammenhängen und die unabhängig von akuten Mangeldurchblutungszuständen sind und bei normalen, nicht-ischämischen Bedingungen auftreten. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.

Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. Die Verbindungen der Formel I und II und deren pharmazeutisch verträgliche Salze eignen sich somit hervorragend zur Behandlung und Prävention der altersbedingt fortschreitenden endothelialen Dysfunktion, insbesondere von claudicatio intermittens.

Beispiel einer weiteren den Altersprozess charakterisierenden Messgröße ist die Abnahme der Kontraktilität des Herzens und die Abnahme der Anpassung des Herzens an eine geforderte Pumpleistung. Diese verminderte Herzleistungsfähigkeit als Folge des Alterungsprozesses ist in den meisten Fällen verbunden mit einer Dysfunktion des Herzens, die unter anderem durch eine Einlagerung von Bindegewebe ins Herzgewebe verursacht wird. Diese Bindegewebseinlagerung ist gekennzeichnet durch eine Zunahme des Herzgewichtes, durch eine Vergrößerung des Herzens und durch eine eingeschränkte Herzfunktion. Es war überraschend, dass eine derartige Alterung des Organs Herz nahezu komplett inhibiert werden konnte. Die Verbindungen der Formel I und II und deren pharmazeutisch verträgliche Salze eignen sich somit hervorragend zur Behandlung und Prävention der Herzinsuffizienz, des congestive heart failure (CHF).

Während die Behandlung von unterschiedlichen, bereits eingetretenen Formen von Krebserkrankungen bereits bekannt ist, war es nun außerordentlich überraschend, dass nicht nur die bereits eingetretene Krebserkrankung durch Proliferationshemmung geheilt werden kann, sondern dass auch die altersbedingte Entstehungshäufigkeit von Krebs durch NHE-Inhibitoren verhindert und hochsignifikant verzögert wird. Besonders bemerkenswert ist der Befund, dass altersbedingt auftretenden Erkrankungen aller Organe und nicht nur bestimmter Krebsformen unterbunden bzw. hochsignifikant verzögert auftreten. Die Verbindungen der Formel I und II und deren pharmazeutische verträgliche Salze eignen sich somit hervorragend zur Behandlung und insbesondere der Prävention von altersbedingten Formen von Krebs.

Mit NHE1-inhibitoren wird nun nicht nur eine über das statistische Normalmaß hinaus, zeitlich hochsignifikant verschobene Verzögerung des Eintretens altersbedingter Erkrankungen aller untersuchten Organe einschließlich Herz, Gefäße, Leber usw., sowie eine hochsignifikante Verzögerung von Alterskrebs festgestellt. Vielmehr kommt es auch überraschenderweise zu einer Lebensverlängerung in einem Ausmaß, das bislang durch keine andere Medikamentengruppe bzw. durch irgendwelche Naturstoffe erreicht werden konnte. Diese einzigartige Wirkung der NHE-Inhibitoren ermöglicht es auch, neben der alleinigen Wirkstoffanwendung an Mensch und Tier diese NHE-Inhibitoren mit anderen gerontologisch verwendeten Wirkprinzipien, Maßnahmen, Substanzen und Naturstoffen zu kombinieren, denen ein anderer Wirkmechanismus zugrunde liegt. Derartige in der gerontologischen Therapie verwendete Wirkstoffklassen sind insbesondere Vitamine und antioxidativ wirksame Stoffe. Da eine Korrelation zwischen kalorischer Belastung bzw. Nahrungsaufnahme und Alterungsprozeß besteht, kann die Kombination mit diätetischen Maßnahmen, zum Beispiel mit Appetitszüglern, erfolgen. Ebenso kann eine Kombination mit blutdrucksenkenden Medikamenten, wie mit ACE-Hemmern, Angiotensinrezeptorantagonisten, Diuretika, Ca²⁺-Antagonisten etc. oder mit stoffwechselnormalisierenden Medikamenten, wie Cholesterinsenkern, erfolgen.

Die Verbindungen der Formel I und II und deren pharmazeutisch verträgliche Salze eignen sich somit hervorragend zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise zur Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Beansprucht wird weiterhin ein Heilmittel für die humane, veterinäre oder phytoprotektive Anwendung, enthaltend eine wirksame Menge einer Verbindung der Formel I und II und deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.

Arzneimittel, die eine Verbindung der Formel I und II und deren pharmazeutisch verträgliche Salze enthalten, können dabei zum Beispiel oral, parenteral, intravenös, rektal, perkutan oder durch Inhalation appliziert werden, wobei die bevorzugte Applikation von dem jeweiligen Erscheinungsbild der Erkrankung abhängig ist. Die Verbindungen der Formel I und II können dabei allein oder zusammen mit galenischen Hilfsstoffen zur Anwendung kommen, und zwar sowohl in der Veterinär- als auch in der Humanmedizin. Die Arzneimittel enthalten Wirkstoffe der Formel I und II und deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, intramuskulären oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage: Wasser, physiologische Kochsalzlösung oder Alkohole, zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen des Wirkstoffes der Formel I und II und deren pharmazeutisch verträgliche Salze in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und II und die Häufigkeit der Verabreichung hängen von der Wirkstärke und Wirkdauer der verwendeten Verbindungen ab, außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I und/oder II und/oder deren pharmazeutisch verträgliche Salze bei einem etwa 75 kg schweren Patienten mindestens 0,001 mg/kg, beispielsweise 0,01 mg/kg, bis höchstens 10 mg/kg, beispielsweise 1 mg/kg Körpergewicht. Bei akuten Ausbrüchen der Krankheit, etwa unmittelbar nach Erleiden eines Herzinfarkts, können auch noch höhere Dosierungen notwendig sein, zum Beispiel bis zu 4 Einzeldosen pro Tag. Insbesondere bei i.v. Anwendung, etwa bei einem Infarktpatienten auf der Intensivstation können bis zu 800 mg pro Tag notwendig werden. Die tägliche Dosis kann auf mehrere, zum Beispiel bis zu 4 Einzeldosen, verteilt werden.

### Liste der Abkürzungen:

| | |
|---|---|
| ADMET | Adsorption - Distribution - Metabolismus - Ausscheidung - Toxikologie |
| Bis-(pinacolato)-dibor | 4,4,4',4',5,5,5',5',-Octamethyl-2,2'-bi-1,3,2-dioxaborolan |
| CDI | Di-imidazol-1-yl-methanon |
| DIP | Diisopropylether |
| DIPEA | Diisopropylethylamin |
| DME | 1,2-Dimethoxyethan |
| DMF | N,N-Dimethylformamid |
| DMSO | Dimethylsulfoxid |
| EE | Ethylacetat (EtOAc) |
| eq. | Äquivalent |
| HEP | n-Heptan |
| HOAc | Essigsäure |
| KOtBu | Kalium-2-methyl-propan-2-olat |
| MeOH | Methanol |
| mp | Schmelzpunkt |
| mCPBA | 3-Chlor-perbenzoesäure |
| MTB | tert.-Butyl-methylether |
| NMP | 1-Methylpyrrolin-2-on |
| Pd(dppf)2 | [1,1'-Bis-(diphenylphosphino)-ferrocen]palladium(II)-chlorid-Methylenchlorid-Komplex (1:1) |
| RT | Raumtemperatur |
| TBTMG | N"-tert-Butyl-N,N,N',N'-tetramethyl-guanidin |
| THF | Tetrahydrofuran |
| TMEDA | N,N,N',N'-Tetramethyl-ethan-1,2-diamin |

### Beispiel 1: N-[5-Methansulfonyl-2-methyl-4-(3-pentafluorosulfanyl-phenoxy)-benzoyl]-guanidin

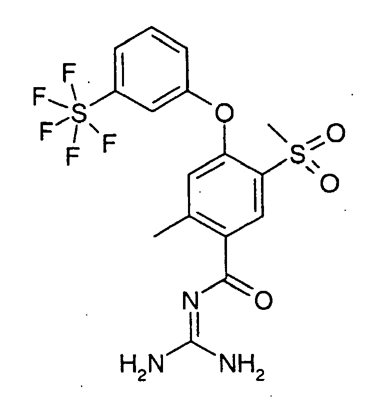

a) 3-Pentafluorosulfanyl-phenol 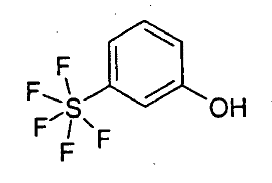
5.0 g 3-Pentafluorosulfanyl-anilin wurden in 50 ml einer 35% wäßrigen H₂SO₄-Lösung suspendiert. Bei 0°C wurde dann eine Lösung von 1.57 g NaNO₂ in 5 ml Wasser innerhalb 10 Minuten zugetropft. 40 Minuten lang wurde bei 0°C nachgerührt. Zu dieser Suspension wurde dann eine auf 0°C gekühlte Lösung von 8.56 g Cu(NO₃)₂ in 50 ml Wasser zugegeben. Direkt danach wurde noch 3.26 g Cu₂O zugegeben, wobei deutliche Gasentwicklung zu beobachten war. 3 mal wurde mit je 100 ml CH₂Cl₂ extrahiert, die org. Phase mit 100 ml einer gesättigten wäßrigen NaCl-Lsg. gewaschen, über MgSO₄ getrocknet und das Solvens im Vakuum entfernt. Chromatographie mit DIP an einer kurzen Kieselgelsäule lieferte 3.5 g des Phenols als farbloses Öl.

| | |
|---|---|
| R_{f} (EE/HEP 1:10) = 0.15 | MS (EI): 220 |

b) 5-Methansulfonyl-2-methy)-4-(3-pentafluorosulfanyl-phenoxy)-benzoesäure-methylester 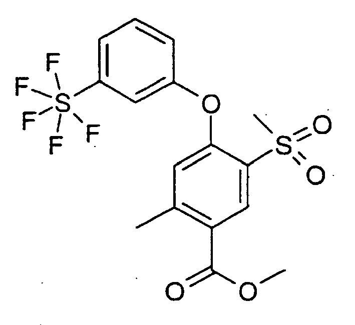
600 mg 4-Fluor-5-methansulfonyl-2-methyl-benzoesäure-methylester, 700 mg 3-Pentafluorosulfanyl-phenol sowie 1.6 g Cs₂CO₃ wurden in 4 ml wasserfreiem DMF 3 h bei 100°C gerührt. Dann wurde auf RT abgekühlt, mit 100 ml EE verdünnt und 3 mal mit je 20 ml Wasser gewaschen. Über MgSO₄ wurde getrocknet, das Solvens im Vakuum entfernt und der Rückstand an Kieselgel mit DIP chromatographiert. Man erhielt 300 mg eines farblosen Öls.

| | |
|---|---|
| R_{f} (DIP) = 0.27 | MS (ES⁺): 446 |

c) N-[5-Methansulfonyl-2-methyl-4-(3-pentafluorosulfanyl-phenoxy)-benzoyl]-guanidin 385 mg Guanidinium-chlorid wurden zusammen mit 377 mg KOtBu 30 Minuten in 10 ml wasserfreiem DMF bei RT gerührt. Dann wurde diese Suspension zu einer Lösung von 300 mg 5-Methansulfonyl-2-methyl-4-(3-pentafluorosulfanyl-phenoxy)-benzoesäure-methylester in 5 ml wasserfreiem DMF gegeben und 5 h bei RT stehen gelassen. Mit 30 ml EE wurde verdünnt und 3 mal mit je 5 ml Wasser gewaschen, über MgSO₄ wurde getrocknet, das Solvens im Vakuum entfernt und der Rückstand an Kieselgel mit EE/MeOH 10:1 1 chromatographiert. Man erhielt 200 mg eines farblosen amorphen Feststoffs. Umkristallisation aus DIP/MTB 3:1 lieferte farblose Kristalle mit mp = 166-168°C.

| | |
|---|---|
| R_{f} (EE/MeOH 10:1) = 0.26 | MS (ES⁺) : 473 |

### Beispiel 2: N-[5-Methansulfonyl-2-methyl-4-(4-pentafluorosulfanyl-phenoxy)-benzoyl]-guanidin

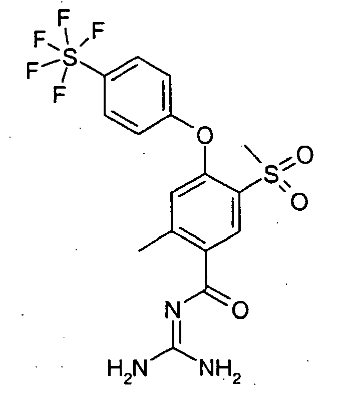

Die Titelverbindung des Beispiels 2 wurde analog Beispiel 1 synthetisiert.

| | |
|---|---|
| R_{f} (EE/MeOH 10:1) = 0.26 | MS (ES⁺) : 473 |

### Beispiel 3: N-[5-Methansulfonyl-2-methyl-4-(4-pentafluorosulfanyl-phenylsulfanyl)-benzoyl]-guanidin

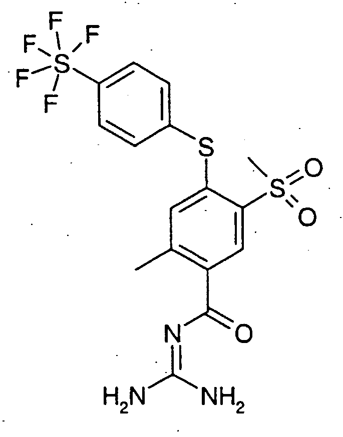

a) 4-Pentafluorosulfanyl-thiophenol 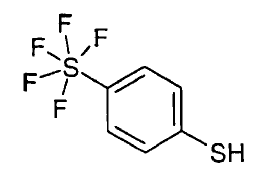
3.0 g 4-Pentafluorosulfanyl-anilin wurden in 10 ml Essigsäure gelöst und zu 30 ml einer 35% wäßrigen H₂SO₄-Lösung zugegeben. Bei 0°C wurde dann eine Lösung von 1.0 g NaNO₂ in 8 ml Wasser zugegeben und 10 Minuten bei 0°C gerührt. Die so erhaltene Lösung des Diazoniumsalzes wurde zu einer 50-60°C warmen Lösung von Na₂S₂ (hergestellt durch Auflösen von 0.61 g Schwefel mit 4.4 g Na₂S und 1.0 g NaOH in 30 ml Wasser) langsam zugegeben. 30 Minuten wurde bei 60°C nachgerührt. Nach Abkühlung wurde mit 300 ml Diethylether extrahiert und anschließend mit 100 ml einer 10% wäßriger HCl-Lösung gewaschen. Über MgSO₄ wurde getrocknet, das Solvens im Vakuum entfernt und der Rückstand in 100 ml wasserfreiem Diethylether aufgenommen. Zu dieser Lösung wurde bei 0°C portionsweise 0.52 g LiAlH₄ gegeben und anschließend 1 h bei RT gerührt. Dann wurde das Reaktionsgemisch vorsichtig zu 100 ml einer 10% wäßrigen HCl-Lösung gegeben und die Etherphase mit 100 ml einer gesättigten wäßrigen NaCl-Lösung gewaschen. Über MgSO₄ wurde getrocknet, das Solvens im Vakuum entfernt und der Rückstand an Kieselgel mit HEP/DIP 1:1 chromatographiert. Man erhielt 280 mg eines farblosen Öls.
R_{f} (DIP/HEP 1:1) = 0.73
b) 5-Methansulfonyl-2-methyl-4-(4-pentafluorosulfanyl-phenylsulfanyl)-benzoesäure-methylester 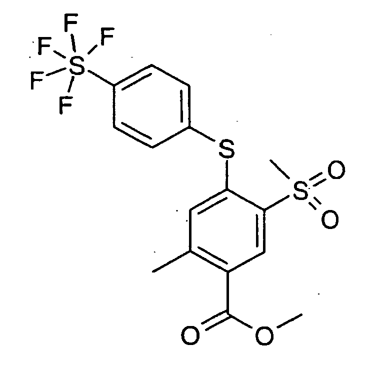
0.28 g 4-Pentafluorosulfanyl-thiophenol, 0.36 g 4-Bromo-5-methansulfonyl-2-methyl-benzoesäure-methylester sowie 0.33 g K₂CO₃ wurden in 8 ml wasserfreiem DMF gelöst und 2 h bei 110°C gerührt. Dann wurde das Reaktionsgemisch auf 100 ml Wasser gegossen und 2 mal mit je 100 ml EE extrahiert. Die EE-Phase wurde anschließend noch 2 mal mit je 30 ml Wasser gewaschen. Über MgSO₄ wurde getrocknet, das Solvens im Vakuum entfernt und der Rückstand an Kieselgel mit CH₂Cl₂/DIP 1:2 chromatographiert. Man erhielt 320 mg eines farblosen Schaumes. R_{f} (CH₂Cl₂/DIP 1:2) = 0.85
c) N-[5-Methansulfonyl-2-methyl-4-(4-pentafluorosulfanyl-phenylsulfanyl)-benzoyl]-guanidin
41 mg KOtBu und 42 mg Guanidinium-Chlorid wurden in 1 ml wasserfreiem DMF 30 Minuten bei RT gerührt. Dann wurde eine Lösung aus 34 mg 5-Methansulfonyl-2-methyl-4-(4-pentafluorosulfanyl-phenylsulfanyl)-benzoesäure-methylester in 1 ml wasserfreiem DMF zugegeben. 5 h wurde bei RT gerührt, anschließend mit 10 ml Wasser verdünnt, mit wäßriger HCl-Lösung auf pH=8 eingestellt und das Produkt abgesaugt. Im Vakuum bei RT wurde getrocknet und man erhielt 35 mg eines amorphen Feststoffs.

| | |
|---|---|
| R_{f} (EE) = 0.16 | MS (ES⁺) : 490 |

### Beispiel 4: N-[5-Methansulfonyl-2-methyl-4-(4-pentafluorosulfanyl-phenylsulfonyl)-benzoyl]-guanidin

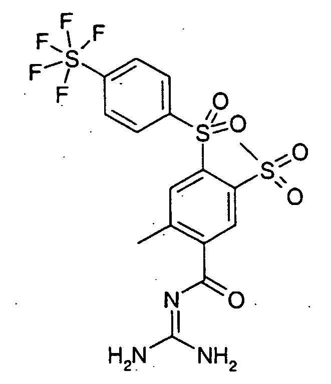

a) 5-Methansulfonyl-2-methyl-4-(4-pentafluorosulfanyl-phenylsulfonyl)-benzoesäure-methylester 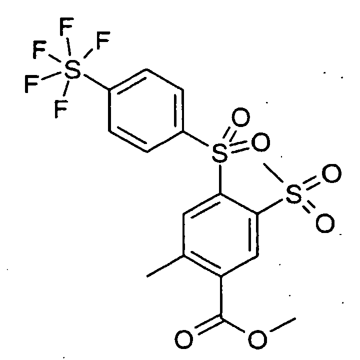
100 mg 5-Methansulfonyl-2-methyl-4-(4-pentafluorosulfanyl-phenylsulfanyl)-benzoesäure-methylester (Beispiel 3b) wurden in 5 ml CH₂Cl₂ gelöst und bei RT mit 136 mg mCPBA versetzt. 20 h wurde bei RT gerührt, anschließend weitere 50 mg mCPBA zugegeben und 1 h bei RT gerührt. Mit 100 ml CH₂Cl₂ wurde verdünnt und anschließend zunächst 2 mal mit je 10 ml einer gesättigten wäßrigen Na₂SO₃-Lösung, dann 2 mal mit je 10 ml einer gesättigten wäßrigen Na₂CO₃-Lösung gewaschen. Über MgSO₄ wurde getrocknet und das Solvens im Vakuum entfernt. Man erhielt 100 mg eines farblosen Schaumes.
R_{f} (CH₂Cl₂) = 0.29
b) N-[5-Methansulfonyl-2-methyl-4-(4-pentafluorosulfanyl-phenylsulfonyl)-benzoyl]-guanidin
114 mg KOtBu und 116 mg Guanidinium-Chlorid wurden in 5 ml wasserfreiem DMF 30 Minuten bei RT gerührt. Dann wurde eine Lösung aus 100 mg 5-Methansulfonyl-2-methyl-4-(4-pentafluorosulfanyl-phenylsulfonyl)-benzoesäure-methylester in 5 ml wasserfreiem DMF zugegeben. 15 h wurde bei RT gerührt, anschließend mit 15 ml Wasser verdünnt, mit wäßriger HCl-Lösung auf pH=8 eingestellt und das Produkt abgesaugt. Im Vakuum bei RT wurde getrocknet und man erhielt 30 mg eines amorphen Feststoffs.

| | |
|---|---|
| R_{f} (EE) = 0.14 | MS (ES+): 1043 (2M+H)⁺ |

### Beispiel 5: N-[5-Methansulfonyl-2-methyl-4-(3-pentafluorosulfanyl-phenylamino)-benzoyl]-guanidin

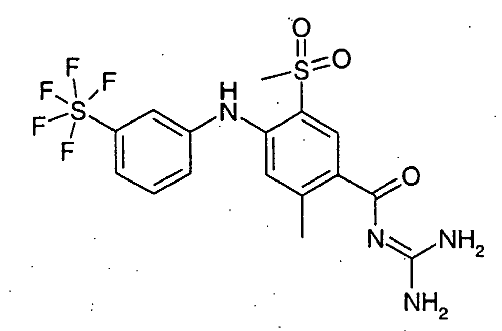

a) N-Methansulfonyl-(3-pentafluorosulfanyl-phenyl)-methansulfonamid 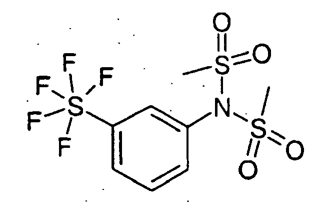
1.5 g 3-Pentafluorosulfanyl-anilin wurden in 100 ml CH₂Cl₂ gelöst und mit 2.8 ml Triethylamin versetzt. Dann wurden 1.6 ml Methansulfonylchlorid langsam bei RT zugetropft. 2 Tage wurde bei RT stehen gelassen, dann die flüchtigen Bestandteile im Vakuum entfernt. Der Rückstand wurde mit 200 ml EE aufgenommen und 3 mal mit je 50 ml einer 10% wäßrigen NaHSO₄-Lösung gewaschen. Über MgSO₄ wurde getrocknet und das Solvens im Vakuum entfernt. Man erhielt 2.3 g eines farblosen Öls.
MS (EI) : 375
b) N-(3-Pentafluorosulfanyl-phenyl)-methansulfonamid 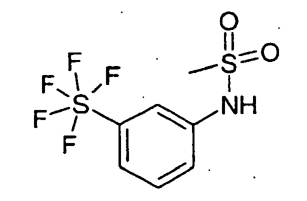
2.3 g N-Methansulfonyl-(3-pentafluorosulfanyl-phenyl)-methansulfonamid wurden in 30 ml MeOH gelöst, 8 ml einer 2 N wäßrigen NaOH-Lösung addiert und 3 h unter Rückfluß erhitzt. Nach Abkühlung wurden die flüchtigen Bestandteile im Vakuum entfernt, der Rückstand dann mit 200 ml Wasser aufgenommen und ungelöste Bestandteile abfiltriert. Das Filtrat wurde dann mit einer wäßrigen HCl-Lösung auf pH=1 gestellt, 2h nachgerührt und das Produkt abgesaugt. Man erhielt 1.2 g eines gelblichen Feststoffs; mp = 80-82°C.
MS (EI) : 297
c) 5-Methansulfonyl-2-methyl-4-(3- pentafluorosulfanyl-phenylamino)-benzoesäure-methylester 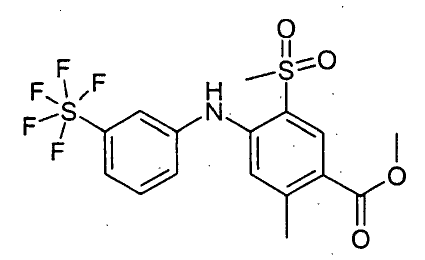 1,0 g N-(3-Pentafluorosulfanyl-phenyl)-methansulfonamid, 0.8 g 4-Fluor-5-methansulfonyl-2-methyl-benzoesäure-methylester sowie 1,4 ml TBTMG wurden in 10 ml NMP (wasserfrei) gelöst und 7h bei 150°C gerührt. Anschließend wurde mit 100 ml EE verdünnt und zunächst 3 mal mit je 30 ml einer gesättigten wäßrigen Na₂CO₃-Lösung, dann 3 mal mit je 30 ml einer gesättigten wäßrigen NaHSO₄-Lösung gewaschen. Über MgSO₄ wurde getrocknet, das Solvens im Vakuum entfernt und der Rückstand an Kieselgel mit DIP chromatographiert. Man erhielt 370 mg eines harzartigen Feststoffs.

| | |
|---|---|
| R_{f} (DIP) = 0.29 | MS (EI) : 445 |

d) 5-Methansulfonyl-2-methyl-4-(3-pentafluorosulfanyl-phenylamino)-benzoesäure 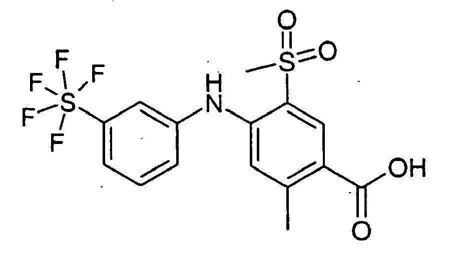
470 mg KOtBu und 480 mg Guanidinium-chlorid wurden in 5 ml DMF (wasserfrei) 1 h bei RT gerührt. Diese Suspension wurde anschließend zu 370 mg 5-Methansulfonyl-2-methyl-4-(3-pentafluorosulfanyl-phenylamino)-benzoesäure-methylester gegeben und 3 h bei RT gerührt. Anschließend wurde 2h bei 70°C gerührt. Es kam zu keiner wesentlichen Umsetzung (zum Acylguanidin), daher wurde der Ester verseift, indem 1 ml einer 2N wäßrigen NaOH-Lösung sowie 5 ml MeOH zugesetzt wurden und 6h bei RT gerührt wurde. Anschließend wurden die flüchtigen Bestandteile im Vakuum entfernt, der Rückstand mit 30 ml Wasser aufgenommen und mit wäßriger HCl-Lösung auf pH = 2 gestellt. Das Produkt fällt als weißer, amorpher Feststoff aus, der auf dem Filter wieder verläuft und daher in 30 ml EE erneut gelöst wird. Das Solvens wird in Vakuum entfernt und man erhält 230 mg eines weißen Schaumes.

| | |
|---|---|
| R_{f} (EE) = 0.47 | MS (ES⁻) : 430 |

e) N-[5-Methansulfonyl-2-methyl-4-(3-pentafluorosulfanyl-phenylamino)-benzoyl]-guanidin
220 mg 5-Methansulfonyl-2-methyl-4-(3-pentafluorosulfanyl-phenylamino)-benzoesäure wurden in 2 ml DMF (wasserfrei) gelöst und mit 108 mg CDI versetzt. Dann wurde 6 h bei RT gerührt. Daneben wurden 290 mg Guanidinium-chlorid und 290 mg KOtBu in 2 ml DMF (wasserfrei) gelöst und 30 Minuten bei RT gerührt. Diese Guanidin-Suspension wurde dann zu dem aktivierten Säurederivat Imidazol-1-yl-[5-methansulfonyl-2-methyl-4-(3-pentafluorosulfanyl-phenylamino)-phenyl]-methanon zugegeben und 20h bei RT stehen gelassen. Dann wurde das Reaktionsgemisch auf 50 ml Wasser gegossen, mit einer wäßrigen HCl-Lösung auf pH = 8 gestellt und 3 mal mit je 30 ml EE extrahiert. Über MgSO₄ wurde getrocknet, das Solvens im Vakuum entfernt und der Rückstand an Kieselgel mit EE/MeOH 10:1 chrömatographiert. Man erhielt 170 mg eines farblosen Schaumes.

| | |
|---|---|
| R_{f} (EE/MeOH 10:1) = 0.13 | MS (ES⁺) : 473 |

### Beispiel 6: N-[5-Methansulfonyl-2-methyl-4-(4-pentafluorosulfanyl-phenylamino)-benzoyl]-guanidin

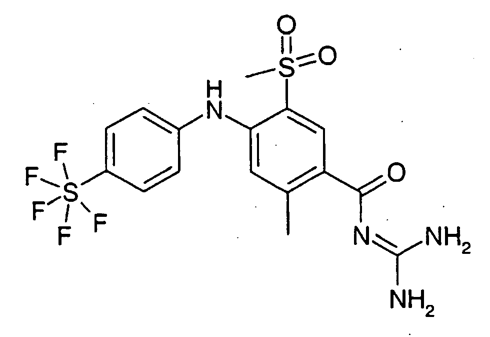

a) 5-Methansulfonyl-2-methyl-4-(4-pentafluorosulfanyl-phenylamino)-benzoesäure-methylester 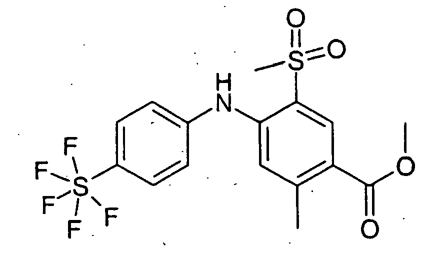
Das Startmaterial wurde analog Beispiel 5 a)-c) synthetisiert.
b) 5-Methansulfonyl-2-methyl-4-(4-pentafluorosulfanyl-phenylamino)-benzoesäure 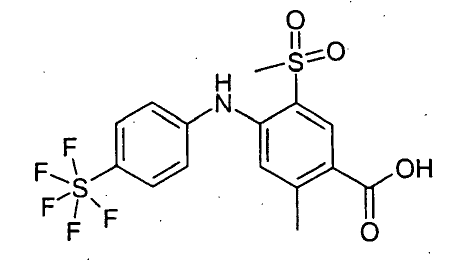
70 mg 5-Methansulfonyl-2-methyl-4-(4-pentafluorosulfanyl-phenylamino)-benzoesäure-methylester wurden in 2 ml MeOH gelöst und mit 0.12 ml einer 2N wäßrigen NaOH-Lösung versetzt. 2h wurde bei RT gerührt und anschließend 16h bei RT stehen gelassen. Dann wurde das Reaktionsgemisch auf 50 ml Wasser gegossen, mit wäßriger HCl-Lösung auf pH = 2 gestellt, 30 Minuten bei RT gerührt und schließlich das Produkt abgesaugt. Man erhielt 66 mg farbloser Kristalle, mp 106-109°C.
MS (ES⁻) : 430
c) N-[5-Methansulfonyl-2-methyl-4-(4-pentafluorosulfanyl-phenylamino)-benzoyl]-guanidin
65 mg 5-Methansulfonyl-2-methyl-4-(4-pentafluorosulfanyl-phenylamino)-benzoesäure wurden in 3 ml DMF (wasserfrei) gelöst, mit 37 mg CDI versetzt und 4h bei RT stehen gelassen. Daneben wurden 87 mg Guanidinium-chlorid und 85 mg KOtBu in 3 ml DMF 30 Minuten bei RT gerührt. Diese Suspension wurde anschließend zu der Lösung des aktivierten Säurederivates Imidazol-1-yl-[5-methansulfonyl-2-methyl-4-(3-pentafluorosulfanyl-phenylamino)-phenyl]-methanon zugegeben und 2h bei RT gerührt. Das Reaktionsgemisch wurde anschließend auf 80 ml Wasser gegossen, mit wäßriger HCl-Lösung auf pH = 8 gestellt und 2 mal mit je 20 ml EE extrahiert. Über MgSO₄ wurde getrocknet, das Solvens im Vakuum entfernt und der Rückstand an Kieselgel mit EE/MeOH 10:1 chromatographiert. Man erhielt 32 mg eines farblosen Schaumes.

| | |
|---|---|
| R_{f} (EE/MeOH 10:1) = 0.31 | MS (ES⁺) : 473 |

### Beispiel 7: N-{5-Methansulfonyl-2-methyl-4-[methyl-(3-pentafluorosulfanyl-phenyl)-amino]-benzoyl}-guanidin

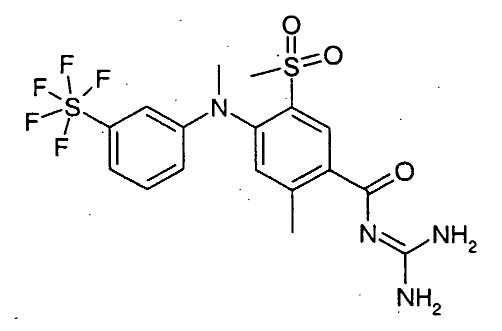

a) 5-Methansulfonyl-2-methyl-4-[methyl-(3-pentafluorosulfanyl-phenyl)-amino]-benzoesäure-methylester 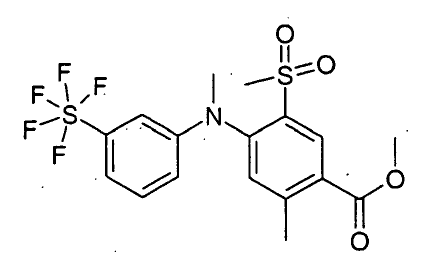
150 mg 5-Methansulfonyl-2-methyl-4-(3-pentafluorosulfanyl-phenylamino)-benzoesäure-methylester (Beispiel 5 c) wurden in 2 ml DMF (wasserfrei) gelöst, mit 13.5 mg NaH versetzt und 15 Minuten bei RT gerührt. Dann wurden 15 µl CH₃l zugespritzt und das Gemisch 18h bei RT stehen gelassen. Anschließend wurde das Reaktionsgemisch auf 10 ml einer gesättigten wäßrigen NaHCO₃-Lösung gegossen und mit 40 ml EE extrahiert. Die organische Phase wurde anschließend mit 10 ml Wasser gewaschen. Über MgSO₄ wurde getrocknet, das Solvens im Vakuum entfernt und man erhielt 120 mg eines blassgelben Schaumes.

| | |
|---|---|
| R_{f} (DIP) = 0.24 | MS (ES⁺) : 460 |

b) N-{5-Methansulfonyl-2-methyl-4-[methyl-(3-pentafluorosulfanyl-phenyl)-amino]-benzoyl}-guanidin
146 mg KOtBu sowie 150 mg Guanidinium-Chlorid wurden in 3 ml DMF (wasserfrei) 30 Minuten bei RT gerührt. Dann wurden 120 mg 5-Methansulfonyl-2-methyl-4-[methyl-(3-pentafluorosulfanyl-phenyl)-amino]-benzoesäure-methylester zugegeben, 2h bei RT gerührt und 15h bei RT stehen gelassen. Das Reaktionsgemisch wurde dann auf 50 ml Wasser gegossen und mit wäßriger HCI-Lösung auf pH = 8 eingestellt. 30 Minuten wurde bei RT gerührt, dann 2 mal mit je 20 ml EE extrahiert und dann die organische Phase noch 2 mal mit je 5 ml Wasser gewaschen. Über MgSO₄ wurde getrocknet, das Solvens im Vakuum entfernt und man erhielt 53 mg eines blassgelben Schaumes.

| | |
|---|---|
| R_{f} (EE/MeOH 10:1) = 0.17 | MS (ES⁺) : 973 (2M+H)⁺ |

### Beispiel 8: N-{5-Methansulfonyl-2-methyl-4-[methyl-(4-pentafluorosulfanyl-phenyl)-amino]-benzoyl}-guanidin

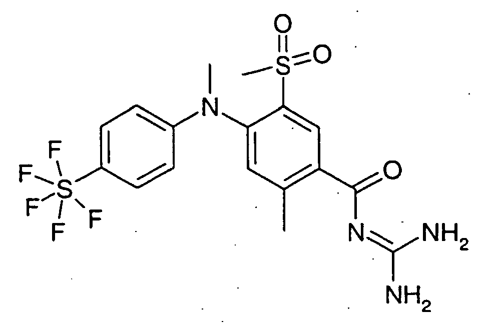

Die Titelverbindung des Beispiels 8 wurde analog Beispiel 7 aus Beispiel 6a) synthetisiert:

| | |
|---|---|
| R_{f} (EE/MeOH 10:1) = 0.26 | MS (ES⁺) : 973 (2M+H)⁺ |

### Beispiel 9: N-(2-Methansulfonyl-5-methyl-4'-pentafluorosulfanyl-biphenyl-4-carbonyl)-guanidin

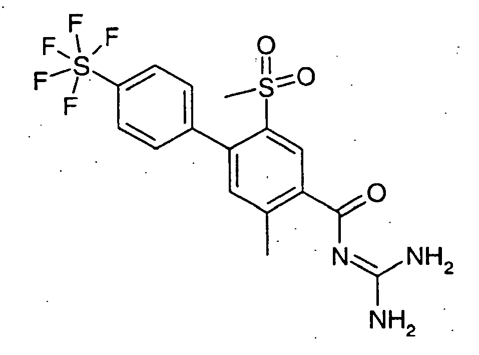

a) 1-Brom-4-pentafluorosulfanyl-benzol 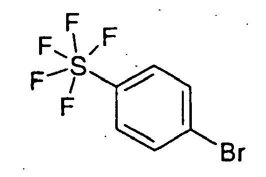
5.0 g 4-Pentafluorosulfanyl-anilin wurden in 20 ml Essigsäure gelöst und bei 0°C mit einer gesättigten wäßrigen HBr-Lösung versetzt. Dann wurde bei 0°C eine Lösung von 1.9 g NaNO₂ in 5 ml Wasser langsam innerhalb 5 Minuten zugetropft. 10 Minuten wurde bei 0°C nachgerührt, dann wurde das Reaktionsgemisch zu einer 0°C kalten Suspension von 6.5 g CuBr in 20 ml einer halbgesättigten wäßrigen HBr-Lösung portionsweise addiert. Dabei wird Stickstoff freigesetzt. 30 Minuten wurde bei 0°C, dann 1 h bei RT nachgerührt. Anschließend wurde 3 mal mit je 100 ml HEP extrahiert, dann wurde das HEP 2 mal mit je 50ml einer gesättigten wäßrigen Na₂CO₃-Lösung gewaschen. Über MgSO₄ wurde getrocknet, das Solvens im Vakuum entfernt und der Rückstand an Kieselgel mit HEP chromatographiert. Man erhielt 1.8 g eines farblosen Öls.

| | |
|---|---|
| R_{f} (HEP) = 0.50 | MS (EI) : 284 |

b) 2-Methansulfonyl-5-methyl-4'-pentafluorosulfanyl-biphenyl-4-carbonsäuremethylester 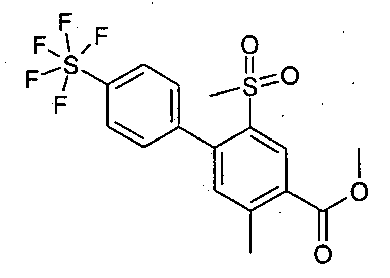
150 mg 1-Brom-4-pentafluorosulfanyl-benzol wurden zusammen mit 135 mg Bis-(pinacolato)-dibor, 156 mg Kaliumacetat sowie 64 mg Pd(dppf)₂ in 4 ml DMF 2h bei 80°C gerührt. Dann wurden 163 mg 4-Brom-5-methansulfonyl-2-methyl-benzoesäure-methylester, 337 mg Na₂CO₃, 64 mg Pd(dppf)₂ sowie 2 ml Wasser zugegeben und weitere 3h bei 80°C gerührt. Nach dem Abkühlen wurde mit 50 ml EE verdünnt und 2 mal mit je 10 ml Wasser gewaschen. Über MgSO₄ wurde getrocknet, das Solvens im Vakuum entfernt und der Rückstand an Kieselgel mit DIP chromatographiert. Man erhielt 150 mg eines farblosen Öls.

| | |
|---|---|
| R_{f} (DIP) = 0.28 | MS (CI) : 430 |

c) N-(2-Methansulfonyl-5-methyl-4'-pentafluorosulfanyl-biphenyl-4-carbonyl)-guanidin
186 mg Guanidinium-Chlorid und 182 mg KOtBu wurden in 5 ml DMF (wasserfrei) 30 Minuten bei RT gerührt. Diese Suspension wurde anschließend zu 140 mg 2-Methansulfonyl-5-methyl-4'-pentafluorosulfanyl-biphenyl-4-carbonsäure-methylester addiert und 4h bei RT gerührt. Dann wurde auf 20 ml Wasser gegossen und mit wäßriger HCI-Lösung auf pH = 8 eingestellt. Daraufhin fällt das Produkt aus und wird abgesaugt. Man erhielt 100 mg weißer Kristalle, mp 238-240°C.

| | |
|---|---|
| R_{f} (EE/MeOH 10:1) = 0.32 | MS (ES⁺) : 458 |

### Beispiel 10: N-(2-Methansulfonyl-5-methyl-3'-pentafluorosulfanyl-biphenyl-4-carbonyl)-guanidin

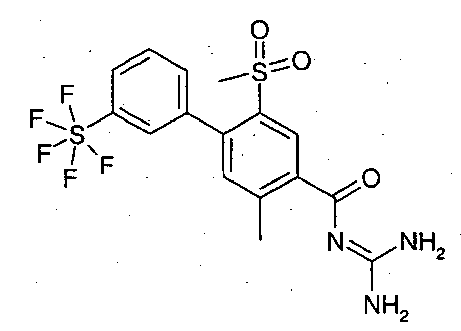

Die Titelverbindung des Beispiels 10 wurde analog Beispiel 9 synthetisiert. mp 169-175°C

| | |
|---|---|
| R_{f} (EE/MeOH 10:1) = 0.32 | MS (ES⁺) : 458 |

### Beispiel 11: N-(2-Methansulfonyl-5,2'-dimethyl-4'-pentafluorosulfanyl-biphenyl-4-carbonyl)-guanidin

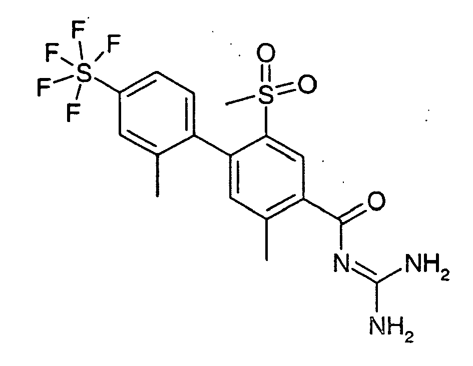

Die Titelverbindung des Beispiels 11 wurde analog Beispiel 9 synthetisiert.
MS (ES⁺) : 943 (2M+H)⁺

### Methode NHE-Hemmung

IC₅₀ für die NHE1-Hemmnung wurden bestimmt in einem FLIPR-Assay mittels Messung der pHᵢ-Erholung in transfizierten Zelllinien, die den humanen NHE-1 exprimieren.

Der Assay wurde im FLIPR (Fluorescent imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 weist als Folge von Mutagenese und anschließender Selektion keine endogene NHE-Aktivität auf), wurden am Vortag mit einer Dichte von ∼25.000 Zellen / Well ausgesät. Das Wachstumsmedium der transfizierten Zellen (Iscove +10 % fötales Kälberserum) enthielt zusätzlich G418 als Selektionsantibiotikum, um die Anwesenheit der transfizierten Sequenzen sicherzustellen.

Der eigentliche Assay begann mit der Entfernung des Wachstumsmediums und Zugabe von 100 µl /Well Beladungspuffer (5 µM BCECF-AM [2',7'-Bis-(Carboxyethyl)-5-(und-6)-Carboxyfluorescein, Acetoxymethyl ester] in 20 mM NH₄Cl, 115 mM Cholinchlorid, 1 mM MgCl₂, 1 mM CaCl₂, 5 mM KCI, 20 mM HEPES, 5 mM Glucose; pH 7,4 [mit KOH eingestellt]). Die Zellen wurden dann 20 Minuten bei 37°C inkubiert. Diese Inkubation führte zur Beladung der Zellen mit dem fluoreszierenden Farbstoff, dessen Fluoreszenzintensität vom pHi abhängt, und mit NH₄Cl, was zu einer leichten Alkalinisierung der Zellen führte.

Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.

Nach dieser 20-minütigen Inkubation wurde der Beladungspuffer, der NH₄Cl und freies BCECF-AM enthielt, durch dreimaliges Waschen im Zellwasher (Tecan Columbus) mit jeweils 400 µl Waschpuffer (133,8 mM Cholinchlorid, 4,7 mM KCI, 1,25 mM MgCl₂, 1,25 mM CaCl₂, 0,97 mM K₂HPO₄, 0,23 mM KH₂PO₄, 5 mM HEPES, 5 mM Glucose; pH 7,4 [mit KOH eingestellt]) entfernt. Das in den Wells verbleibende Restvolumen betrug 90 µl (50 - 125 µl möglich). Dieser Waschschritt entfernte das freie BCECF-AM und führte als Folge der Entfernung der externen NH₄⁺-Ionen zu einer intrazellulären Ansäuerung (∼ pHᵢ 6.3 - 6.4).

Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wurde, führte der Waschprozess dazu, dass intrazellulär H⁺ zurückblieb, was die Ursache für die intrazelluläre Ansäuerung war. Diese kann letztlich zum Zelltod führen, wenn sie lang genug anhält. An dieser Stelle war es wichtig, dass der Waschpuffer natriumfrei (<1 mM) war, da extrazelluläre Natrium-Ionen zu einer augenblicklichen Erholung des pHi durch die Aktivität der klonierten NHE-Isoformen führen würde.

Es war ebenfalls wichtig, dass alle verwendeten Puffer (Beladungspuffer, Waschpuffer, Recoverypuffer) keine HCO₃⁻-Ionen enthielten, da die Anwesenheit von Bicarbonat zur Aktivierung störender bicarbonatabhängiger pHᵢ-Regulationssysteme führen würde, die in der parentalen LAP-1 Zelllinie enthalten sind.

Die Mikrotiterplatten mit den angesäuerten Zellen wurden dann (bis zu 20 Minuten nach der Ansäuerung) zum FLIPR transferiert. Im FLIPR wurde der intrazelluläre Fluoreszenzfarbstoff durch Licht mit einer Wellenlänge von 488 nm, das von einem Argon-Laser erzeugt wurde, angeregt, und die Messparameter (Laserleistung, Belichtungszeit und Blende der im FLIPR eingebauten CCD-Kamera) wurden so gewählt, dass das durchschnittliche Fluoreszenzsignal pro Well zwischen 30000 und 35000 relativen Fluoreszenzeinheiten lag.

Die eigentliche Messung im FLIPR begann damit, dass Software-gesteuert alle zwei Sekunden eine Aufnahme mit der CCD-Kamera gemacht wurde. Nach zehn Sekunden wurde die Erholung des intrazellulären pH's durch Zugabe von 90 µl Recoverypuffer (133,8 mM NaCl, 4,7 mM KCI, 1,25 mM MgCl₂, 1,25 mM CaCl₂, 0,97 mM K₂HPO₄, 0,23 mM KH₂PO₄, 10 mM HEPES, 5 mM Glucose; pH 7.4 [mit NaOH eingestellt]) mittels des im FLIPR eingebauten 96-Well-Pipettierers eingeleitet.

Als Positivkontrollen (100 % NHE-Aktivität) dienten Wells, denen reiner Recoverypuffer zugegeben wurde, Negativkontrollen (0 % NHE-Aktivität) erhielten Waschpuffer. In allen anderen Wells wurde Recoverypuffer mit der zweifach konzentrierten Testsubstanz hinzugegeben. Die Messung im FLIPR endete nach 60 Messpunkten (zwei Minuten).

Die Rohdaten werden in das Programm ActivityBase exportiert. Mit diesem Programm werden zunächst die NHE-Aktivitäten für jede getestete Substanzkonzentration und daraus die IC₅₀-Werte für die Substanzen berechnet. Da der Verlauf der pHᵢ-Erholung nicht während des ganzen Experiments linear war, sondern am Ende aufgrund abnehmender NHE-Aktivität bei höheren pHᵢ-Werten abfiel, war es wichtig, für die Auswertung der Messung den Teil auszuwählen, in dem die Fluoreszenzzunahme der Positivkontrollen linear war.

| Beispiel | NHE1-Hemmung IC₅₀ [nM] |
|---|---|
| 1 | 3,9 |
| 2 | 1,9 |
| 3 | 4,7 |
| 4 | 4675 |
| 5 | 6,4 |
| 6 | 27,1 |
| 7 | 14,5 |
| 8 | 3,7 |
| 9 | 2,1 |
| 10 | 38,8 |
| 11 | 81,5 |

In vivo Pharmakokinetik - Profilierung mit der "n in one Methode"

Als pharmakokinetische Kenndaten wurden die Expositionsdaten und die Halbwertszeit wie folgt bestimmt:
Zwei erfindungsgemäße NHE-1 Inhibitoren (Beispiel 1 und Beispiel 9) und eine bekannte NHE-1 Referenzsubstanz (Cariporid) 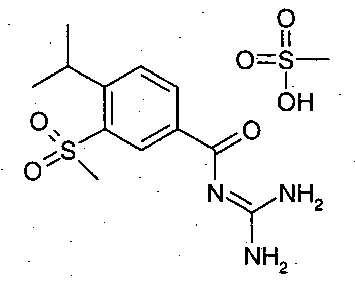 wurden in wässrigem, leicht saurem Medium (Wasser, pH 4, eingestellt mit 1 M Salzsäure) gelöst. Die Konzentration der so hergestellten wässrigen Formulierung betrug ca. 1,5 mg je Substanz pro 1 g Lösung. 10 ml dieser Formulierung wurden einem männlichen, nüchternen Beagle-Hund in die Vena jugularis mittels Katheter als Bolus einmal appliziert (Dosis ca 1 mg je applizierter Substanz pro kg Körpergewicht des Hundes). Mittels eines zweiten Katheters wurden nach 5min, 15min, 30min, 1 h, 2h, 4h, 8h und 24 h Blutproben gewonnen und heparinisiertes Plasma durch Zentrifugation bei 1000G in entsprechenden Plasmaröhrchen hergestellt.

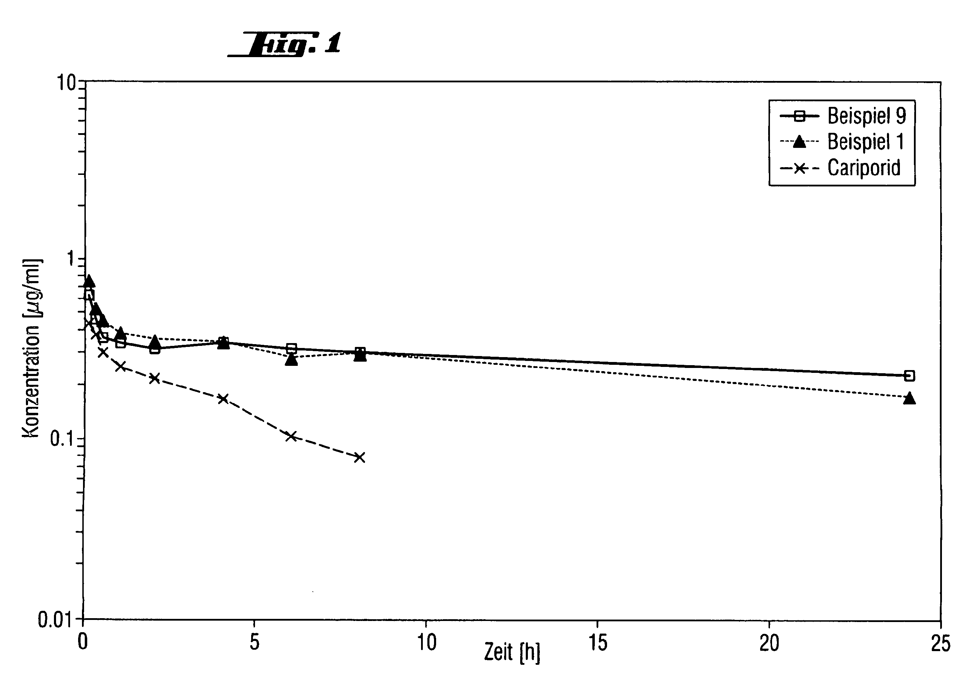

Die Plasmaproben wurden aufgearbeitet und nach einer HPLC-Trennung mittels MS/MS quantifiziert. Diese Methode erlaubte wegen ihrer hohen Spezifität, die gleichzeitige Bestimmung mehrerer Substanzen. Aus den Konzentrations - Zeitkurven (siehe Abbildung 1) ließen sich die Halbwertszeiten mittels des Computerprogramms WinNonlin berechnen und mit der Halbwertszeit der bekannten NHE-1 Referenzsubstanz vergleichen. Da die verschiedenen Substanzen im gleichen Tier zum gleichen Zeitpunkt gemessen wurden, ergab sich ein exakter Vergleich der Verbindungen und es konnte ein Ranking der Halbwertszeiten erstellt werden.

| Verbindung | Halbwertszeit t½ [h] |
|---|---|
| Beispiel 1 | 20.2 |
| Beispiel 9 | 39.9 |
| Vergleichsbeispiel Cariporid | 4.1 |

Aus den Konzentrations-Zeitkurven in Abbildung 1 und den ermittelten Halbwertszeiten ist deutlich ersichtlich, das die erfindungsgemäßen Verbindungen im Blut auch über einen längeren Zeitraum länger erhalten bleiben und damit die Halbwertszeiten 5 bis 10 mal größer sind als bei der Referenzsubstanz Cariporid.

In der Abbildung wurden folgende Beschriftungen und Kennzeichnungen vorgenommen:
Abbildung 1: Konzentrations - Zeitkurven im Blutplasma von Hunden nach Gabe von etwa 1 mg/kg von Beispiel 1, Beispiel 9 und Cariporid.
Y-Achse: Konzentration der gemessenen Verbindung in µg/ml im Plasma
X-Achse: Zeit in h

## Patentansprüche

1. Pentafluorosulfanylphenyl-substituierte Benzoylguanidine der Formel I oder II 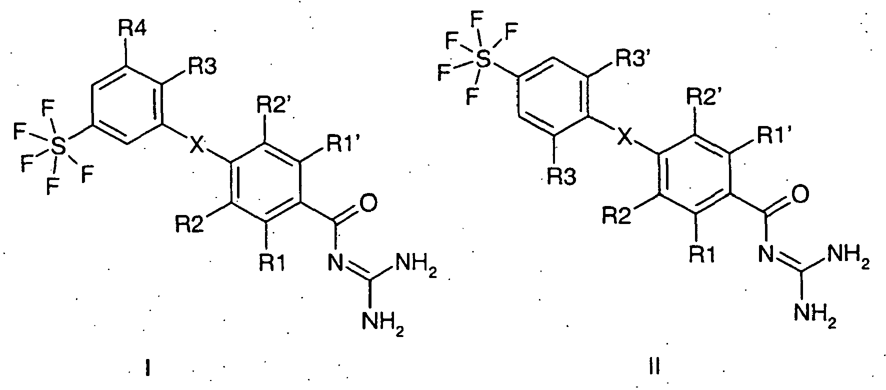 worin bedeuten
R1 und R1' unabhängig voneinander Wasserstoff, Alkyl mit 1, 2, 3 oder 4 C-Atomen, Alkoxy mit 1, 2, 3 oder 4 C-Atomen, F, Cl, Br, I, -CN, NR5R6, -Oₐ-(CH₂)_{b}-(CF₂)_{c}-CF₃ oder -(SO_{d})ₑ-(CH₂)_{f}-(CF₂)_{g}-CF₃;
R5 und R6 unabhängig voneinander Wasserstoff, Alkyl mit 1, 2, 3 oder 4 C- Atomen oder -CH₂-CF₃;
d Null, 1 oder 2;
a, b, c, e, f und g unabhängig voneinander Null oder 1;
R2 und R2' unabhängig voneinander Wasserstoff, F, Cl, Br, I, -CN, -SO₂CH₃, Alkoxy mit 1, 2, 3 oder 4 C-Atomen, NR5R6, -Oₐ-(CH₂)_{b}-(CF₂)_{c}-CF₃, -(SOₕ)ₖ-(CH₂)ₗ-(CF₂)ₘ- CF₃, Alkyl mit 1, 2, 3, 4, 5 oder 6 C-Atomen, Cycloalkyl mit 3, 4, 5, 6, 7 oder 8 C- Atomen, in dem 1, 2, 3 oder 4 Wasserstoff-Atome durch Fluoratome ersetzt sein können, -(CH₂)ₙ -Phenyl, das unsubstituiert ist oder substituiert ist mit 1, 2 oder 3 Resten ausgewählt aus der Gruppe bestehend aus F, Cl, Br, I, -Oₒ-(CH₂)ₚ-CF₃, Alkoxy mit 1, 2, 3 oder 4 C-Atomen, Alkyl mit 1, 2, 3 oder 4 C-Atomen und -SO₂CH₃, oder -(CH₂)_{q} -Heteroaryl, das unsubstituiert ist oder substituiert ist mit 1, 2 oder 3 Resten ausgewählt aus der Gruppe bestehend aus F, Cl, Br, I, -Oᵣ-(CH₂)ₛ-CF₃, Alkoxy mit 1, 2, 3 oder 4 C-Atomen, Alkyl mit 1, 2, 3 oder 4 C- Atomen und -SO₂CH₃;
R5 und R6 unabhängig voneinander Wasserstoff, Alkyl mit 1, 2, 3 oder 4 C- Atomen oder -CH₂-CF₃;
a, b und c unabhängig voneinander Null oder 1;
h Null, 1 oder 2;
k Null oder 1;
l Null, 1, 2, 3 oder 4;
m und o unabhängig voneinander Null oder 1;
p Null, 1, 2 oder 3;
n Null, 1, 2, 3 oder 4;
r Null oder 1;
s Null, 1, 2 oder 3;
q Null, 1, 2, 3 oder 4;
R3 und R3' unabhängig voneinander Wasserstoff, F, Cl, Br, I, -CN, -SO₂CH₃, Alkyl mit 1, 2, 3, 4, 5 oder 6 C-Atomen, Alkoxy mit 1, 2, 3 oder 4 C-Atomen oder -Oₜ-(CH₂)ᵤ-CF₃;
t Null oder 1;
u Null, 1, 2 oder 3;
R4 Wasserstoff, F, Cl, Br, I, -CN, -SO₂CH₃, NR5R6, -(SOᵥ)_{w}-(CH₂)ₓ-(CF₂)_{y}-CF₃, -O_{z}-(CH₂)ₐₐ -(CF₂)_{bb}-CF₃, Alkyl mit 1, 2, 3, 4, 5 oder 6 C-Atomen, Alkoxy mit 1, 2, 3 oder 4 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R5 und R6 unabhängig voneinander Wasserstoff, Alkyl mit 1, 2, 3 oder 4 C- Atomen oder -CH₂-CF₃;
v Null, 1 oder 2;
x Null, 1, 2, 3 oder 4;
w, y, z, aa und bb unabhängig voneinander Null oder 1;
oder
R4 -(CH₂)_{cc} -Phenyl, das unsubstituiert ist oder substituiert ist mit 1, 2 oder 3 Resten ausgewählt aus der Gruppe bestehend aus F, Cl, Br, I, -O_{dd}-(CH₂)ₑₑ-CF₃, Alkoxy mit 1, 2, 3 oder 4 C-Atomen, Alkyl mit 1, 2, 3 oder 4 C-Atomen und -SO₂CH₃;
dd Null oder 1;
ee Null, 1, 2 oder 3;
cc Null, 1, 2, 3 oder 4;
oder
R4 -(CH₂)_{ff}-Heteroaryl, das unsubstituiert ist oder substituiert ist mit 1, 2 oder 3 Resten ausgewählt aus der Gruppe bestehend aus F, Cl, Br, I, -O_{gg}-(CH₂)ₕₕ- CF₃, Alkoxy mit 1, 2, 3 oder 4 C-Atomen, Alkyl mit 1,2, 3 oder 4 C-Atomen und -SO₂CH₃;
gg Null oder 1;
hh Null, 1, 2 oder 3;
ff Null, 1, 2, 3 oder 4;
X eine direkte Bindung, O, NR7, S(O)ₖₖ;
R7 Wasserstoff, Alkyl mit 1, 2, 3 oder 4 C-Atomen, -(CH2)ₘₘ-CF₃ oder -SO₂CH₃
kk Null, 1 oder 2;
mm Null, 1, 2 oder 3;
wobei -Oₐ-(CH₂)_{b}-(CF₂)_{c}-CF₃ in den Definitionen von R1 und R1' und R2 und R2' unabhängig voneinander gewählt werden kann,
wobei NR5R6 in den Definitionen von R1 und R1', R2 und R2' und R4 unabhängig voneinander gewählt werden kann,
sowie deren pharmazeutisch verträgliche Salze.

2. Verbindung der Formel I oder II nach Anspruch 1, in denen bedeuten:
R1 und R1' unabhängig voneinander Wasserstoff, Alkyl mit 1, 2, 3 oder 4 C-Atomen, Methoxy, Ethoxy, F, Cl, NR5R6, -O-CH₂-CF₃ oder -(SO_{d})ₑ-(CH₂)_{f}-CF₃;
R5 und R6 unabhängig voneinander Wasserstoff, Alkyl mit 1, 2, 3 oder 4 C-Atomen oder -CH₂-CF₃;
d Null, 1 oder 2;
e und f unabhängig voneinander Null oder 1;
R2 und R2' unabhängig voneinander Wasserstoff, F, Cl, -SO₂CH₃, -(SOₕ)ₖ-(CH₂)ₗ-CF₃, Alkyl mit 1, 2, 3 oder 4 C-Atomen, Cycloalkyl mit 3, 4, 5, 6 oder 7 C-Atomen, in dem 1, 2, 3 oder 4 Wasserstoff-Atome durch Fluoratome ersetzt sein können, Phenyl, das unsubstituiert ist oder substituiert ist mit 1 - 2 Resten ausgewählt aus der Gruppe betstehend aus F, Cl, -Oₒ-(CH₂)ₚ-CF₃, Methoxy, Ethoxy, Alkyl mit 1, 2, 3 oder 4 C-Atomen und -SO₂CH₃, oder Heteroaryl, das unsubstituiert ist oder substituiert ist mit 1 - 2 Resten ausgewählt aus der Gruppe bestehend aus F, Cl, -Oᵣ-(CH₂)ₛ- CF₃, Methoxy, Ethoxy, Alkyl mit 1, 2, 3 oder 4 C-Atomen und -SO₂CH₃;
h Null, 1 oder 2;
k Null oder 1;
l Null, 1, 2, 3 oder 4;
o Null oder 1;
p Null, 1, 2 oder 3;
r Null oder 1 ;
s Null, 1, 2 oder 3;
R3 und R3' unabhängig voneinander Wasserstoff, F, Cl, -SO₂CH₃, Alkyl mit 1, 2, 3 oder 4 C- Atomen, Methoxy, Ethoxy oder -Oₜ-(CH₂)ᵤ-CF₃,
t Null oder 1;
u Null, 1, 2 oder 3;
R4 Wasserstoff, F, Cl, -SO₂CH₃, -(SOᵥ)_{w}-(CH₂)ₓ-CF₃, -O_{z}-(CH₂)ₐₐ-CF₃, Alkyl mit 1, 2, 3 oder 4 C-Atomen, Methoxy, Ethoxy oder Cycloalkyl mit 3, 4, 5, 6 oder 7 C- Atomen, in dem 1, 2, 3 oder 4 Wasserstoff-Atome durch Fluoratome ersetzt sein können;
v Null, 1 oder 2;
w, x, z und aa unabhängig voneinander Null oder 1;
oder
R4 Phenyl, das unsubstituiert ist oder substituiert ist mit 1 - 2 Resten ausgewählt aus der Gruppe bestehend aus F, Cl, -O_{dd}-(CH₂)ₑₑ-CF₃, Methoxy, Ethoxy, Alkyl mit 1, 2, 3 oder 4 C-Atomen und -SO₂CH₃;
dd und ee unabhängig voneinander Null oder 1;
oder
R4 Heteroaryl, das unsubstituiert ist oder substituiert ist mit 1 - 2 Resten ausgewählt aus der Gruppe bestehend aus F, Cl, -O_{gg}-(CH₂)ₕₕ-CF₃, Methoxy, Ethoxy, Alkyl mit 1, 2, 3 oder 4 C-Atomen und -SO₂CH₃;
gg und hh unabhängig voneinander Null oder 1;
X eine direkte Bindung, O, NR7, S(O)ₖₖ
R7 Wasserstoff, Alkyl mit 1, 2, 3 oder 4 C-Atomen, -CH₂-CF₃ oder -SO₂CH₃;
kk Null, 1 oder 2;
sowie deren pharmazeutisch verträgliche Salze.

3. Verbindung der Formel I oder II nach Anspruch 1 oder 2, in denen bedeuten:
R1 und R1' unabhängig voneinander Wasserstoff, Alkyl mit 1, 2, 3 oder 4 C-Atomen, Methoxy, Ethoxy, F, Cl, NR5R6, -O-CH₂-CF₃ oder -(SO_{d})ₑ-(CH₂)_{f}-CF₃;
R5 und R6 unabhängig voneinander Wasserstoff, Alkyl mit 1, 2, 3 oder 4 C- Atomen oder -CH₂-CF₃;
d Null, 1 oder 2;
e und f unabhängig voneinander Null oder 1;
R2 und R2' unabhängig voneinander Wasserstoff, F, Cl, -SO₂CH₃, -(SOₕ)ₖ-(CH₂)ₗ-CF₃, Methyl, Cycloalkyl mit 3, 4, 5, 6 oder 7 C-Atomen, in dem 1, 2, 3 oder 4 Wasserstoff-Atome durch Fluoratome ersetzt sein können, Phenyl, das unsubstituiert ist oder substituiert ist mit einem Rest ausgewählt aus der Gruppe bestehend aus F, Cl, -Oₒ-(CH₂)ₚ-CF₃, Methoxy, Ethoxy, Alkyl mit 1, 2, 3 oder 4 C-Atomen und -SO₂CH₃, oder Heteroaryl, das unsubstituiert ist oder substituiert ist mit einem Rest ausgewählt aus der Gruppe bestehend aus F, Cl, -Oᵣ-(CH₂)ₛ₋ CF₃, Methoxy, Ethoxy, Alkyl mit 1, 2, 3 oder 4 C-Atomen und -SO₂CH₃;
h Null, 1 oder 2;
k, l, o, p, r und s unabhängig voneinander Null oder 1;
R3 und R3' unabhängig voneinander Wasserstoff, F, Cl, -SO₂CH₃, Methyl, Methoxy, Ethoxy oder -Oₜ-(CH₂)ᵤ-CF₃;
t und u unabhängig voneinander Null oder 1;
R4 Wasserstoff, F, Cl, -SO₂CH₃, -(SOᵥ)_{w}-(CH₂)ₓ-CF₃, -O_{z}-(CH₂)ₐₐ-CF₃, Methyl, Methoxy, Ethoxy oder Cycloalkyl mit 3, 4, 5, 6 oder 7 C-Atomen, in dem 1, 2, 3 oder 4 Wasserstoff-Atome durch Fluoratome ersetzt sein können;
v Null, 1 oder 2;
w, x, z und aa unabhängig voneinander Null oder 1;
X eine direkte Bindung, O, NR7 oder S(O)ₖₖ;
R7 Wasserstoff, Methyl, Ethyl, -CH₂-CF₃ oder -SO₂CH₃;
kk Null, 1 oder 2
sowie deren pharmazeutisch verträgliche Salze.

4. Verbindung der Formel I oder II nach einem der Ansprüche 1 bis 3, in denen bedeuten:
R1 und R1' unabhängig voneinander Wasserstoff, Methyl, F, Cl, -CF₃ oder -O-CH₂-CF₃,
R2 und R2' unabhängig voneinander Wasserstoff, F, Cl, -SO₂CH₃, -SO₂-CF₃, -CF₃ oder Methyl;
R3 und R3' unabhängig voneinander Wasserstoff, F, Cl, -SO₂CH₃, Methyl, -CF₃ oder -O-CH₂-CF₃;
R4 Wasserstoff, F, Cl, -SO₂CH₃, -O-CH₂-CF₃ oder Methyl;
X eine direkte Bindung, O, NR7 oder S(O)ₖₖ;
R7 Wasserstoff, Methyl, Ethyl, -CH₂-CF₃ oder -SO₂CH₃;
kk Null, 1 oder 2;
sowie deren pharmazeutisch verträgliche Salze.

5. Verbindung der Formel I oder II nach einem der Ansprüche 1 bis 4 ausgewählt aus der Gruppe:
N-[5-Methansulfonyl-2-methyl-4-(3-pentafluorosulfanyl-phenoxy)-benzoyl]-guanidin,
N-[5-Methansulfonyl-2-methyl-4-(4-pentafluorosulfanyl-phenoxy)-benzoyl]-guanidin,
N-[5-Methansulfonyl-2-methyl-4-(4-pentafluorosulfanyl-phenylsulfanyl)-benzoyl]-guanidin,
N-[5-Methansulfonyl-2-methyl-4-(4-pentafluorosulfanyl-phenylsulfonyl)-benzoyl]-guanidin,
N-[5-Methansulfonyl-2-methyl-4-(3-pentafluorosulfanyl-phenylamino)-benzoyl]-guanidin,
N-[5-Methansulfonyl-2-methyl-4-(4-pentafluorosulfanyl-phenylamino)-benzoyl]-guanidin,
N-[5-Methansulfonyl-2-methyl-4-(4-pentafluorosulfanyl-phenylamino)-benzoyl]-guanidin,
N-{5-Methansulfonyl-2-methyl-4-[methyl-(3-pentafluorosulfanyl-phenyl)-amino]-benzoyl}-guanidin,
N-{5-Methansulfonyl-2-methyl-4-[methyl-(4-pentafluorosulfanyl-phenyl)-amino]-benzoyl}-guanidin,
N-(2-Methansulfonyl-5-methyl-4'-pentafluorosulfanyl-biphenyl-4-carbonyl)-guanidin,
N-(2-Methansulfonyl-5-methyl-3'-pentafluorosulfanyl-biphenyl-4-carbonyl)-guanidin und
N-(2-Methansulfonyl-5,2'-dimethyl-4'-pentafluorosulfanyl-biphenyl-4-carbonyl)-guanidin sowie deren pharmazeutisch verträgliche Salze.

6. Verfahren zur Herstellung einer Verbindung der Formeln I oder II und/oder deren pharmazeutisch verträgliche Salze, worin X Sauerstoff ist, **dadurch gekennzeichnet, dass** man
a) ein Phenol der Formeln III oder IV mit einem Aromaten der Formel V zu einer Verbindung der Formeln VIa oder VIIa umsetzt
und
b) eine Verbindung der Formeln VIa oder Vlla mit Guanidin zum Acylguanidin der Formeln Ia oder IIa umsetzt, 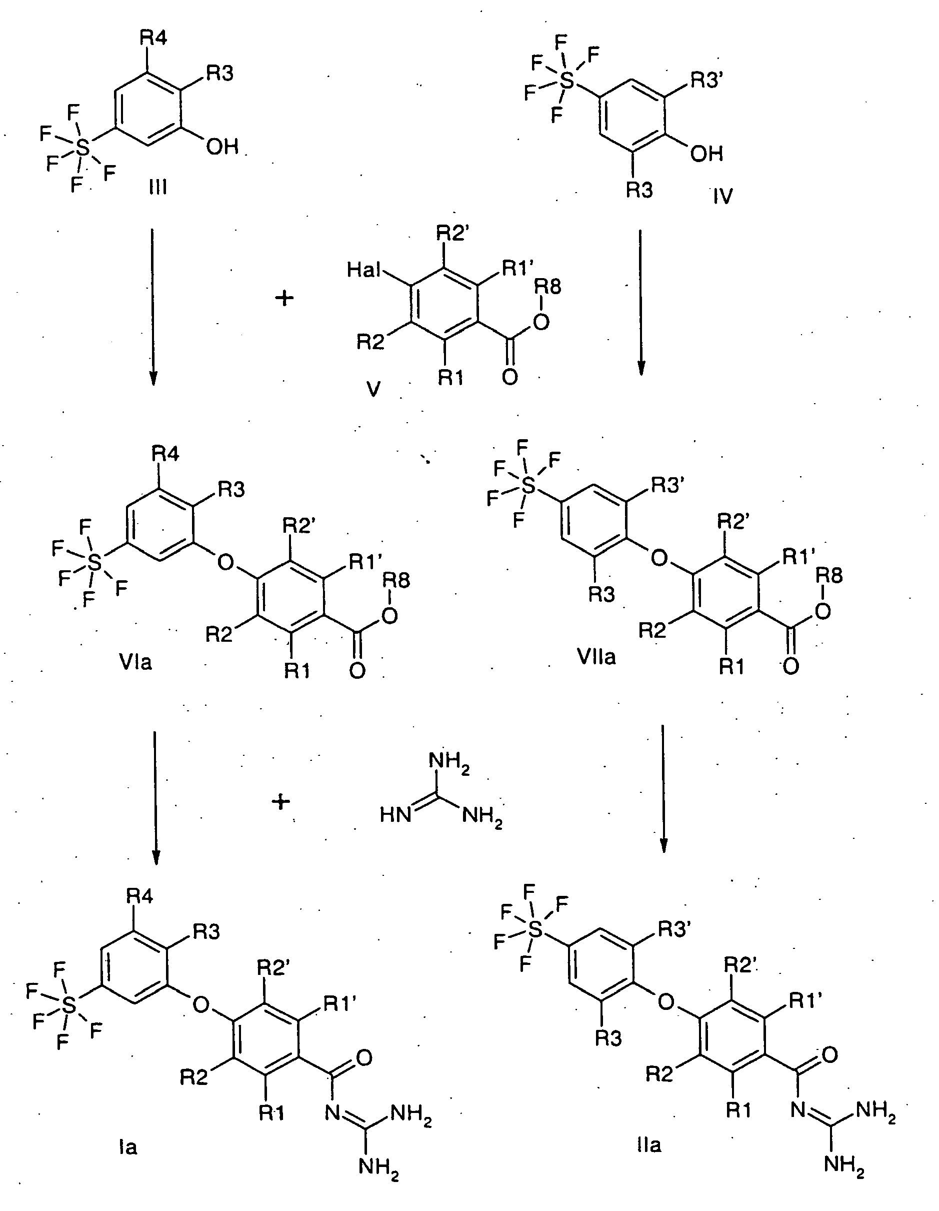
worin R1, R1', R2, R2', R3, R3' und R4 die in den Ansprüchen 1, 2, 3 und/oder 4 angegebene Bedeutung besitzen und worin bedeuten
Hal F, Cl, Br oder I,
R8 Wasserstoff oder Alkyl mit 1, 2, 3 oder 4 C-Atomen.

7. Verfahren zur Herstellung einer Verbindung der Formeln I oder II und/oder deren pharmazeutisch verträgliche Salze, worin X NR7 ist, **dadurch gekennzeichnet, dass** man
a) ein Anilin der Formeln VIII oder IX mit einem Sulfonsäurechlorid sulfoniert zu einer Verbindung der Formeln X oder XI,
b) eine Verbindung der Formeln X oder XI mit einem Aromaten der Formel V zu einer Verbindung der Formeln Vlb oder Vllb umsetzt,
c) zur Herstellung einer Verbindung der Formeln Ib oder IIb, in der R7 verschieden von Wasserstoff ist, eine Verbindung der Formeln VIb oder Vllb zu einer Verbindung der Formeln VIc oder VIIc derivatisiert,
und
d) eine Verbindung der Formeln VIb/c oder VIIb/c mit Guanidin zu einem Acylguanidin der Formeln Ib oder IIb umsetzt, 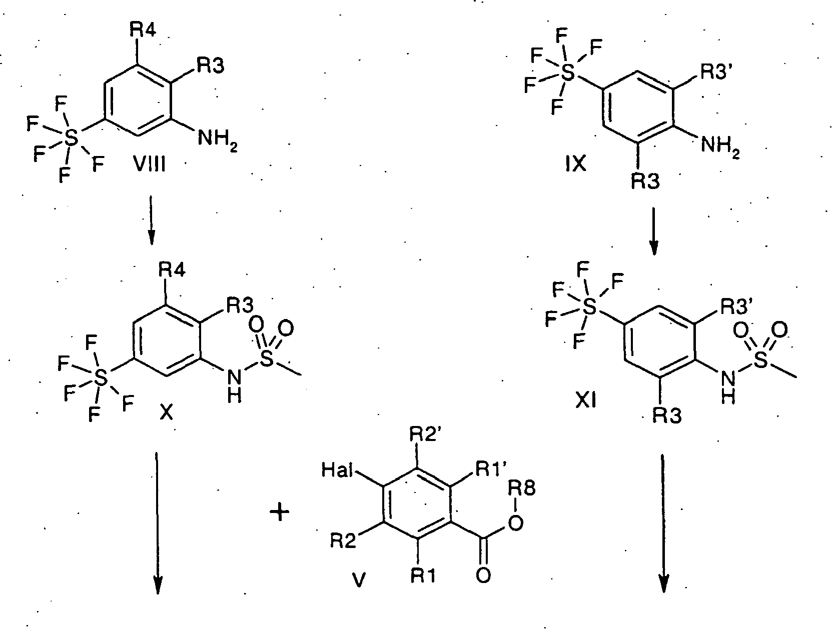 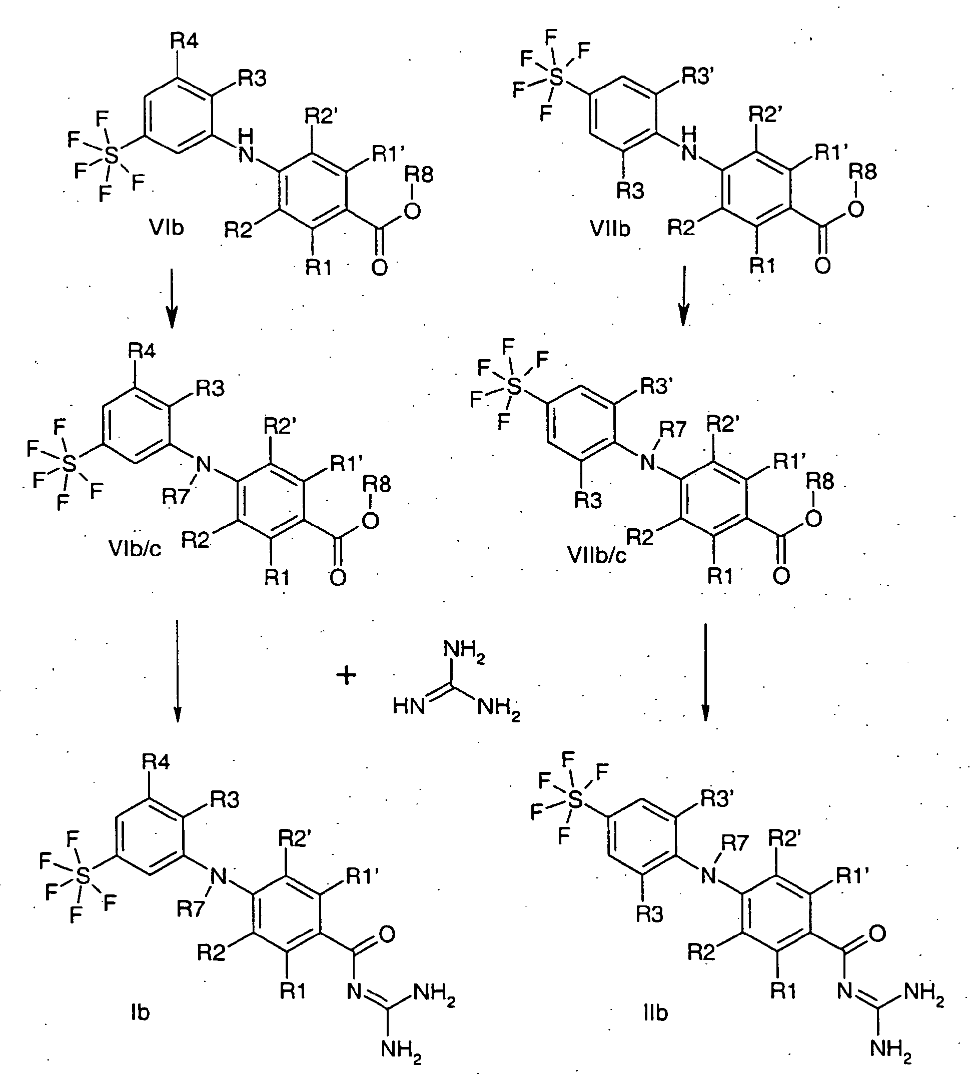
worin R1, R1', R2, R2', R3, R3', R4 und R7 die in den Ansprüchen 1, 2, 3 und/oder 4 angegebene Bedeutung besitzen und worin bedeuten
Hal F, Cl, Br oder I
R8 Wasserstoff oder Alkyl mit 1, 2, 3 oder 4 C-Atomen.

8. Verfahren zur Herstellung einer Verbindung der Formeln I der II und/oder deren pharmazeutisch verträgliche Salze, worin X S(O)ₖₖ ist, **dadurch gekennzeichnet, dass** man
a) ein Thiophenol der Formeln XII oder XIII mit einem Aromaten der Formel V zu einer Verbindung der Formeln Vle oder Vlle umsetzt,
b) zur Herstellung einer Verbindung der Formeln Ic oder IIc, in der kk verschieden von Null ist, eine Verbindung der Formeln VIe oder VIIe zu einer Verbindung der Formeln Vlf oder Vllf oxidiert
und
c) eine Verbindung der Formeln VIe/f oder VII/ef mit Guanidin zu einem Acylguanidin der Formeln Ic oder IIc umsetzt, 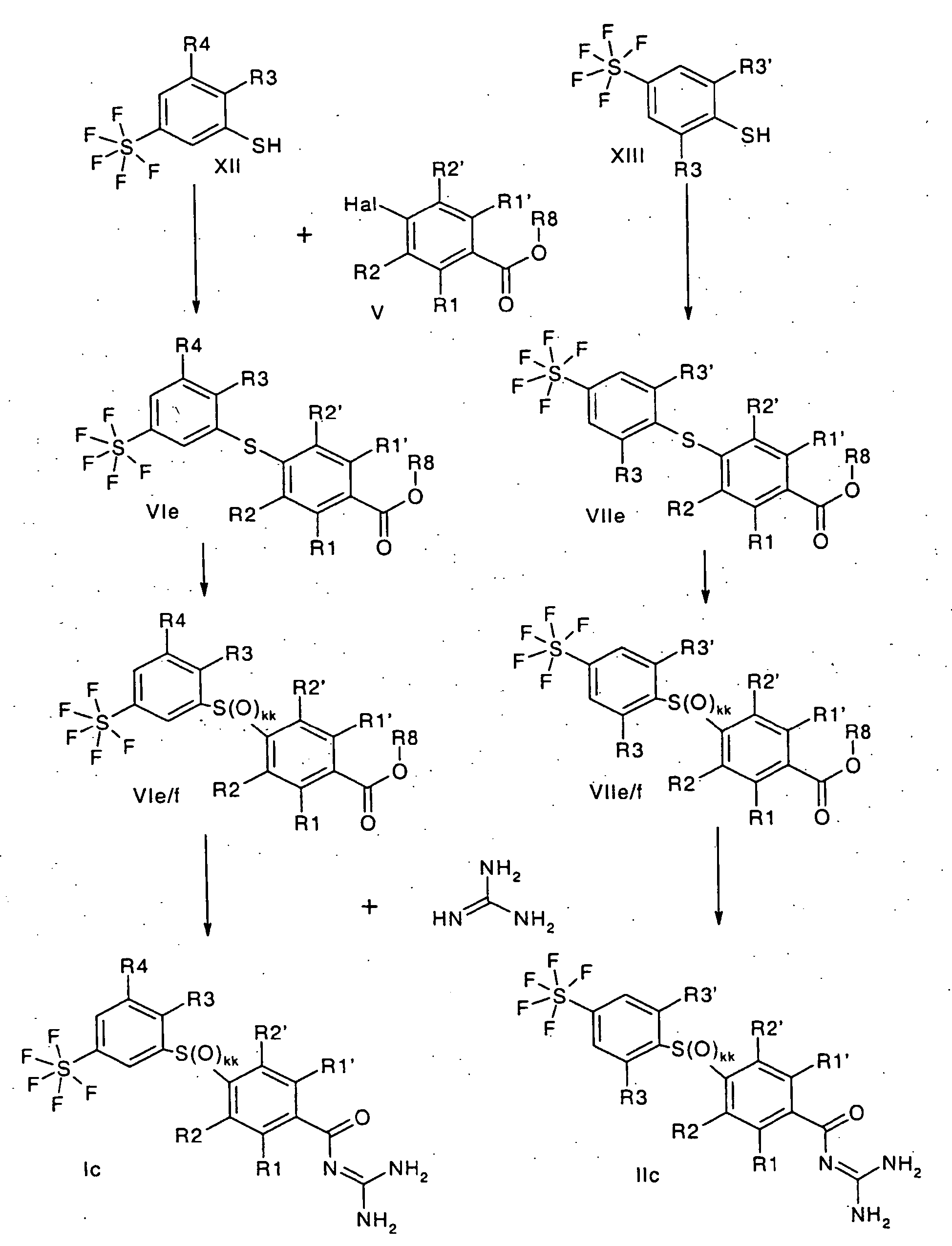
worin R1, R1', R2, R2', R3, R3', R4 und kk die in den Ansprüchen 1, 2, 3 und/oder 4 angegebene Bedeutung besitzen und worin bedeuten
Hal F, Cl, Br oder I
R8 Wasserstoff oder Alkyl mit 1, 2, 3 oder 4 C-Atomen

9. Verfahren zur Herstellung einer Verbindung der Formeln I oder II und/oder deren pharmazeutisch verträgliche Salze, worin X eine direkte Bindung ist, **dadurch gekennzeichnet, dass** man
a) ein Halogenid der Formeln XIV oder XV in einer Suzuki-Kopplung mit einem Benzoesäureester der Formeln Va zu einem Biphenyl-Derivat der Formeln VIg oder VIIg koppelt
und
b) eine Verbindung der Formeln VIg und Vllg mit Guanidin zu einem Acylguanidin der Formeln Id oder IId umsetzt, 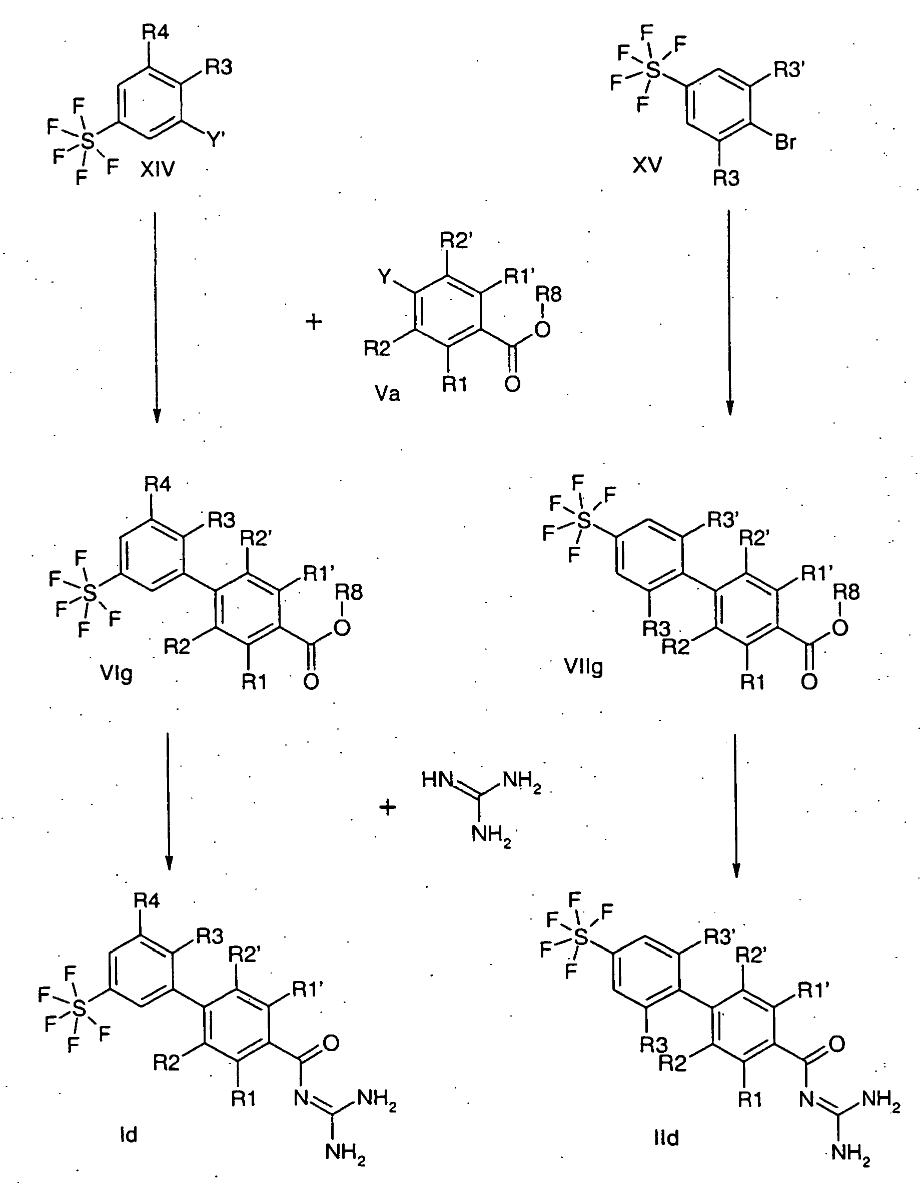
worin R1, R1', R2, R2', R3, R3' und R4 die in den Ansprüchen 1, 2, 3 und/oder 4 angegebene Bedeutung besitzen und worin bedeuten
Y und Y' unabhängig voneinander Cl, Br oder I
R8 Wasserstoff oder Alkyl mit 1, 2, 3 oder 4 C-Atomen.

10. Verbindung der Formel I und/oder II und/oder deren pharmazeutisch verträgliche Salze nach einem oder mehreren der Ansprüche 1 bis 5 zur Verwendung als Medikament.

11. Verwendung einer Verbindung der Formel I und/oder 11 und/oder deren pharmazeutisch verträgliche Salze nach einem oder mehreren der Ansprüche 1 bis 5 zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasie, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, wie Epilepsie oder zentral ausgelöste Krämpfe, von Erkrankungen des Zentralnervensystems, von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe-der Herzinsuffizienz oder des Congestive Heart Failure, akuter oder chronischer inflammatorischer Erkrankungen, von Erkrankungen, die durch Protozoen verursacht werden, von Malaria oder der Hühnercoccidiose oder zur Herstellung eines Medikaments zum Einsatz bei chirurgischen Operationen und Organtransplantationen, zur Konservierung und Lagerung von Transplantaten für chirurgische Maßnahmen, zur Verhinderung altersbedingter Gewebsveränderung, zur Herstellung eines gegen die Alterung gerichteten Medikaments oder zur Herstellung eines Medikaments zur Lebensverlängerung, zur Behandlung oder Senkung der cardiotoxischen Wirkungen in der Thyreotoxikose oder zur Herstellung Diagnostikums.

12. Verwendung nach Anspruch 11, wobei die Verbindung der Formel I und/oder II und/oder deren pharmazeutisch verträgliche Salze in Kombination mit anderen Arzneimitteln oder Wirkstoffen vorliegen.

13. Verwendung einer Verbindung der Formel I und/oder II und/oder deren pharmazeutisch verträgliche Salze nach Anspruch 12 in der Kombination mit cardiotoxischen oder cytotoxischen Arzneimitteln oder Wirkstoffen zur Herstellung eines Medikaments mit verminderten cardiotoxischen oder cytotoxischen Eigenschaften.

14. Verwendung einer Verbindung der Formel I und/oder II und/oder deren pharmazeutisch verträgliche Salze allein oder in Kombination mit anderen Arzneimitteln oder Wirkstoffen nach Anspruch 11 und/oder 12 zur Herstellung eines Medikaments zur Behandlung oder Prophylaxe von akuten oder chronischen Schäden, Erkrankungen oder indirekten Folgeerkrankungen von Organen und Geweben, die durch Ischämie- oder durch Reperfusionsereignisse verursacht werden.

15. Verwendung einer Verbindung der Formel I und/oder II und/oder deren pharmazeutisch verträgliche Salze allein oder in Kombination mit anderen Arzneimitteln oder Wirkstoffen nach Anspruch 11 und/oder 12 zur Herstellung eines Medikaments zur Behandlung lebensbedrohlichen Kammerflimmerns des Herzens.

16. Verwendung einer Verbindung der Formel I und/oder II und/oder deren pharmazeutisch verträgliche Salze allein oder in Kombination mit anderen Arzneimitteln oder Wirkstoffen nach Anspruch 11 und/oder 12 zur Herstellung eines Medikaments zur Behandlung oder zur Prophylaxe der Metastasierung.

17. Verwendung einer Verbindung der Formel I und/oder II und/oder deren pharmazeutisch verträgliche Salze allein oder in Kombination mit anderen Arzneimitteln oder Wirkstoffen nach Anspruch 11 und/oder 12 zur Herstellung eines Medikaments zur Behandlung oder Prophylaxe fibrotischer Erkrankungen des Herzens, der Herzinsuffizienz oder des Congestive Heart Failure.

18. Heilmittel für die humane, veterinäre und/oder phytoprotektive Anwendung, enthaltend eine wirksame Menge mindestens einer Verbindung der Formel I und/oder II und/oder deren pharmazeutisch verträgliche Salze nach einem oder mehreren der Ansprüche 1 bis 5, zusammen mit pharmazeutisch annehmbaren Träger- und Zusatzstoffen.

19. Heilmittel nach Anspruch 18, wobei die Verbindung der Formel I und/oder II und/oder deren pharmazeutisch verträgliche Salze zusammen mit pharmazeutisch annehmbaren Träger- und Zusatzstoffen in Kombination mit mindestens einem anderen pharmakologischen Wirkstoff oder Arzneimittel vorliegen.

20. Verbindung der Formel VI oder VII 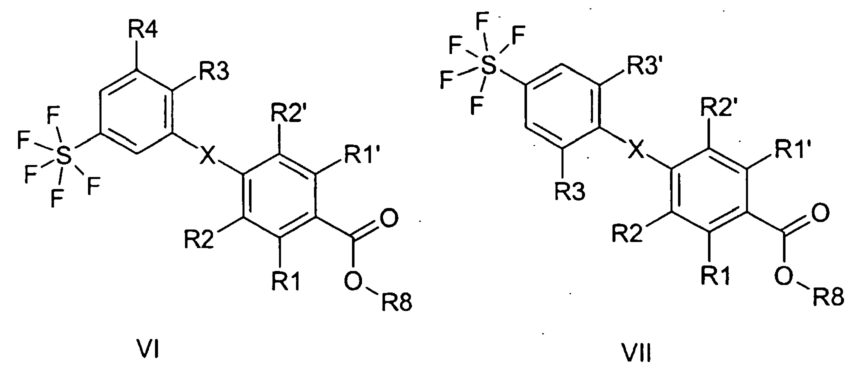 wobei R1 bis R4, R1' bis R3' und X definiert sind wie in Anspruch 1, 2, 3 oder 4 und R8 Wasserstoff oder Alkyl mit 1, 2, 3 oder 4 C-Atomen ist.

21. Verwendung der Verbindungen der Formel VI oder VII nach Anspruch 20 als Syntheseintermediat.

## Claims

1. A pentafluorosulfanylphenyl-substituted benzoylguanidine of the formula I or II 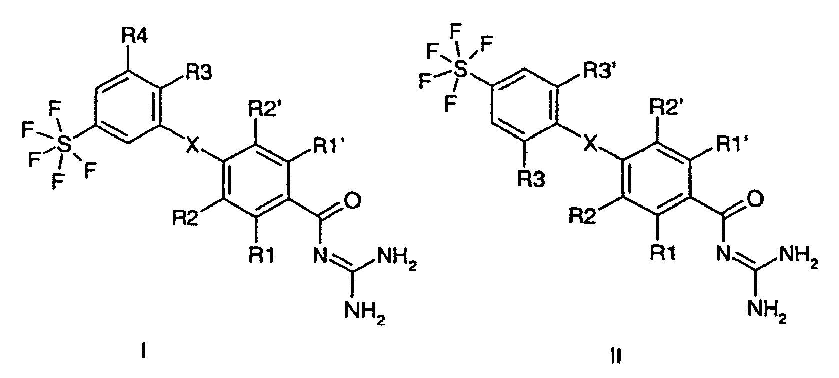 in which the meanings are
R1 and R1' independently of one another hydrogen, alkyl having 1, 2, 3 or 4 carbon atoms, Alkoxy having 1, 2, 3 or 4 carbon atoms, F, Cl, Br, I, -CN, NR5R6, -Oₐ-(CH₂)_{b}-(CF₂)_{c}-CF₃ or -(SO_{d})ₑ-(CH₂)_{f}-(CF₂)_{g}-CF₃;
R5 and R6 independently of one another hydrogen, alkyl having 1, 2, 3 or 4 carbon atoms or -CH₂-CF₃;
d zero, 1 or 2;
a, b, c, e, f and g independently of one another zero or 1;
R2 and R2' independently of one another hydrogen, F, Cl, Br, I, -CN, -SO₂CH₃, alkoxy having 1, 2, 3 or 4 carbon atoms, NR5R6, -Oa-(CH2)b-(CF2)c-CF3, -(SOₕ)ₖ-(CH₂)ₗ-(CF₂)ₘ-CF₃, alkyl having 1, 2, 3, 4, 5 or 6 carbon atoms, cycloalkyl having 3, 4, 5, 6, 7 or 8 carbon atoms, in which 1, 2, 3 or 4 hydrogen atoms may be replaced by fluorine atoms, -(CH₂)ₙ-phenyl which is unsubstituted or substituted by 1, 2 or 3 radicals selected from the group consisting of F, Cl, Br, I, -Oₒ-(CH₂)ₚ-CF₃, alkoxy having 1, 2, 3 or 4 carbon atoms, alkyl having 1, 2, 3 or 4 carbon atoms and -SO₂CH₃, or -(CH₂)_{q}-heteroaryl which is unsubstituted or substituted by 1, 2 or 3 radicals selected from the group consisting of F, Cl, Br, I, -Oᵣ-(CH₂)ₛ-CF₃, alkoxy having 1, 2, 3 or 4 carbon atoms, alkyl having 1, 2, 3 or 4 carbon atoms and -SO₂CH₃;
R5 and R6 independently of one another hydrogen, alkyl having 1, 2, 3 or 4 carbon atoms or -CH₂-CF₃;
a, b and c independently of one another zero or 1;
h zero, 1 or 2;
k zero or 1;
l ro, 1, 2, 3, or 4;
m and o independently of one another zero or 1;
p zero, 1, 2 or 3;
n zero, 1, 2, 3 or 4;
r zero or 1;
s zero, 1, 2 or 3;
q zero, 1, 2, 3, or 4;
R3 and R3' independently of one another hydrogen, F, Cl, Br, I, -CN, -SO₂CH₃, alkyl having 1, 2, 3, 4, 5 or 6 carbon atoms, alkoxy having 1, 2, 3, or 4 carbon atoms or -Oₜ-(CH₂)ᵤ-CF₃;
t zero or 1;
u zero, 1, 2 or 3;
R4 hydrogen, F, Cl, Br, I, -CN, -SO₂CH₃, NR5R6, -(SOᵥ)_{w}-(CH₂)ₓ- (CF₂)_{y}-CF₃, -O_{z}-(CH₂)ₐₐ-(CF₂)_{bb}-CF₃, alkyl having 1, 2, 3, 4, 5 or 6 carbon atoms, alkoxy having 1, 2, 3 or 4 carbon atoms or cycloalkyl having 3, 4, 5, 6, 7 or 8 carbon atoms, in which 1, 2, 3 or 4 hydrogen atoms may be replaced by fluorine atoms;
R5 and R6 independently of one another hydrogen, alkyl having 1, 2, 3 or 4 carbon atoms or -CH₂-CF₃;
v zero, 1 or 2;
x zero, 1, 2, 3 or 4;
w, y, z, aa and bb independently of one another zero or 1;
or
R4 -(CH₂)_{cc}-phenyl which is unsubstituted or substituted by 1, 2, or 3 radicals selected from the group consisting of F, Cl, Br, I, -O_{dd}-(CH₂)ₑₑ-CF₃, alkoxy having 1, 2, 3 or 4 carbon atoms, alkyl having 1, 2, 3 or 4 carbon atoms and -SO₂CH₃;
dd zero or 1;
ee zero, 1, 2 or 3;
cc zero, 1, 2, 3 or 4;
or
R4 -(CH₂)_{ff}-heteroaryl which is unsubstituted or substituted by 1, 2 or 3 radicals selected from the group consisting of F, Cl, Br, I, -O_{gg}-(CH₂)ₕₕ-CF₃, alkoxy having 1, 2, 3 or 4 carbon atoms, alkyl having 1, 2, 3 or 4 carbon atoms and -SO₂CH₃;
gg zero or 1;
hh zero, 1, 2 or 3;
ff zero, 1, 2, 3 or 4;
X a direct linkage, O, NR7, S(O)ₖₖ;
R7 hydrogen, alkyl having 1, 2, 3 or 4 carbon atoms, -(CH₂)ₘₘ-CF₃ or -SO₂CH₃
kk zero, 1 or 2;
mm zero, 1, 2 or 3;
where -Oₐ-(CH₂)_{b}-(CF₂)_{c}-CF₃ in the definitions of R1 and R1' and R2 and R2' can be selected independently of one another,
where NR5R6 in the definitions of R1 and R1', R2 and R2' and R4 can be selected independently of one another,
and the pharmaceutically acceptable salts thereof.

2. A compound of the formula I or II as claimed in claim 1, in which the meanings are:
R1 and R1' independently of one another hydrogen, alkyl having 1, 2, 3 or 4 carbon atoms, methoxy, ethoxy, F, Cl, NR5R6, -O-CH₂-CF₃ or -(SO_{d})ₑ-(CH₂)_{f}-CF₃;
R5 and R6 independently of one another hydrogen, alkyl having 1, 2, 3 or 4 carbon atoms or -CH₂-CF₃;
d zero, 1 or 2;
e and f independently of one another zero or 1;
R2 and R2' independently of one another hydrogen, F, Cl, -SO₂CH₃, -(SOₕ)ₖ- (CH₂)ₗ-CF₃, alkyl having 1, 2, 3 or 4 carbon atoms, cycloalkyl having 3, 4, 5, 6 or 7 carbon atoms, in which 1, 2, 3 or 4 hydrogen atoms may be replaced by fluorine atoms, phenyl which is unsubstituted or substituted by 1-2 radicals selected from the group consisting of F, Cl, -Oₒ-(CH₂)ₚ-CF₃, methoxy, ethoxy, alkyl having 1, 2, 3 or 4 carbon atoms and -SO₂CH₃, or heteroaryl which is unsubstituted or substituted by 1-2 radicals selected from the group consisting of F, Cl, -Oᵣ(CH₂)ₛ-CF₃, methoxy, ethoxy, alkyl having 1, 2, 3 or 4 carbon atoms and -SO₂CH₃;
h zero, 1 or 2;
k zero or 1;
l zero, 1, 2, 3, or 4;
o zero or 1;
p zero, 1, 2 or 3;
r zero or 1;
s zero, 1, 2 or 3;
R3 and R3' independently of one another hydrogen, F, Cl, -SO₂CH₃, alkyl having 1, 2, 3 or 4 carbon atoms, methoxy, ethoxy or -Oₜ-(CH₂)ᵤ-CF₃,
t zero or 1;
u zero, 1, 2 or 3;
R4 hydrogen, F, Cl, -SO₂CH₃, -(SOᵥ)_{w}-(CH₂)ₓ-CF₃, -O_{z}-(CH₂)ₐₐ-CF₃, alkyl having 1, 2, 3 or 4 carbon atoms, methoxy, ethoxy or cycloalkyl having 3, 4, 5, 6 or 7 carbon atoms, in which 1, 2, 3 or 4 hydrogen atoms may be replaced by fluorine atoms;
v zero, 1 or 2;
w, x, z, and aa independently of one another zero or 1;
or
R4 phenyl which is unsubstituted or substituted by 1-2 radicals selected from the group consisting of F, Cl, -O_{dd}-(CH₂)ₑₑ-CF₃, methoxy, ethoxy, alkyl having 1, 2, 3 or 4 carbon atoms and -SO₂CH₃;
dd and ee independently of one another zero or 1;
or
R4 heteroaryl which is unsubstituted or substituted by 1-2 radicals selected from the group consisting of F, Cl, -O_{gg}-(CH₂)ₕₕ-CF₃, methoxy, ethoxy, alkyl having 1, 2, 3 or 4 carbon atoms and -SO₂CH₃;
gg and hh independently of one another zero or 1;
X a direct linkage, O, NR7, S(O)ₖₖ;
R7 hydrogen, alkyl having 1, 2, 3 or 4 carbon atoms, -CH₂-CF₃ or -SO₂CH₃;
kk zero, 1 or 2;
and the pharmaceutically acceptable salts thereof.

3. A compound of the formula I or II as claimed in claim 1 or 2, in which the meanings are:
R1 and R1' independently of one another hydrogen, alkyl having 1, 2, 3 or 4 carbon atoms, methoxy, ethoxy, F, Cl, NR5R6, -O-CH₂-CF₃ or -(SO_{d})ₑ-(CH₂)_{f}-CF₃;
R5 and R6 independently of one another hydrogen, alkyl having 1, 2, 3 or 4 carbon atoms or -CH₂-CF₃;
d zero, 1 or 2;
e and f independently of one another zero or 1;
R2 and R2' independently of one another hydrogen, F, Cl, -SO₂CH₃, -(SOₕ)ₖ- (CH₂)ₗ-CF₃, methyl, cycloalkyl having 3, 4, 5, 6 or 7 carbon atoms, in which 1, 2, 3 or 4 hydrogen atoms may be replaced by fluorine atoms, phenyl which is unsubstituted or substituted by a radical selected from the group consisting of F, Cl, -Oₒ-(CH₂)ₚ-CF₃, methoxy, ethoxy, alkyl having 1, 2, 3 or 4 carbon atoms and -SO₂CH₃, or heteroaryl which is unsubstituted or substituted by a radical selected from the group consisting of F, Cl, -Oᵣ-(CH₂)ₛ-CF₃, methoxy, ethoxy, alkyl having 1, 2, 3 or 4 carbon atoms and -SO₂CH₃;
h zero, 1 or 2;
k, l, o, p, r and s independently of one another zero or 1;
R3 and R3' independently of one another hydrogen, F, Cl, -SO₂CH₃, methyl, methoxy, ethoxy or -Oₜ-(CH₂)ᵤ-CF₃;
t and u independently of one another zero or 1;
R4 hydrogen, F, Cl, -SO₂CH₃, -(SOᵥ)_{w}-(CH₂)ₓ-CF₃, -O_{z}-(CH₂)ₐₐ-CF₃, methyl, methoxy, ethoxy or cycloalkyl having 3, 4, 5, 6 or 7 carbon atoms, in which 1, 2, 3 or 4 hydrogen atoms may be replaced by fluorine atoms;
v zero, 1 or 2;
w, x, z and aa independently of one another zero or 1;
X a direct linkage, O, NR7 or S(O)ₖₖ;
R7 hydrogen, methyl, ethyl, -CH₂-CF₃ or -SO₂CH₃;
kk zero, 1 or 2;
and the pharmaceutically acceptable salts thereof.

4. A compound of the formula I or II as claimed in any of claims 1 to 3, in which the meanings are:
R1 and R1' independently of one another hydrogen, methyl, F, Cl, -CF₃ or -O-CH₂-CF₃;
R2 and R2' independently of one another hydrogen, F, Cl, -SO₂CH₃, -SO₂-CF₃, -CF₃ or methyl;
R3 and R3' independently of one another hydrogen, F, Cl, -SO₂CH₃, methyl, -CF₃ or -O-CH₂-CF₃; R4 hydrogen, F, Cl, -SO₂CH₃, -O-CH₂-CF₃ or methyl;
X a direct linkage, O, NR7 or S(O)ₖₖ;
R7 hydrogen, methyl, ethyl, -CH₂-CF₃ or -SO₂CH₃;
kk zero, 1 or 2;
and the pharmaceutically acceptable salts thereof.

5. A compound of the formula I or II as claimed in any of claims 1 to 4 selected from the group consisting of:
N-[5-methanesulfonyl-2-methyl-4-(3-pentafluorosulfanylphenoxy)benzoyl]-guanidine,
N-[5-methanesulfonyl-2-methyl-4-(4-pentafluorosulfanylphenoxy)benzoyl]-guanidine,
N-[5-methanesulfonyl-2-methyl-4-(4-pentafluorosulfanylphenylsulfanyl)-benzoyl]guanidine,
N-[5-methanesulfonyl-2-methyl-4-(4-pentafluorosulfanylphenylsulfonyl)-benzoyl]guanidine,
N-[5-methanesulfonyl-2-methyl-4-(3-pentafluorosulfanylphenylamino)-benzoyl]guanidine,
N-[5-methanesulfonyl-2-methyl-4-(4-pentafluorosulfanylphenylamino)-benzoyl]guanidine,
N-[5-methanesulfonyl-2-methyl-4-(4-pentafluorosulfanylphenylamino)-benzoyl]guanidine,
N-{5-methanesulfonyl-2-methyl-4-[methyl(3-pentafluorosulfanylphenyl)-amino]benzoyl}guanidine,
N-{5-methanesulfonyl-2-methyl-4-[methyl(4-pentafluorosulfanylphenyl)-amino]benzoyl}guanidine,
N-(2-methanesulfonyl-5-methyl-4'-pentafluorosulfanylbiphenyl-4-carbonyl)-guanidine,
N-(2-methanesulfonyl-5-methyl-3'-pentafluorosulfanylbiphenyl-4-carbonyl)-guanidine
and
N-(2-methanesulfonyl-5,2'-dimethyl-4'-pentafluorosulfanylbiphenyl-4-carbonyl)guanidine
and its pharmaceutically acceptable salts.

6. A process for preparing a compound of the formula I and/or II and/or the pharmaceutically acceptable salts thereof, in which X is oxygen, which comprises
a) reacting a phenol of the formula III or IV with an aromatic of the formula V to give a compound of the formula VIa or VIIa
and
b) reacting a compound of the formula VIa or VIIa with guanidine to give the acylguanidine of the formula la or IIa, 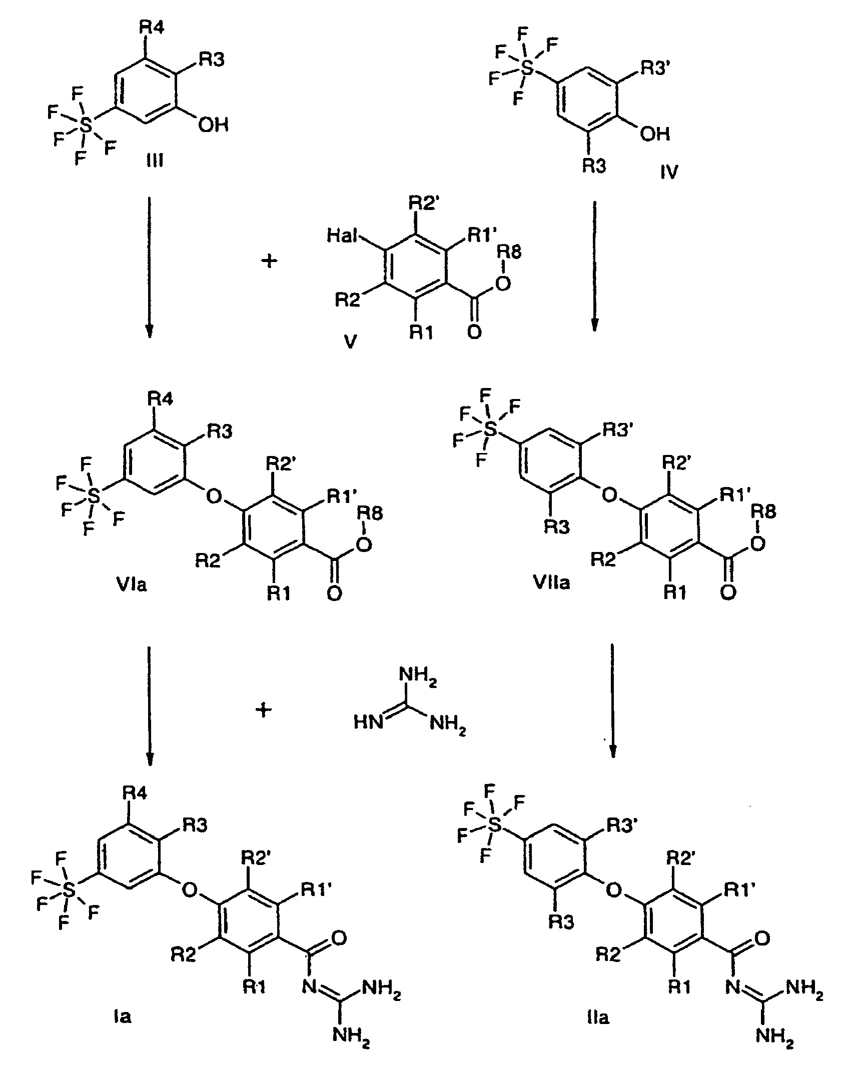
in which R1, R1', R2, R2', R3, R3' and R4 possess the meaning stated in claims 1, 2, 3 and/or 4, and in which
Hal is F, Cl, Br or I,
R8 is hydrogen or alkyl having 1, 2, 3 or 4 carbon atoms.

7. A process for preparing a compound of the formula I or II and/or the pharmaceutically acceptable salts thereof, in which X is NR7, which comprises
a) sulfonating an aniline of the formula VIII or IX with a sulfonyl chloride to give a compound of the formula X or XI,
b) reacting a compound of the formula X or XI with an aromatic of the formula V to give a compound of the formula Vlb or VIIb,
c) for preparing a compound of the formula Ib or IIb in which R7 is other than hydrogen, derivatizing a compound of the formula VIb or VIIb to give a compound of formula VIc or Vllc,
and
d) reacting a compound of the formula VIb/c or VIIb/c with guanidine to give an acylguanidine of the formula Ib or IIb 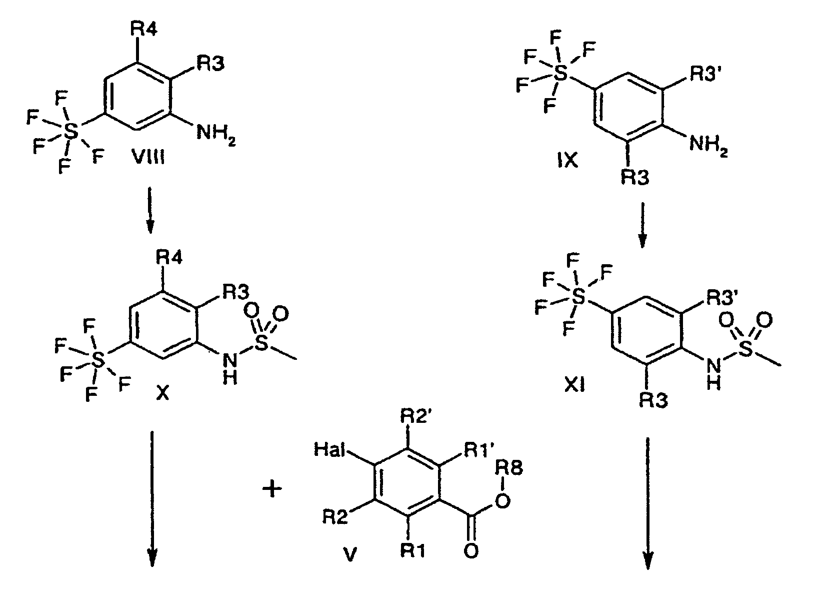 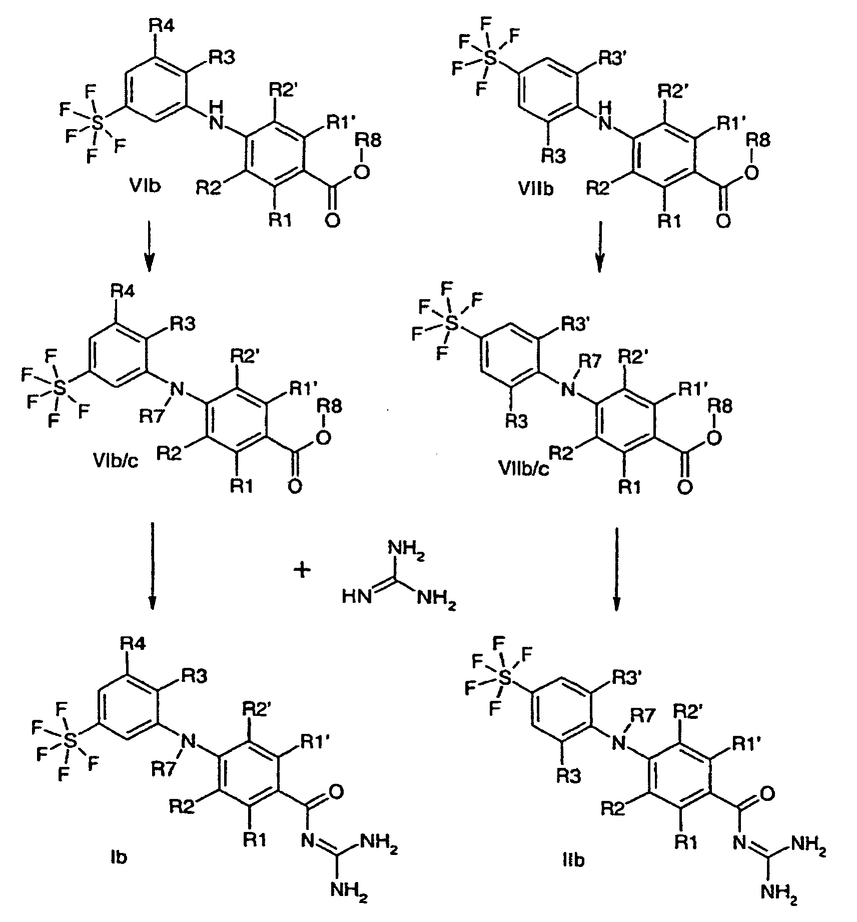
in which R1, R1', R2, R2', R3, R3', R4 and R7 possess the meaning stated in claims 1, 2, 3 and/or 4, and in which
Hal is F, Cl, Br or I,
R8 is hydrogen or alkyl having 1, 2, 3 or 4 carbon atoms.

8. A process for preparing a compound of the formula I or II and/or the pharmaceutically acceptable salts thereof, in which X is S(O)ₖₖ, which comprises
a) reacting a thiophenol of the formula XII or XIII with an aromatic of the formula V to give a compound of the formula VIe or VIIe,
b) for preparing a compound of the formula Ic or IIc in which kk is other than zero, oxidizing a compound of the formula VIe or VIIe to give a compound of the formula Vlf or VIIf
and
c) reacting a compound of the formula VIe/f or VIIe/f with guanidine to give an acylguanidine of the formula Ic or IIc 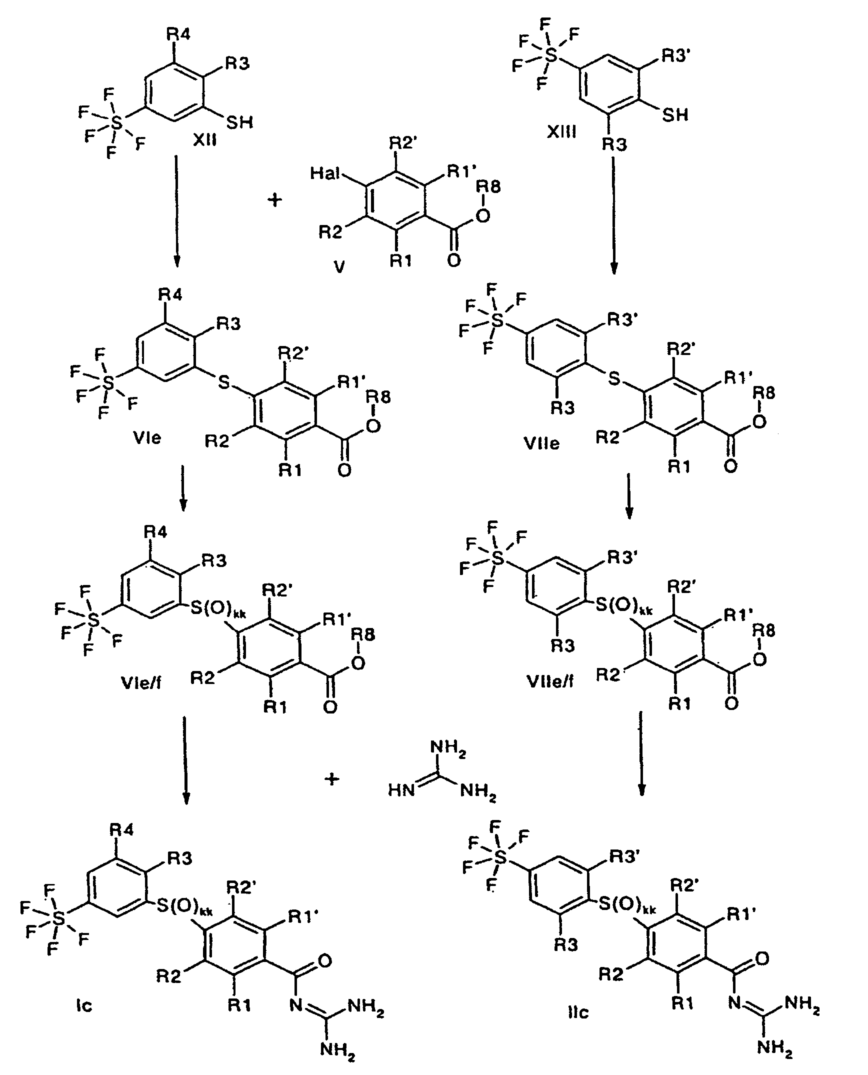
in which R1, R1', R2, R2', R3, R3', R4 and kk possess the meaning stated in claims 1, 2, 3 and/or 4 and in which
Hal is F, Cl, Br or I,
R8 is hydrogen or alkyl having 1, 2, 3 or 4 carbon atoms.

9. A process for preparing a compound of the formula I or II and/or the pharmaceutically acceptable salts thereof, in which X is a direct bond, which comprises
a) coupling a halide of the formula XIV or XV in a Suzuki coupling reaction with a benzoic ester of the formula Va to give a biphenyl derivative of the formula VIg or Vllg
and
b) reacting a compound of the formula VIg or Vllg with guanidine to give an acylguanidine of the formula Id or IId 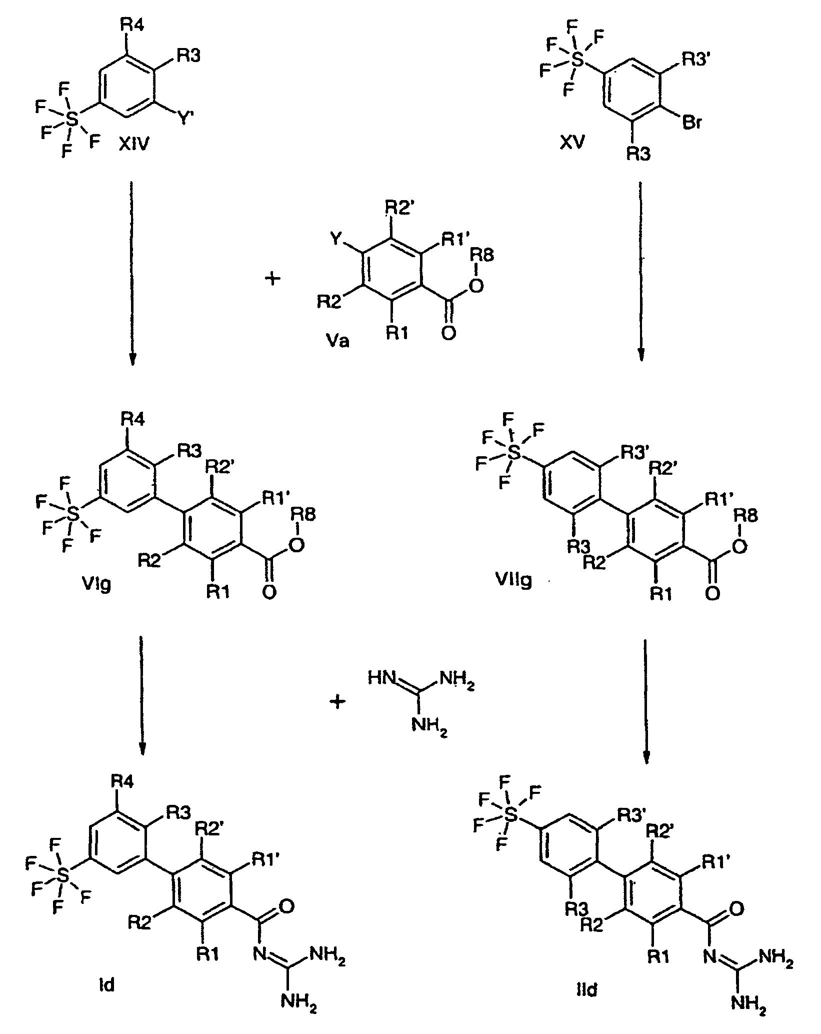
in which R1, R1', R2, R2', R3, R3' and R4 possess the meaning stated in claims 1, 2, 3 and/or 4 and in which
Y and Y' independently of one another are Cl, Br or I
R8 is hydrogen or alkyl having 1, 2, 3 or 4 carbon atoms.

10. The compound of the formula I and/or II and/or the pharmaceutically acceptable salts thereof as claimed in one or more of claims 1 to 5 for use as medicament.

11. The use of a compound of the formula I and/or II and/or the pharmaceutically acceptable salts thereof as claimed in one or more of claims 1 to 5 for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, such as epilepsy or centrally induced convulsions, of disorders of the central nervous system, of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria or of coccidiosis in poultry, or for producing a medicament for use for surgical operations and organ transplantations, for preserving and storing transplants for surgical procedures, for preventing age-related tissue change, for producing a medicament directed against aging or for producing a medicament for prolonging life, for the treatment or reduction of the cardiotoxic effects in thyrotoxicosis or for producing a diagnostic aid.

12. The use as claimed in claim 11, the compound of the formula I and/or II and/or the pharmaceutically acceptable salts thereof being present in combination with other medicaments or active ingredients.

13. The use of a compound of the formula I and/or II and/or the pharmaceutically acceptable salts thereof as claimed in claim 12 in combination with cardiotoxic or cytotoxic medicaments or active ingredients for producing a medicament with reduced cardiotoxic or cytotoxic properties.

14. The use of a compound of the formula I and/or II and/or the pharmaceutically acceptable salts thereof alone or in combination with other medicaments or active ingredients as claimed in claim 11 and/or 12 for producing a medicament for the treatment or prophylaxis of acute or chronic damage, disorders or indirect sequelae of organs and tissues caused by ischemic or reperfusion events.

15. The use of a compound of the formula I and/or II and/or the pharmaceutically acceptable salts thereof alone or in combination with other medicaments or active ingredients as claimed in claim 11 and/or 12 for producing a medicament for the treatment of life-threatening cardiac ventricular fibrillation.

16. The use of a compound of the formula I and/or II and/or the pharmaceutically acceptable salts thereof alone or in combination with other medicaments or active ingredients as claimed in claim 11 and/or 12 for producing a medicament for the treatment or prophylaxis of metastasis.

17. The use of a compound of the formula I and/or II and/or the pharmaceutically acceptable salts thereof alone or in combination with other medicaments or active ingredients as claimed in claim 11 and/or 12 for producing a medicament for the treatment or prophylaxis of fibrotic disorders of the heart, of heart failure or of congestive heart failure.

18. A medicine for human, veterinary and/or phytoprotective use comprising an effective amount of at least one compound of the formula I and/or II and/or the pharmaceutically acceptable salts thereof as claimed in one or more of claims 1 to 5, together with pharmaceutically acceptable carriers and additives.

19. A medicine as claimed in claim 18, the compound of the formula I and/or II and/or the pharmaceutically acceptable salts thereof being present together with pharmaceutically acceptable carriers and additives in combination with at least one other pharmacological active ingredient or medicament.

20. A compound of the formula VI or VII 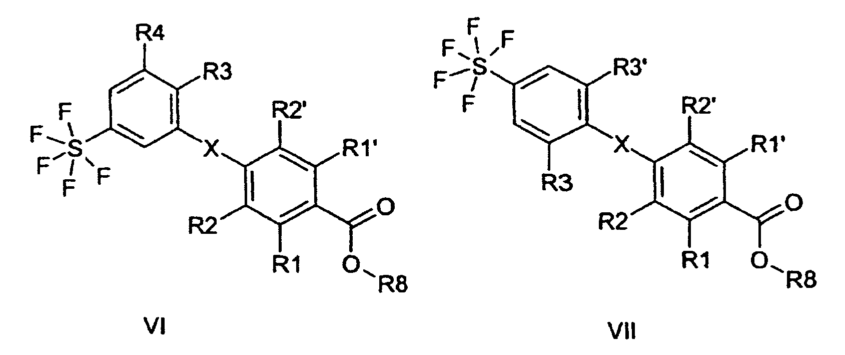 where R1 to R4, R1' to R3' and X are defined as in claim 1, 2, 3 or 4 and R8 is hydrogen or alkyl having 1, 2, 3 or 4 carbon atoms.

21. The use of a compound of the formula VI or VII as claimed in claim 20 as a synthetic intermediate.

## Revendications

1. Benzoylguanidines substituées par
pentafluorosulfanylphényle de formule I ou II 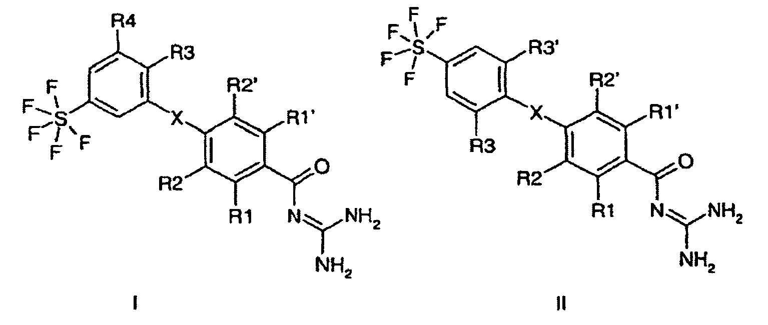 où
R1 et R1' signifient, indépendamment l'un de l'autre, hydrogène, alkyle comprenant 1, 2, 3 ou 4 atomes de carbone, alcoxy comprenant 1, 2, 3 ou 4 atomes de carbone, F, Cl, Br, I, -CN, NR5R6, -Oₐ-(CH₂)_{b}- (CF₂)_{c}-CF₃ ou -(SO_{d})ₑ-(CH₂)_{f}-(CF₂)_{g}-CF₃ ;
R5 et R6 signifient, indépendamment l'un de l'autre, hydrogène, alkyle comprenant 1, 2, 3 ou 4 atomes de carbone ou -CH₂-CF₃ ;
d vaut zéro, 1 ou 2 ;
a, b, c, e, f et g valent, indépendamment l'un de l'autre, zéro ou 1 ;
R2 et R2' signifient, indépendamment l'un de l'autre, hydrogène, F, Cl, Br, I, -CN, -SO₂CH₃, alcoxy comprenant 1, 2, 3 ou 4 atomes de carbone, NR5R6, -Oₐ-(CH₂)_{b}-(CF₂)_{c}-CF₃, - (SOₕ)ₖ-(CH₂)ₗ-(CF₂)ₘ-CF₃, alkyle comprenant 1, 2, 3, 4, 5 ou 6 atomes de carbone, cycloalkyle comprenant 3, 4, 5, 6, 7 ou 8 atomes de carbone, dans lequel 1, 2, 3 ou 4 atomes d'hydrogène peuvent être remplacés par des atomes de fluor, -(CH₂)ₙ-phényle, qui est non substitué ou substitué par 1, 2 ou 3 radicaux choisis dans le groupe constitué par F, Cl, Br, I, -Oₒ-(CH₂)ₚ-CF₃, alcoxy comprenant 1, 2, 3 ou 4 atomes de carbone, alkyle comprenant 1, 2, 3 ou 4 atomes de carbone et -SO₂CH₃, ou -(CH₂)_{q}-hétéroaryle, qui est non substitué ou substitué par 1, 2 ou 3 radicaux choisis dans le groupe constitué par F, Cl, Br, I, -Oᵣ-(CH₂)ₛ-CF₃, alcoxy comprenant 1, 2, 3 ou 4 atomes de carbone, alkyle comprenant 1, 2, 3 ou 4 atomes de carbone et -SO₂CH₃ ;
R5 et R6 signifient, indépendamment l'un de l'autre, hydrogène, alkyle comprenant 1, 2, 3 ou 4 atomes de carbone ou -CH₂-CF₃ ;
a, b et c valent, indépendamment l'un de l'autre, zéro ou 1 ;
h vaut zéro, 1 ou 2 ;
k vaut zéro ou 1 ;
l vaut zéro, 1, 2, 3 ou 4 ;
m et o valent, indépendamment l'un de l'autre, zéro ou 1 ;
p vaut zéro, 1, 2 ou 3 ;
n vaut zéro, 1, 2, 3 ou 4 ;
r vaut zéro ou 1 ;
s vaut zéro, 1, 2 ou 3 ;
q vaut zéro, 1, 2, 3 ou 4 ;
R3 et R3' signifient, indépendamment l'un de l'autre, hydrogène, F, Cl, Br, I, -CN, -SO₂CH₃, alkyle comprenant 1, 2, 3, 4, 5 ou 6 atomes de carbone, alcoxy comprenant 1, 2, 3 ou 4 atomes de carbone ou -Oₜ-(CH₂)ᵤ-CF_{3 ;}
t vaut zéro ou 1 ;
u vaut zéro, 1, 2 ou 3 ;
R4 signifie hydrogène, F, Cl, Br, I, -CN, -SO₂CH₃, NR5R6, -(SOᵥ)_{w}-(CH₂)ₓ-(CF₂)_{y}-CF₃, -O_{z}-(CH₂)ₐₐ-(CF₂)_{bb}- CF₃, alkyle comprenant 1, 2, 3, 4, 5 ou 6 atomes de carbone, alcoxy comprenant 1, 2, 3 ou 4 atomes de carbone ou cycloalkyle comprenant 3, 4, 5, 6, 7 ou 8 atomes de carbone, où 1, 2, 3 ou 4 atomes d'hydrogène peuvent être remplacés par des atomes de fluor ;
R5 et R6 signifient, indépendamment l'un de l'autre, hydrogène, alkyle comprenant 1 2, 3 ou 4 atomes de carbone ou -CH₂-CF₃ ;
v vaut zéro, 1 ou 2 ;
x vaut zéro, 1, 2, 3 ou 4 ;
w, y, z, aa et bb valent, indépendamment l'un de l'autre, zéro ou 1 ;
ou
R4 signifie -(CH₂)_{cc}-phényle, qui est non substitué ou substitué par 1, 2 ou 3 radicaux, choisis dans le groupe constitué par F, Cl, Br, I, -O_{dd}-(CH₂)ₑₑ-CF₃, alcoxy comprenant 1, 2, 3 ou 4 atomes de carbone, alkyle comprenant 1, 2, 3 ou 4 atomes de carbone et -SO₂CH₃ ;
dd vaut zéro ou 1 ;
ee vaut zéro, 1, 2 ou 3 ;
cc vaut zéro, 1, 2, 3 ou 4 ;
ou
R4 signifie -(CH₂)_{ff}-hétéroaryle, qui est non substitué ou substitué par 1, 2 ou 3 radicaux, choisis dans le groupe constitué par F, Cl, Br, I, -O_{gg}-(CH₂)ₙₙ-CF₃, alcoxy comprenant 1, 2, 3 ou 4 atomes de carbone, alkyle comprenant 1, 2, 3 ou 4 atomes de carbone et -SO₂CH₃ ;
gg vaut zéro ou 1 ;
hh vaut zéro, 1, 2 ou 3 ;
ff vaut zéro, 1, 2, 3 ou 4 ;
X signifie une liaison directe, O, NR7, S(O)_{kk ;}
R7 signifie hydrogène, alkyle comprenant 1, 2, 3 ou 4 atomes de carbone, -(CH₂)ₘₘ-CF₃ ou -SO₂CH₃
kk vaut zéro, 1 ou 2 ;
mm vaut zéro, 1, 2 ou 3 ;
où -Oₐ-(CH₂)_{b}-(CF₂)_{c}-CF₃ dans les définitions de R1 et R1' et R2 et R2' peut être choisi indépendamment l'un de l'autre,
où NR5R6 dans les définitions de R1 et R1', R2 et R2' et R4 peut être choisi indépendamment l'un de l'autre, ainsi que leurs sels pharmaceutiquement acceptables.

2. Composé de formule I ou II selon la revendication 1, où
R1 et R1' signifient, indépendamment l'un de l'autre, hydrogène, alkyle comprenant 1, 2, 3 ou 4 atomes de carbone, méthoxy, éthoxy, F, Cl, NR5R6, -O-CH₂- CF₃ ou -(SO_{d})ₑ-(CH₂)_{f}-CF₃ ;
R5 et R6 signifient, indépendamment l'un de l'autre, hydrogène, alkyle comprenant 1 2, 3 ou 4 atomes de carbone ou -CH₂-CF₃ ;
d vaut zéro, 1 ou 2 ;
e et f valent, indépendamment l'un de l'autre, zéro ou 1 ;
R2 et R2' signifient, indépendamment l'un de l'autre, hydrogène, F, Cl, -SO₂CH₃, -(SOₕ)ₖ-(CH₂)ₗ-CF₃, alkyle comprenant 1, 2, 3 ou 4 atomes de carbone, cycloalkyle comprenant 3, 4, 5, 6 ou 7 atomes de carbone, où 1, 2, 3 ou 4 atomes d'hydrogène peuvent être remplacés par des atomes de fluor, phényle, qui est non substitué ou substitué par 1- 2 radicaux choisis dans le groupe constitué par F, Cl, -Oₒ-(CH₂)ₚ-CF₃, méthoxy, éthoxy, alkyle comprenant 1, 2, 3 ou 4 atomes de carbone et -SO₂CH₃, ou hétéroaryle, qui est non substitué ou substitué par 1-2 radicaux choisis dans le groupe constitué par F, Cl, -Oᵣ-(CH₂)ₛ-CF₃, méthoxy, éthoxy, alkyle comprenant 1, 2, 3 ou 4 atomes de carbone et -SO₂CH₃ ;
h vaut zéro, 1 ou 2 ;
k vaut zéro ou 1 ;
l vaut zéro, 1, 2, 3 ou 4 ;
o vaut zéro ou 1 ;
p vaut zéro, 1, 2 ou 3 ;
r vaut zéro ou 1 ;
s vaut zéro, 1, 2 ou 3 ;
R3 et R3' signifient, indépendamment l'un de l'autre, hydrogène, F, Cl, -SO₂CH₃, alkyle comprenant 1, 2, 3 ou 4 atomes de carbone, méthoxy, éthoxy ou -Oₜ- (CH₂)ᵤ-CF₃ ;
t vaut zéro ou 1 ;
u vaut zéro, 1, 2 ou 3 ;
R4 signifie hydrogène, F, Cl, -SO₂CH₃, -(SOᵥ)_{w}-(CH₂)ₓ- CF₃, -O_{z}-(CH₂)ₐₐ-CF₃, alkyle comprenant 1, 2, 3 ou 4 atomes de carbone, méthoxy, éthoxy ou cycloalkyle comprenant 3, 4, 5, 6 ou 7 atomes de carbone, où 1, 2, 3 ou 4 atomes d'hydrogène peuvent être remplacés par des atomes de fluor ;
v vaut zéro, 1 ou 2 ;
w, x, z et aa valent, indépendamment l'un de l'autre, zéro ou 1 ;
ou
R4 signifie phényle, qui est non substitué ou substitué par 1-2 radicaux choisis dans le groupe constitué par F, Cl, -O_{dd}-(CH₂)ₑₑ-CF₃, méthoxy, éthoxy, alkyle comprenant 1, 2, 3 ou 4 atomes de carbone et -SO₂CH₃ ;
dd et ee valent, indépendamment l'un de l'autre, zéro ou 1 ;
ou
R4 signifie hétéroaryle, qui est non substitué ou substitué par 1-2 radicaux choisis dans le groupe constitué par F, Cl, -Ogg- (CH₂)ₕₕ-CF₃, méthoxy, éthoxy, alkyle comprenant 1, 2, 3 ou 4 atomes de carbone et -SO₂CH₃ ;
gg et hh valent, indépendamment l'un de l'autre, zéro ou 1 ;
X signifie une liaison directe, O, NR7, S(O)ₖₖ ;
R7 signifie hydrogène, alkyle comprenant 1, 2, 3 ou 4 atomes de carbone, -CH₂-CF₃ ou -SO₂CH₃ ;
kk vaut zéro, 1 ou 2 ;
ainsi que leurs sels pharmaceutiquement acceptables.

3. Composé de formule I ou II selon la revendication 1 ou 2, où
R1 et R1' signifient, indépendamment l'un de l'autre, hydrogène, alkyle comprenant 1, 2, 3 ou 4 atomes de carbone, méthoxy, éthoxy, F, Cl, NR5R6, -O-CH₂- CF₃ ou -(SO_{d})ₑ-(CH₂)_{f}-CF₃ ;
R5 et R6 signifient, indépendamment l'un de l'autre, hydrogène, alkyle comprenant 1 2, 3 ou 4 atomes de carbone ou -CH₂-CF₃ ;
d vaut zéro, 1 ou 2 ;
e et f valent, indépendamment l'un de l'autre, zéro ou 1 ;
R2 et R2' signifient, indépendamment l'un de l'autre, hydrogène, F, Cl, -SO₂CH₃, - (SOₕ)ₖ-(CH₂)ₗ-CF₃, méthyle, cycloalkyle comprenant 3, 4, 5, 6 ou 7 atomes de carbone, où 1, 2, 3 ou 4 atomes d'hydrogène peuvent être remplacés par des atomes de fluor, phényle, qui est non substitué ou substitué par un radical choisi dans le groupe constitué par F, Cl, -Oₒ-(CH₂)ₚ-CF₃, méthoxy, éthoxy, alkyle comprenant 1, 2, 3 ou 4 atomes de carbone et -SO₂CH₃, ou hétéroaryle, qui est non substitué ou substitué par un radical choisi dans le groupe constitué par F, Cl, -Or- (CH₂)ₛ-CF₃, méthoxy, éthoxy, alkyle comprenant 1, 2, 3 ou 4 atomes de carbone et -SO₂CH₃ ;
h vaut zéro, 1 ou 2 ;
k, l, o, p, r et s valent, indépendamment l'un de l'autre, zéro ou 1 ;
R3 et R3' signifient, indépendamment l'un de l'autre, hydrogène, F, Cl, -SO₂CH₃, méthyle, méthoxy, éthoxy ou -Oₜ-(CH₂)ᵤ-CF₃ ;
t et u valent, indépendamment l'un de l'autre, zéro ou 1 ;
R4 signifie hydrogène, F, Cl, -SO₂CH₃, -(SOᵥ)_{w}-(CH₂)ₓ- CF₃, -O_{z}-(CH₂)ₐₐ-CF₃, méthyle, méthoxy, éthoxy ou cycloalkyle comprenant 3, 4, 5, 6 ou 7 atomes de carbone, où 1, 2, 3 ou 4 atomes d'hydrogène peuvent être remplacés par des atomes de fluor ;
v vaut zéro, 1 ou 2 ;
w, x, z et aa valent, indépendamment l'un de l'autre, zéro ou 1 ;
X signifie une liaison directe, 0, NR7 ou S(O)ₖₖ ;
R7 signifie hydrogène, méthyle, éthyle, -CH₂-CF₃ ou -SO₂CH₃ ;
kk vaut zéro, 1 ou 2 ;
ainsi que leurs sels pharmaceutiquement acceptables.

4. Composé de formule I ou II selon l'une
quelconque des revendications 1 à 3, où
R1 et R1' signifient, indépendamment l'un de l'autre, hydrogène, méthyle, F, Cl, -CF₃ ou -O-CH₂-CF₃ ;
R2 et R2' signifient, indépendamment l'un de l'autre, hydrogène, F, Cl, -SO₂CH₃, -SO₂-CF₃, -CF₃ ou méthyle ;
R3 et R3' signifient, indépendamment l'un de l'autre, hydrogène, F, Cl, -SO₂CH₃, méthyle, -CF₃ ou -O-CH₂- CF₃ ;
R4 signifie hydrogène, F, Cl, -SO₂CH₃, -O-CH₂-CF₃ ou méthyle ;
X signifie une liaison directe, O, NR7 ou S(O)ₖₖ ;
R7 signifie hydrogène, méthyle, éthyle, -CH₂-CF₃ ou -SO₂CH₃ ;
kk vaut zéro, 1 ou 2 ;
ainsi que leurs sels pharmaceutiquement acceptables.

5. Composé de formule I ou II selon l'une quelconque des revendications 1 à 4, choisi dans le groupe formé par :
- la N-[5-méthanesulfonyl-2-méthyl-4-(3-pentafluorosulfanylphénoxy)-benzoyl]-guanidine,
- la N-[5-méthanesulfonyl-2-méthyl-4-(4-pentafluorosulfanylphénoxy)-benzoyl]-guanidine,
- la N-[5-méthanesulfonyl-2-méthyl-4-(4-pentafluorosulfanylphénylsulfanyl)-benzoyl]-guanidine,
- la N-[5-méthanesulfonyl-2-méthyl-4-(4-pentafluorosulfanylphénylsulfonyl)-benzoyl]-guanidine,
- la N-[5-méthanesulfonyl-2-méthyl-4-(3-pentafluorosulfanylphénylamino)-benzoyl]-guanidine,
- la N-[5-méthanesulfonyl-2-méthyl-4-(4-pentafluorosulfanylphénylamino)benzoyl]-guanidine,
- la N-[5-méthanesulfonyl-2-méthyl-4-(4-pentafluorosulfanylphénylamino)-benzoyl]-guanidine,
- la N-{5-méthanesulfonyl-2-méthyl-4-[méthyl-(3-pentafluorosulfanylphényl)-amino]-benzoyl}-guanidine,
- la N-{5-méthanesulfonyl-2-méthyl-4-[méthyl-(4-pentafluorosulfanylphényl)-amino]-benzoyl}-guanidine,
- la N-(2-méthanesulfonyl-5-méthyl-4'-pentafluorosulfanylbiphényl-4-carbonyl)-guanidine,
- la N-(2-méthanesulfonyl-5-méthyl-3'-pentafluorosulfanylbiphényl-4-carbonyl)-guanidine
et
- la N-(2-méthanesulfonyl-5,2'-diméthyl-4'-pentafluorosulfanylbiphényl-4-carbonyl)-guanidine ainsi que leurs sels pharmaceutiquement acceptables.

6. Procédé pour la préparation d'un composé des formules I ou II et/ou de ses sels pharmaceutiquement acceptables, où X représente oxygène, **caractérisé en ce que**
a) on transforme un phénol des formules III ou IV avec un aromatique de formule V en un composé des formules VIa ou VIIa
et
b) on transforme un composé des formules VIa ou VIIa avec de la guanidine en acylguanidine des formules Ia ou IIa, 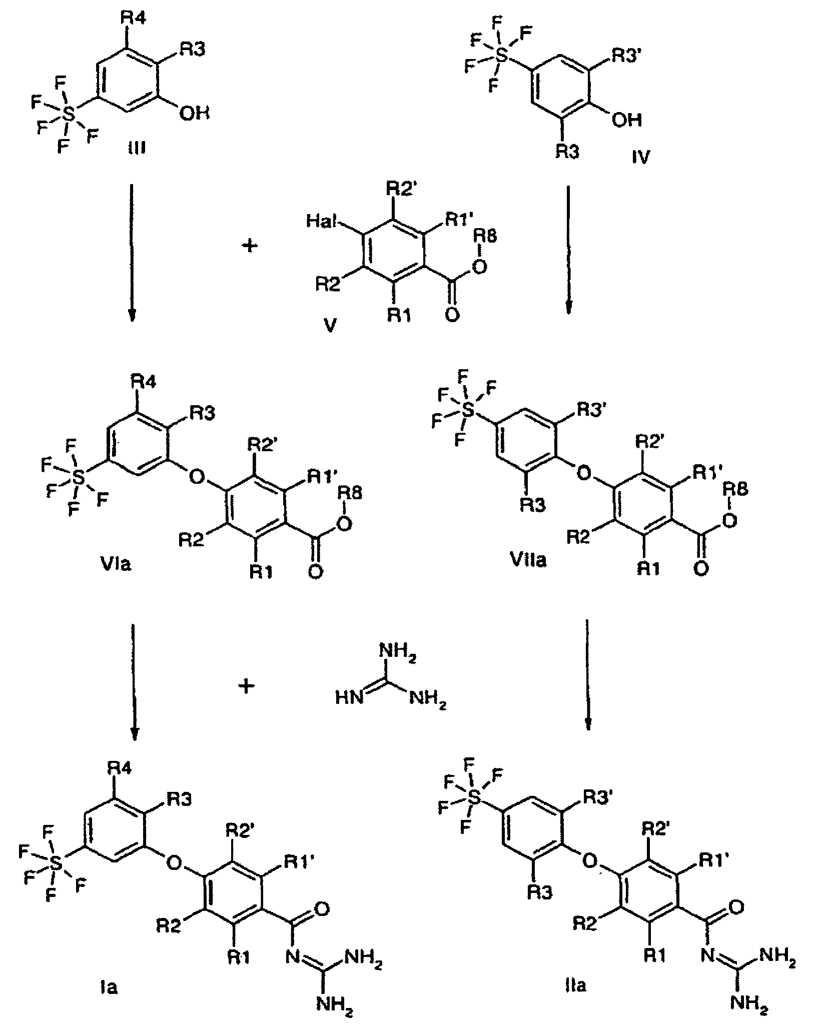
où R1, R1', R2, R2', R3, R3' et R4 présentent la signification indiquée dans les revendications 1, 2, 3 et/ou 4 et où
Hal signifie F, Cl, Br ou I,
R8 signifie hydrogène ou alkyle comprenant 1, 2, 3 ou 4 atomes de carbone.

7. Procédé pour la préparation d'un composé des formules I ou II et/ou de ses sels pharmaceutiquement acceptables, où X représente NR7, **caractérisé en ce que**
a) on sulfone une aniline des formules VIII ou IX avec un chlorure d'acide sulfonique en un composé des formules X ou XI,
b) on transforme un composé des formules X ou XI avec un aromatique de formule V en un composé des formules VIb ou VIIb,
c) pour la préparation d'un composé des formules Ib ou IIb, où R7 est différent d'hydrogène, on dérive un composé des formules VIb ou VIIb en un composé des formules VIc ou VIIc,
et
d) on transforme un composé des formules VIb/c ou VIIb/c avec de la guanidine en une acylguanidine des formules Ib ou IIb, 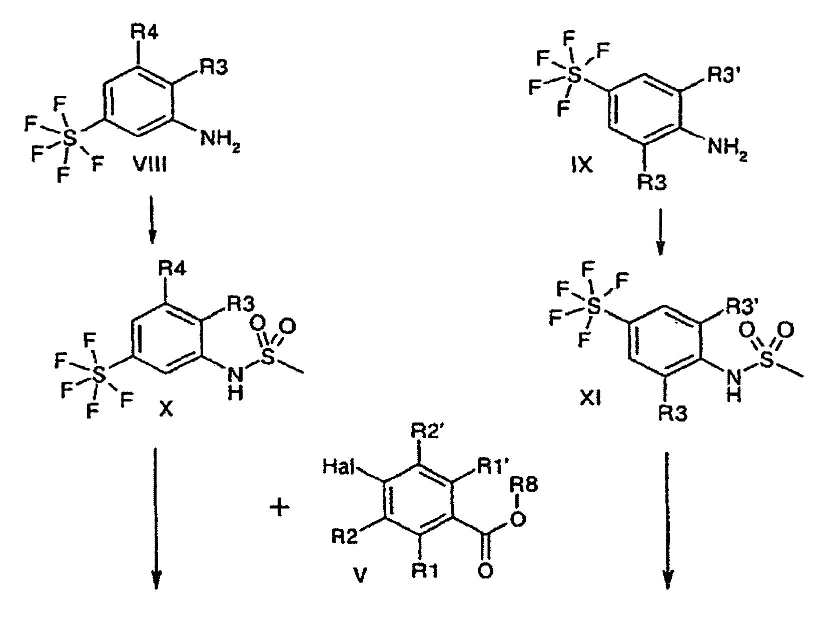 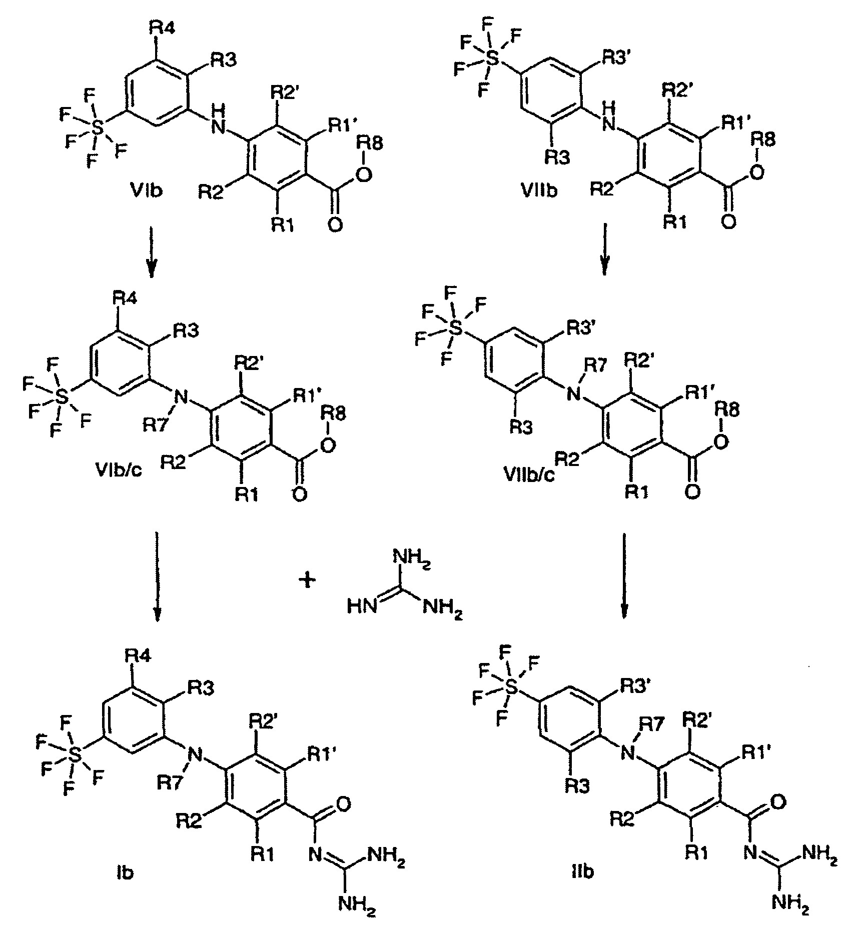
où R1, R1', R2, R2', R3, R3', R4 et R7 présentent la signification indiquée dans les revendications 1, 2, 3 et/ou 4 et où
Hal signifie F, Cl, Br ou I,
R8 signifie hydrogène ou alkyle comprenant 1, 2, 3 ou 4 atomes de carbone.

8. Procédé pour la préparation d'un composé des formules I ou II et/ou de ses sels pharmaceutiquement acceptables, où X représente S(O)ₖₖ, **caractérisé en ce que**
a) on transforme un thiophénol des formules XII ou XIII avec un aromatique de formule V en un composé des formules VIe ou VIIe,
b) pour la préparation d'un composé des formules Ic ou IIc, où kk est différent de zéro, on oxyde un composé des formules VIe ou VIIe en un composé des formules VIf ou VIIf,
et
c) on transforme un composé des formules VIe/f ou VIIe/f avec de la guanidine en une acylguanidine des formules Ic ou IIc 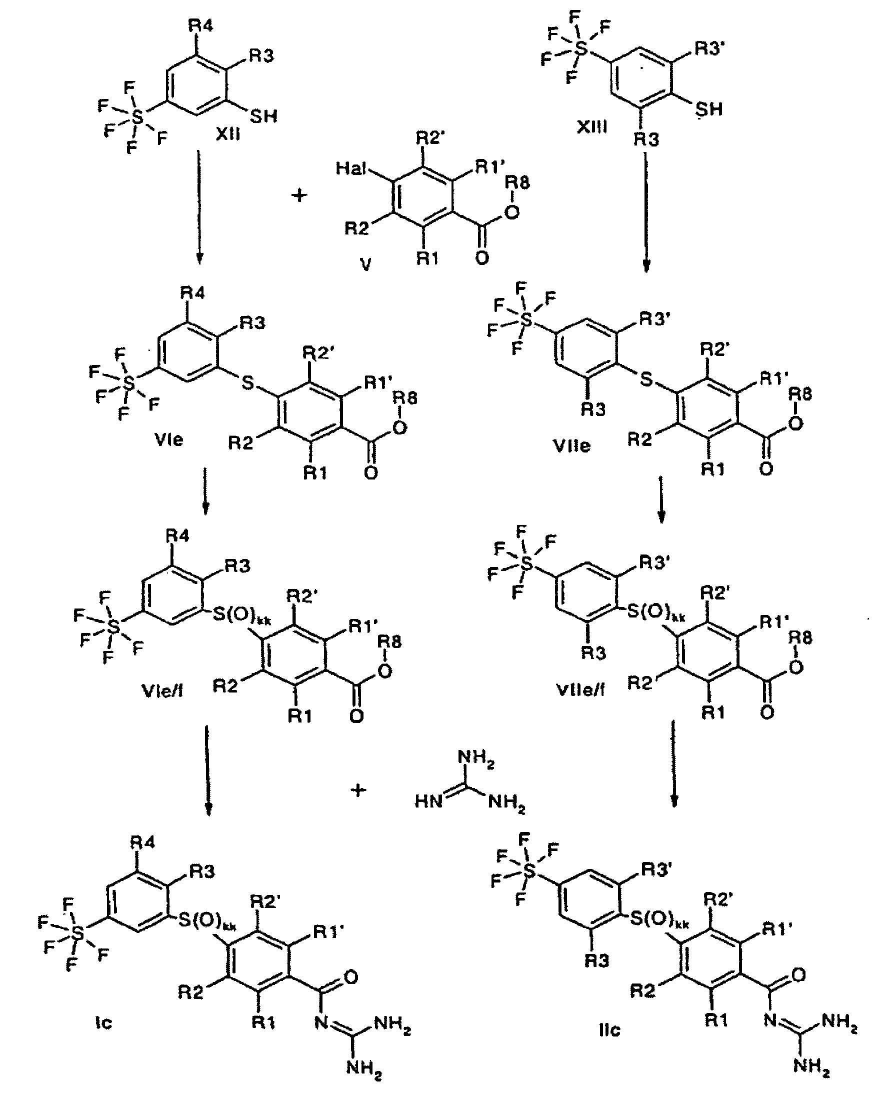
où R1, R1', R2, R2', R3, R3', R4 et kk présentent la signification indiquée dans les revendications 1, 2, 3 et/ou 4 et où
Hal signifie F, Cl, Br ou I,
R8 signifie hydrogène ou alkyle comprenant 1, 2, 3 ou 4 atomes de carbone.

9. Procédé pour la préparation d'un composé des formules I ou II et/ou de ses sels pharmaceutiquement acceptables, où X représente une liaison directe, **caractérisé en ce que**
a) on couple un halogénure des formules XIV ou XV dans un couplage de Suzuki avec un ester de l'acide benzoïque de formule Va en un dérivé biphényle des formules VIg ou VIIg
et
b) on transforme un composé des formules VIg ou VIIg avec de la guanidine en une acylguanidine des formules Id ou IId 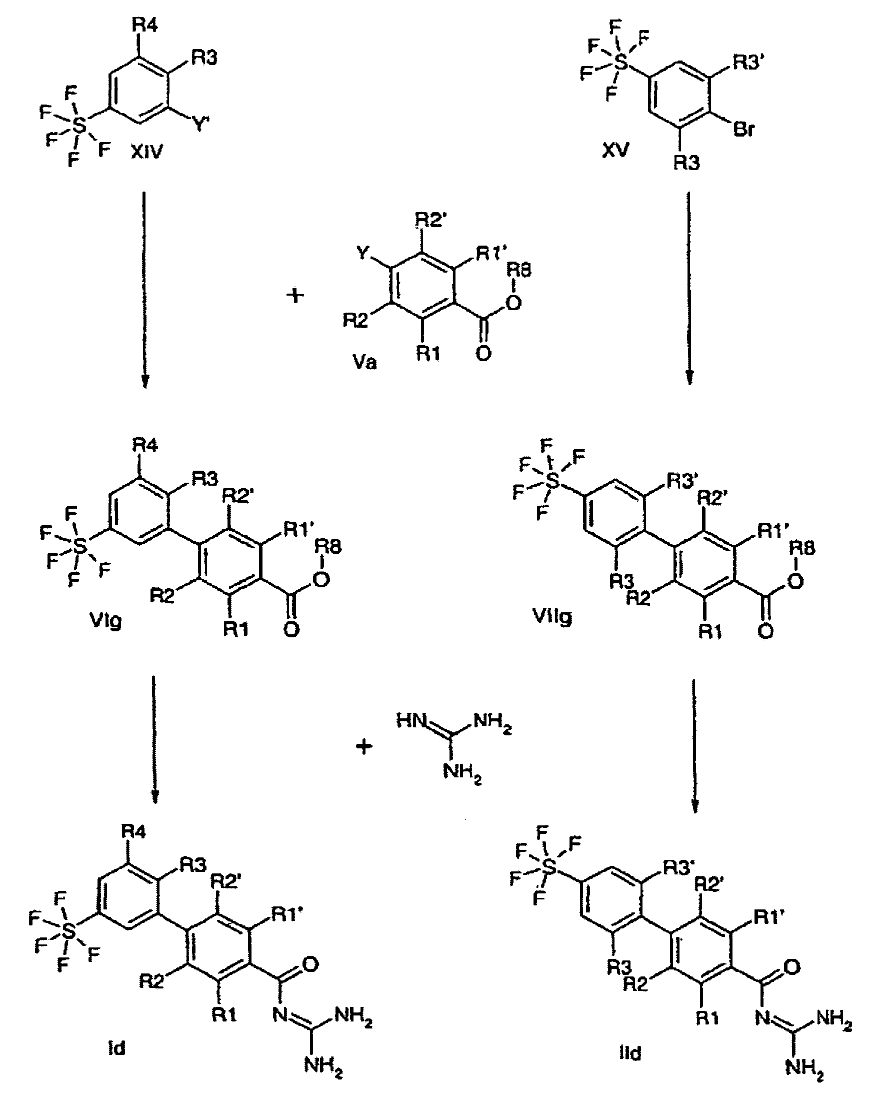
où R1, R1', R2, R2', R3, R3' et R4 présentent la signification indiquée dans les revendications 1, 2, 3 et/ou 4 et où
Y et Y' indépendamment l'un de l'autre, signifient Cl, Br ou I
R8 signifie hydrogène ou alkyle comprenant 1, 2, 3 ou 4 atomes de carbone.

10. Composés de formule I et/ou II et/ou ses sels pharmaceutiquement acceptables selon l'une ou plusieurs des revendications 1 à 5 pour une utilisation comme médicament.

11. Utilisation d'un composé de formule I et/ou II et/ou de ses sels pharmaceutiquement acceptables selon l'une ou plusieurs des revendications 1 à 5 pour la préparation d'un médicament destiné au traitement ou à la prophylaxie de dommages, de maladies ou de maladies secondaires indirectes, aigu(ë)s ou chroniques, d'organes et de tissus, qui sont provoqué(e)s par des événements d'ischémie ou de reperfusion, au traitement ou à la prophylaxie d'arythmies, de la fibrillation ventriculaire du coeur, de l'infarctus cardiaque, de l'angine de poitrine mettant en danger la vie du malade, au traitement ou à la prophylaxie d'états ischémiques du coeur, d'états ischémiques du système nerveux périphérique et central ou d'une attaque ou d'états ischémiques d'organes et de tissus périphériques, au traitement ou à la prophylaxie d'états de choc, de maladies, dans lesquelles la prolifération cellulaire représente une cause primaire ou secondaire, du cancer, de la formation de métastases, de l'hypertrophie ou de l'hyperplasie de la prostate, de l'athérosclérose ou de troubles du métabolisme des graisses, de l'hypertension, de l'hypertonie essentielle, de maladies du système nerveux central, de maladies qui résultent d'une surexcitabilité du SNC, telles que l'épilepsie ou des crampes déclenchées de manière centrale, de maladies du système nerveux central, d'états d'anxiété, de dépressions ou de psychoses, au traitement ou à la prophylaxie du diabète sucré non insulinodépendant (non insulin dependent diabetes mellitus - NIDDM) ou de dommages tardifs du diabète, de thromboses, de maladies suite à un dysfonctionnement endothélial, de la claudication intermittente (claudicatio intermittens), au traitement ou à la prophylaxie de maladies fibrotiques d'organes internes, de maladies fibrotiques du foie, de maladies fibrotiques des reins, de maladies fibrotiques de veines et de maladies fibrotiques du coeur, au traitement ou à la prophylaxie de l'insuffisance cardiaque ou de l'insuffisance cardiaque congestive, de maladies inflammatoires aiguës ou chroniques, de maladies qui sont provoquées par des protozoaires, de la malaria ou de la coccidiose aviaire ou à la préparation d'un médicament destiné à être utilisé lors d'opérations chirurgicales et de transplantations d'organes, à la conservation et à l'entreposage de greffes pour des mesures chirurgicales, à empêcher une modification des tissus liée au vieillissement, à la préparation d'un médicament visant le vieillissement ou à la préparation d'un médicament destiné à l'allongement de la durée de vie, au traitement ou à la diminution des effets cardiotoxiques dans la thyréotoxicose ou à la préparation d'agents diagnostiques.

12. Utilisation selon la revendication 11, où le composé de formule I et/ou II et/ou ses sels pharmaceutiquement acceptables se trouve(nt) en combinaison avec d'autres médicaments ou substances actives.

13. Utilisation d'un composé de formule I et/ou II et/ou de ses sels pharmaceutiquement acceptables selon la revendication 12 en combinaison avec des médicaments ou des substances actives cardiotoxiques ou cytotoxiques pour la préparation d'un médicament présentant des propriétés cardiotoxiques ou cytotoxiques réduites.

14. Utilisation d'un composé de formule I et/ou II et/ou de ses sels pharmaceutiquement acceptables, seul(s) ou en combinaison avec d'autres médicaments ou substances actives selon la revendication 11 et/ou 12 pour la préparation d'un médicament destiné au traitement ou à la prophylaxie de dommages, de maladies ou de maladies secondaires indirectes, aigu(ë)s ou chroniques, d'organes et de tissus qui sont provoqué(e)s par des événements d'ischémie ou de reperfusion.

15. Utilisation d'un composé de formule I et/ou II et/ou de ses sels pharmaceutiquement acceptables, seul(s) ou en combinaison avec d'autres médicaments ou substances actives selon la revendication 11 et/ou 12 pour la préparation d'un médicament destiné au traitement de la fibrillation ventriculaire du coeur mettant en danger la vie du malade.

16. Utilisation d'un composé de formule I et/ou II et/ou de ses sels pharmaceutiquement acceptables, seul(s) ou en combinaison avec d'autres médicaments ou substances actives selon la revendication 11 et/ou 12 pour la préparation d'un médicament destiné au traitement ou à la prophylaxie de la formation de métastases.

17. Utilisation d'un composé de formule I et/ou II et/ou de ses sels pharmaceutiquement acceptables, seul(s) ou en combinaison avec d'autres médicaments ou substances actives selon la revendication 11 et/ou 12 pour la préparation d'un médicament destiné au traitement ou à la prophylaxie de maladies fibrotiques du coeur, de l'insuffisance cardiaque ou de l'insuffisance cardiaque congestive.

18. Remède destiné à une utilisation humaine, vétérinaire ou phytoprotectrice, contenant une quantité efficace d'au moins un composé de formule I et/ou II et/ou de ses sels pharmaceutiquement acceptables selon l'une ou plusieurs des revendications 1 à 5, ensemble avec des supports et des additifs pharmaceutiquement acceptables.

19. Remède selon la revendication 18, où le composé de formule I et/ou II et/ou ses sels pharmaceutiquement acceptables, ensemble avec des supports et des additifs pharmaceutiquement acceptables, se trouvent en combinaison avec au moins une autre substance active pharmacologique ou au moins un autre médicament.

20. Composés de formule VI ou VII 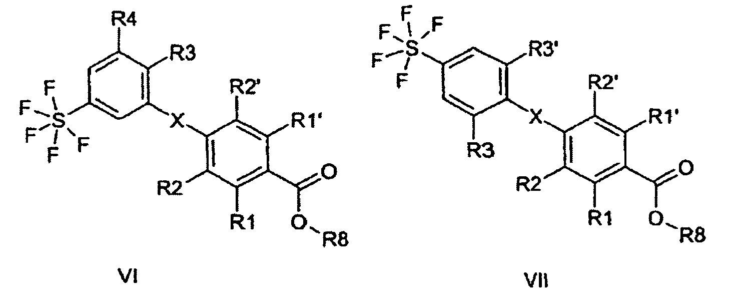 où R1 à R4, R1' à R3' et X sont définis comme dans la revendication 1, 2, 3 ou 4 et R8 représente hydrogène ou alkyle comprenant 1, 2, 3 ou 4 atomes de carbone.

21. Utilisation des composés de formule VI ou VII selon la revendication 20 comme intermédiaire de synthèse.
